# EUROPEAN PATENT APPLICATION

(11) **EP 4 470 539 A1**
(43) Date of publication of application: **04.12.2024**
(21) Application number: 23746577.8
(22) Date of filing: 30.01.2023
(51) Int. Cl.: A61K 31/44, A61K 31/445, A61K 9/08, C07D 211/72, C07D 405/12, C07J 43/00, A61P 7/02, A61P 9/00

(54) **PHARMACEUTICAL COMPOSITION OF ANTIPLATELET DRUG, AND USE THEREOF**

(30) Priority: 28.01.2022 CN 202210107273
(71) Applicant: Shanghai Curegene Pharmaceutical Co., Ltd., Shanghai 201210 (CN)
(72) Inventor: HE, Gongxin, Shanghai 201210 (CN); LU, Changliang, Shanghai 201210 (CN); WU, Hao, Shanghai 201210 (CN)
(74) Representative: Simmons & Simmons LLP (Munich)
(86) International application number: PCT/IB2023/050766
(87) International publication number: WO 2023/144782

(57) **Abstract**

A pharmaceutical composition comprising a compound shown in formula (I) or a pharmaceutically acceptable salt thereof, a cyclodextrin, and optionally, a buffer. Additionally provided are a preparation method for the pharmaceutical composition and a method for using same for treating vascular diseases or inhibiting platelet aggregation.

## Description

### TECHNICAL FIELD

The present invention relates to a pharmaceutical composition comprising a compound of formula (I) or a pharmaceutically acceptable salt thereof, cyclodextrin, and optionally, a buffer. The present application also relates to a preparation method for the pharmaceutical composition and a method for using the same for treating vascular diseases or inhibiting platelet aggregation.

### BACKGROUND

Clopidogrel is a known anti-platelet agent widely used throughout the world and has the structure shown below:

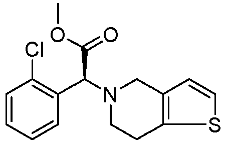

Clopidogrel is a prodrug. After entering the human body, about 85% of it produces inactive metabolites through non-oxidative hydrolysis and about 15% undergoes two-step oxidation under the action of hepatocyte cytochrome P450 isozyme (mainly human liver oxidase CYP2C19) to form the active metabolite H4:

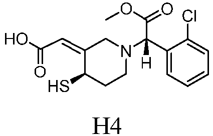

However, clopidogrel has many shortcomings, including: 1) low solubility in aqueous solutions, low conversion to active metabolites and hence high loading dose (300-600 mg) and slow onset of therapeutic effect (2 hours after loading dose administration), and the like; 2) some patients being likely to develop tolerance to clopidogrel because clopidogrel metabolism is mediated by CYP2C19 enzyme and thereby antiplatelet differences are generated among different individuals due to different CYP2C19 expression levels; and 3) drug interactions due to the action of CYP2C19 enzyme, and the like. Due to the above reasons, the dosage form of currently marketed clopidogrel products is an oral tablet, but the oral tablet cannot realize fast acting and thereby cannot meet the requirement of emergent anticoagulation (particularly for patients in an acute stage).

To remedy the above-mentioned deficiencies, attempts have been made in the prior art to prepare clopidogrel as an injection. WO9717064 uses mannitol and alanine to improve the stability of the clopidogrel salt and attempts to prepare a lyophilized powder thereof, but the desired lyophilized powder cannot be obtained because insoluble aggregates are easily formed during lyophilization; WO0010534 discloses an injectable composition comprising clopidogrel or a pharmaceutically acceptable salt thereof (preferably clopidogrel bisulfate), Pluronic F68, a basic pH adjuster compatible with administration via injection and Solutol HS15 and a lyophilized formulation of the injectable composition. This patent application claims that the injection tolerance can be improved, but no effect data is provided to prove this; besides, the method is very troublesome and high in cost, and the injection is not favorable for clinical use due to certain irritation caused by the use of various pharmaceutical excipients. In fact, rapid onset of therapeutic effect cannot be achieved even if clopidogrel is formulated into an injection. This is because the metabolization of clopidogrel must be mediated by CYP2C19 enzyme, and this enzyme is present only in the liver and the metabolic process involving this enzyme is a rate-limiting step in the hepatic metabolic process.

Therefore, there is still a need in the art to develop an anti-platelet aggregation drug and a formulation thereof featuring improved solubility and capability of being used for injection to meet the need for rapid anticoagulation.

### SUMMARY

The present disclosure provides a novel anti-platelet aggregation compound and a pharmaceutical composition thereof having improved solubility, stability, and drug effect.

The compound of the present disclosure further irreversibly inhibits platelet coagulation activity by the production of the active metabolite H4 *in vivo* under the action of a hydrolytic enzyme. The compound of the present disclosure is a compound of formula (I): 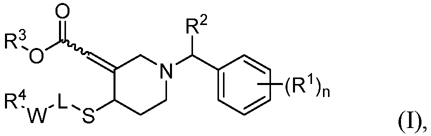 or a pharmaceutically acceptable salt thereof.

The compound of the present disclosure has good solubility in water at pH 1-2, but when the pH is increased, the solubility is greatly reduced, the stability is gradually reduced, and the compound cannot be directly used as a medicament. The inventors have unexpectedly found that when the compound of the present disclosure is mixed with a cyclodextrin, the solubility of the compound at pH 3-4 is improved, the stability of a pharmaceutical formulation containing the compound of the present disclosure as an effective component is enhanced, and the drug effect and intracellular exposure of the compound of the present disclosure are also significantly improved. This helps to provide a pharmaceutical formulation that can be easily administered and has a fast-acting property to meet the need for rapid anticoagulation in acute patients.

Accordingly, in one aspect, the present disclosure provides a pharmaceutical composition comprising:
a compound of formula (I): 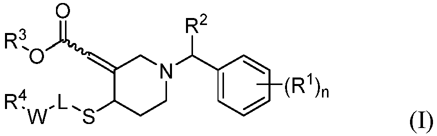
or a pharmaceutically acceptable salt thereof, and
a cyclodextrin,
   wherein 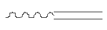
   represents a double bond in Z or E configuration;
   R¹ is selected from the group consisting of hydrogen, halogen, nitro, cyano, hydroxyl, amino, alkyl, alkenyl, alkynyl, heteroalkyl, heteroalkenyl, and heteroalknyl, wherein each of alkyl, alkenyl, alkynyl, heteroalkyl, heteroalkenyl, and heteroalknyl is optionally substituted with one or more R^{a};
   R² is -C(O)R^{b};
   R³ is selected from the group consisting of hydrogen, alkyl, alkenyl, alkynyl, heteroalkyl, heteroalkenyl, heteroalknyl, saturated or partially unsaturated cycloalkyl, saturated or partially unsaturated heterocyclyl, aryl, and heteroaryl, wherein each of alkyl, alkenyl, alkynyl, heteroalkyl, heteroalkenyl, heteroalkynyl, cycloalkyl, heterocyclyl, aryl and heteroaryl is optionally substituted with cyano, halogen, hydroxyl, amino, alkyl, alkenyl, alkynyl, heteroalkyl, heteroalkenyl, or heteroalkynyl;
   L is selected from the group consisting of a direct bond, alkyl, alkenyl, alkynyl, heteroalkyl, heteroalkenyl, heteroalknyl, saturated or partially unsaturated cycloalkyl, saturated or partially unsaturated heterocyclyl, aryl, and heteraryl, wherein each of alkyl, alkenyl, alkynyl, heteroalkyl, heteroalkenyl, heteroalknyl, cycloalkyl, heterocyclyl, aryl and heteroaryl is optionally substituted with one or more R^{f};
   W is selected from the group consisting of: 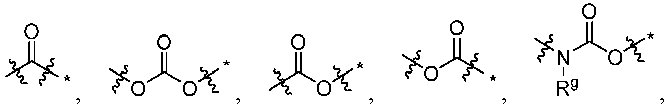 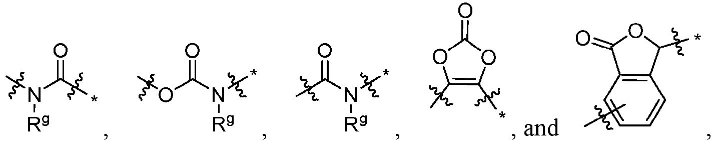
   wherein the * end of W is connected to L;
   R⁴ is selected from the group consisting of hydrogen, alkyl, alkenyl, alkynyl, heteroalkyl, heteroalkenyl, heteroalknyl, saturated or partially unsaturated cycloalkyl, saturated or partially unsaturated heterocyclyl, aryl, and heteraryl, wherein each of alkyl, alkenyl, alkynyl, heteroalkyl, heteroalkenyl, heteroalkynyl, cycloalkyl, heterocyclyl, aryl and heteroaryl is optionally substituted with cyano, halogen, hydroxyl, amino, alkyl, alkenyl, alkynyl, heteroalkyl, heteroalkenyl, heteroalkynyl;
   each of R^{a} is independently selected from the group consisting of hydrogen, halogen, hydroxyl, amino, cyano, nitro, or -NR^{c}R^{d};
   R^{b} is selected from the group consisting of hydrogen, hydroxyl, alkyl, alkenyl, alkynyl, heteroalkyl, heteroalkenyl, heteroalknyl, saturated or partially unsaturated cycloalkyl, saturated or partially unsaturated heterocyclyl, aryl, heteroaryl, -NR^{c}R^{d} and -OR^{e};
   each of R^{c} and R^{d} is independently selected from the group consisting of hydrogen, alkyl, alkenyl, alkynyl, heteroalkyl, heteroalkenyl, heteroalkynyl, aryl, and heteroaryl, wherein each of alkyl, alkenyl, alkynyl, heteroalkyl, heteroalkenyl, heteroalkynyl, aryl and heteroaryl is optionally substituted with cyano, halogen, hydroxyl, or amino;
   R^{e} is selected from the group consisting of hydrogen, alkyl, alkenyl, alkynyl, heteroalkyl, heteroalkenyl, heteroalkynyl, saturated or partially unsaturated cycloalkyl, saturated or partially unsaturated heterocyclyl, aryl, and heteroaryl, wherein each of alkyl, alkenyl, alkynyl, heteroalkyl, heteroalkenyl, heteroalkynyl, cycloalkyl, heterocyclyl, aryl and heteroaryl is optionally substituted with cyano, halogen, hydroxyl, amino, alkyl, alkenyl, alkynyl, heteroalkyl, heteroalkenyl, or heteroalkynyl;
   each of R^{f} is independently selected from the group consisting of hydrogen, cyano, halogen, hydroxyl, amino, alkyl, alkenyl, alkynyl, heteroalkyl, heteroalkenyl, heteroalkynyl, saturated or partially unsaturated cycloalkyl, saturated or partially unsaturated heterocyclyl, aryl, and heteroaryl; or
   two R^{f} together with the atom to which they are both attached, form saturated or partially unsaturated cycloalkyl, or saturated or partially unsaturated heterocyclyl, wherein each of cycloalkyl and heterocyclyl is optionally substituted with cyano, halogen, hydroxyl, amino and alkyl;
   R^{g} is selected from the group consisting of hydrogen, alkyl, alkenyl, alkynyl, heteroalkyl, heteroalkenyl, heteroalkynyl, saturated or partially unsaturated cycloalkyl, saturated or partially unsaturated heterocyclyl, aryl, and heteroaryl, wherein each of hydrogen, alkyl, alkenyl, alkynyl, heteroalkyl, heteroalkenyl, heteroalkynyl, cycloalkyl, heterocyclyl, aryl and heteroaryl is optionally substituted with cyano, halogen, hydroxyl, or amino;
   n is 0, 1, 2, 3, 4 or 5.

In another aspect, the present disclosure also provides a method for preparing a pharmaceutical composition of the present disclosure, which comprises:
mixing a cyclodextrin with water to form a first mixture;
optionally, adding a buffer to the first mixture to form a second mixture;
adding an acid to the second mixture to adjust the pH to 1-1.5, thus forming a third mixture;
adding the compound or the pharmaceutically acceptable salt thereof to the third mixture to form a fourth mixture;
adding a base to the fourth mixture to adjust the pH to 3-4, thus forming a fifth mixture; and optionally, adding water for injection to the fifth mixture to form the pharmaceutical composition.

In yet another aspect, the present disclosure provides a method for treating a vascular disease in a subject in need thereof, which comprises administering to the subject a therapeutically effective amount of the pharmaceutical composition of the present disclosure.

In still another aspect, the present disclosure provides a method for inhibiting platelet aggregation in a subject in need thereof, which comprises administering to the subject a therapeutically effective amount of the pharmaceutical composition of the present disclosure.

### BRIEF DESCRIPTION OF THE DRAWINGS

FIG. 1 shows the concentrations of the active metabolite in rat plasma after oral administration of (a) clopidogrel, compound 1a, and compound 1b, and (b) clopidogrel, compound 2a, and compound 2b at a dose of 10 mg/kg.
FIG. 2 shows the concentrations of the active metabolite in rat plasma after oral administration of clopidogrel at a dose of 10 mg/kg, oral administration of compound 3 at a dose of 2 mg/kg, and intravenous injection of compound 3 at a dose of 1 mg/kg.
FIG. 3 shows the inhibition (%) of agglomeration after oral administration of the test compounds at doses of 10 mg/kg (clopidogrel), 0.5 mg/kg (compound 1a), and 2 mg/kg (compound 1b) in rats.

### DETAILED DESCRIPTION

The present disclosure relates to novel anti-platelet aggregation compounds and pharmaceutical compositions thereof and methods of using such pharmaceutical compositions to treat vascular diseases or inhibit platelet aggregation. When describing the compounds, compositions and methods of the present disclosure, the following terms have the following meanings, unless otherwise indicated.

### Definitions

Definitions of specific functional groups and chemical terms are described in more detail below. For purposes of this disclosure, the chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Organic Chemistry, Thomas Sorrell, 2nd Edition, University Science Books, Sausalito, 2006; Smith and March March's Advanced Organic Chemistry, 6th Edition, John Wiley & Sons, Inc., New York, 2007; Larock, Comprehensive Organic Transformations, 3rd Edition, VCH Publishers, Inc., New York, 2018; Carruthers, Some Modern Methods of Organic Synthesis, 4th Edition, Cambridge University Press, Cambridge, 2004; the entire contents of each of which are incorporated herein by reference.

At various places in the present disclosure, linking substituents are described. It is specifically intended that each linking substituent includes both the forward and backward forms of the linking substituent. For example, -NR(CR'R")- includes both -NR(CR'R")- and -(CR'R")NR-. Where the structure clearly requires a linking group, the Markush variables listed for that group are understood to be linking groups. For example, if the structure requires a linking group and the Markush group definition for that variable lists "alkyl", then it is understood that the "alkyl" represents a linking alkylene group.

When a bond to a substituent is shown to cross a bond connecting two atoms in a ring, then such substituent may be bonded to any atom in the ring. When a substituent is listed without indicating the atom via which such substituent is bonded to the rest of the compound of a given formula, then such substituent may be bonded via any atom in such formula. Combinations of substituents and/or variables are permissible, but only if such combinations result in stable compounds.

When "*" is shown adjacent to an atom of a compound, it indicates that the compound comprises such atom as an asymmetric center that is in either (R) or (S) stereo-configuration.

When any variable (e.g., Rⁱ) occurs more than one time in any constituent or formula for a compound, its definition at each occurrence is independent of its definition at every other occurrence. Thus, for example, if a group is shown to be substituted with 0-2 Rⁱ moieties, then the group may optionally be substituted with up to two Rⁱ moieties and Rⁱ at each occurrence is selected independently from the definition of Rⁱ. Also, combinations of substituents and/or variables are permissible, but only if such combinations result in stable compounds.

As used herein, the term "Cᵢ₋ⱼ" indicates a range of the carbon atoms numbers, wherein i and j are integers and the range of the carbon atoms numbers includes the endpoints (i.e. i and j) and each integer point in between, and wherein j is greater than i. For examples, C₁₋₆ indicates a range of one to six carbon atoms, including one carbon atom, two carbon atoms, three carbon atoms, four carbon atoms, five carbon atoms and six carbon atoms. In some embodiments, the term "C₁₋₁₂" indicates 1 to 12, particularly 1 to 10, particularly 1 to 8, particularly 1 to 6, particularly 1 to 5, particularly 1 to 4, particularly 1 to 3 or particularly 1 to 2 carbon atoms.

As used herein, the term "alkyl", whether as part of another term or used independently, refers to a saturated linear or branched-chain hydrocarbon radical, which may be optionally substituted independently with one or more substituents described below. The term "Cᵢ₋ⱼ alkyl" refers to an alkyl having i to j carbon atoms.In some embodiments, the alkyl group contains 1 to 12 carbon atoms. In some embodiments, the alkyl group contains 1 to 11 carbon atoms. In some embodiments, the alkyl group contains 1 to 11 carbon atoms, 1 to 10 carbon atoms, 1 to 9 carbon atoms, 1 to 8 carbon atoms, 1 to 7 carbon atoms, 1 to 6 carbon atoms, 1 to 5 carbon atoms, 1 to 4 carbon atoms, 1 to 3 carbon atoms, or 1 to 2 carbon atoms. Examples of the alkyl group include, but are not limited to, methyl, ethyl, 1-propyl (*n*-propyl), 2-propyl (isopropyl), 1-butyl (*n*-butyl), 2-methyl-1-propyl (i-butyl), 2-butyl (*sec-*butyl), 2-methyl-2-propyl (*tert* butyl), 1-pentyl (*n*-pentyl), 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, 1-heptyl, 1-octyl, and the like. Examples of "C₁₋₁₂ alkyl" include, but are not limited to, methyl, ethyl, propyl, butyl, pentyl, hexyl, heptyl, octyl, nonyl, decyl, undecyl, and dodecyl. Examples of "C₁₋₆ alkyl" are methyl, ethyl, propyl, isopropyl, *n*-butyl, isobutyl, sec-butyl, *tert*-butyl, *n*-pentyl, 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, and the like.

The alkyl group may be further substituted with a substituent that independently substitutes for one or more hydrogen atoms on one or more carbon atoms of the alkyl group. Examples of such substituents may include, but are not limited to, acyl, alkyl, alkenyl, alkynyl, halogen, hydroxy, alkoxy, haloalkyl, haloalkoxy, alkylcarbonyloxy, arylcarbonyloxy, alkoxycarbonyloxy, aryloxycarbonyloxy, carboxylate group, alkylcarbonyl, arylcarbonyl, alkoxycarbonyl, aminocarbonyl, alkylaminocarbonyl, dialkylaminocarbonyl, alkylthiocarbonyl, phosphate group, phosphonyl, phosphinyl, amino (including alkylamino, dialkylamino, arylamino, diarylamino, and alkylarylamino), acylamino (including alkylcarbonylamino, arylcarbonylamino, carbamoyl, and ureido), amidino, imino, mercapto, alkylthio, arylthio, thiocarboxylate group, sulfate group, alkylsulfonyl, sulfonate group, sulfamoyl, sulfonamido, nitro, trifluoromethyl, cyano, nitro, azido, heterocyclyl, alkylaryl, or aromatic or heteroaromatic moieties. Alkenyl, alkynyl, saturated or partially unsaturated cycloalkyl, heteroalkyl, heterocyclyl, arylalkyl, heteroarylalkyl, heterocyclylalkyl, cycloalkylalkyl, aryl, and heteroaryl groups described below may also be similarly substituted.

As used herein, the term "alkenyl", whether as part of another term or used independently, refers to linear or branched-chain hydrocarbon radical having at least one carbon-carbon double bond, which may be optionally substituted independently with one or more substituents described herein, and includes radicals having "cis" and "trans" orientations, or alternatively, "E" and "Z" orientations. In some embodiments, alkenyl groups contain 2 to 12 carbon atoms. In some embodiments, alkenyl groups contain 2 to 11 carbon atoms. In some embodiments, alkenyl groups contain 2 to 11 carbon atoms, 2 to 10 carbon atoms, 2 to 9 carbon atoms, 2 to 8 carbon atoms, 2 to 7 carbon atoms, 2 to 6 carbon atoms, 2 to 5 carbon atoms, 2 to 4 carbon atoms, 2 to 3 carbon atoms, and in some embodiments, alkenyl groups contain 2 carbon atoms. Examples of alkenyl group include, but are not limited to, ethylenyl (or vinyl), propenyl (allyl), butenyl, pentenyl, 1-methyl-2 buten-1-yl, 5-hexenyl, and the like.

As used herein, the term "alkynyl", whether as part of another term or used independently, refers to a linear or branched hydrocarbon radical having at least one carbon-carbon triple bond, which may be optionally substituted independently with one or more substituents described herein. In some embodiments, alkenyl groups contain 2 to 12 carbon atoms. In some embodiments, alkynyl groups contain 2 to 11 carbon atoms. In some embodiments, alkynyl groups contain 2 to 11 carbon atoms, 2 to 10 carbon atoms, 2 to 9 carbon atoms, 2 to 8 carbon atoms, 2 to 7 carbon atoms, 2 to 6 carbon atoms, 2 to 5 carbon atoms, 2 to 4 carbon atoms, 2 to 3 carbon atoms, and in some embodiments, alkynyl groups contain 2 carbon atoms. Examples of alkynyl group include, but are not limited to, ethynyl, 1-propynyl, 2-propynyl, and the like.

As used herein, the term "amino" refers to -NH₂ group. Amino groups may also be substituted with one or more groups such as alkyl, aryl, carbonyl or other amino groups.

As used herein, the term "aryl", whether as part of another term or used independently, refers to monocyclic and polycyclic ring systems having a total of 5 to 20 ring members, wherein at least one ring in the system is aromatic and wherein each ring in the system contains 3 to 12 ring members. Examples of "aryl" include, but are not limited to, phenyl, biphenyl, naphthyl, anthracyl and the like, which may bear one or more substituents. Also included within the scope of the term "aryl", as it is used herein, is a group in which an aromatic ring is fused to one or more additional rings. In the case of polycyclic ring system, only one of the rings needs to be aromatic (e.g., 2,3-dihydroindole), although all of the rings may be aromatic (e.g., quinoline). The second ring can also be fused or bridged. Examples of polycyclic aryl include, but are not limited to, benzofuranyl, indanyl, phthalimidyl, naphthimidyl, phenanthridinyl, or tetrahydronaphthyl, and the like. Aryl groups can be substituted at one or more ring positions with substituents as described above.

As used herein, the term "cycloalkyl", whether as part of another term or used independently, refer to a monovalent non-aromatic, saturated or partially unsaturated monocyclic and polycyclic ring system, in which all the ring atoms are carbon and which contains at least three ring forming carbon atoms. In some embodiments, the cycloalkyl may contain 3 to 12 ring forming carbon atoms, 3 to 10 ring forming carbon atoms, 3 to 9 ring forming carbon atoms, 3 to 8 ring forming carbon atoms, 3 to 7 ring forming carbon atoms, 3 to 6 ring forming carbon atoms, 3 to 5 ring forming carbon atoms, 4 to 12 ring forming carbon atoms, 4 to 10 ring forming carbon atoms, 4 to 9 ring forming carbon atoms, 4 to 8 ring forming carbon atoms, 4 to 7 ring forming carbon atoms, 4 to 6 ring forming carbon atoms, 4 to 5 ring forming carbon atoms. Cycloalkyl groups may be saturated or partially unsaturated. Cycloalkyl groups may be substituted. In some embodiments, the cycloalkyl group may be a saturated cyclic alkyl group. In some embodiments, the cycloalkyl group may be a partially unsaturated cyclic alkyl group that contains at least one double bond or triple bond in its ring system. In some embodiments, the cycloalkyl group may be monocyclic or polycyclic. Examples of monocyclic cycloalkyl group include, but are not limited to, cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, cyclohexadienyl, cycloheptyl, cyclooctyl, cyclononyl, cyclodecyl, cycloundecyl and cyclododecyl. Examples of polycyclic cycloalkyl group include, but are not limited to, adamantyl, norbornyl, fluorenyl, spiro-pentadienyl, spiro[3.6]-decanyl, bicyclo[1,1,1]pentenyl, bicyclo[2,2,1]heptenyl, and the like.

As used herein, the term "cyano" refers to -CN.

As used herein, the term "halogen" refers to an atom selected from fluorine (or fluoro), chlorine (or chloro), bromine (or bromo) and iodine (or iodo).

As used herein, the term "heteroatom" refers to nitrogen, oxygen, sulfur, or phosphorus, and includes any oxidized form of nitrogen, sulfur, or phosphorus as well as any quaternized form of basic nitrogen.

As used herein, the term "heteroalkyl" refers to alkyl having at least one carbon atom substituted with a heteroatom selected from N, O, or S. Heteroalkyl may be a carbon atom or heteroatom group (i.e., the heteroatom may be present in the middle or at the end of the group), and may be optionally independently substituted with one or more substituents described herein. The term "heteroalkyl" includes alkoxy and heteroalkoxy groups.

As used herein, the term "heteroalkenyl" refers to an alkenyl, at least one of the carbon atoms of which is replaced with a heteroatom selected from N, O, or S. The heteroalkenyl may be a carbon radical or heteroatom radical (i.e., the heteroatom may appear in the middle or at the end of the radical), and may be optionally substituted independently with one or more substituents described herein.

As used herein, the term "heteroalkynyl" refers to an alkynyl, at least one of the carbon atoms of which is replaced with a heteroatom selected from N, O, or S. The heteroalkynyl may be a carbon radical or heteroatom radical (i.e., the heteroatom may appear in the middle or at the end of the radical), and may be optionally substituted independently with one or more substituents described herein.

As used herein, the term "heteroaryl", whether used as part of another term or independently, refers to aryl having one or more heteroatoms in addition to carbon atoms. Heteroaryl groups may be monocyclic. Examples of monocyclic heteroaryl include, but are not limited to, thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, pyridinyl, pyridazinyl, pyrimidinyl, pyrazinyl, and the like. Heteroaryl groups may also be polycyclic, wherein the heteroaryl ring is fused to one or more aryl, cycloalkyl, or heterocyclyl rings, and the point of attachment is on the heteroaryl ring. Examples of polycyclic heteroaryl include, but are not limited to, indolyl, isoindolyl, benzothienyl, benzofuranyl, dibenzofuranyl, indazolyl, benzimidazolyl, benzothiazolyl, quinolinyl, isoquinolinyl, cinnolinyl, phthaloyl, quinazolinyl, quinoxalinyl, 4H-quinolinyl, carbazolyl, acridinyl, phenazinyl, phenothiazinyl, benzoxazinyl, tetrahydroquinolinyl, tetrahydroisoquinolinyl, and the like.

As used herein, the term "heterocyclyl" refers to a saturated or partially unsaturated carbocyclyl group in which one or more ring atoms are heteroatoms independently selected from oxygen, sulfur, nitrogen, phosphorus, and the like, the remaining ring atoms being carbon, wherein one or more ring atoms may be optionally substituted independently with one or more substituents. In some embodiments, the heterocyclyl is a saturated heterocyclyl. In some embodiments, the heterocyclyl is a partially unsaturated heterocyclyl having one or more double bonds in its ring system. In some embodiments, the heterocyclyl may contains any oxidized form of carbon, nitrogen or sulfur, and any quaternized form of a basic nitrogen. "Heterocyclyl" also includes radicals wherein the heterocyclyl radicals are fused with a saturated, partially unsaturated, or fully unsaturated (i.e., aromatic) carbocyclic or heterocyclic ring. The heterocyclyl radical may be carbon linked or nitrogen linked where such is possible. In some embodiments, the heterocycle is carbon linked. In some embodiments, the heterocycle is nitrogen linked. For example, a group derived from pyrrole may be pyrrol-1-yl (nitrogen linked) or pyrrol-3-yl (carbon linked). Further, a group derived from imidazole may be imidazol-1-yl (nitrogen linked) or imidazol-3-yl (carbon linked).

In some embodiments, the term "3-12 membered heterocyclyl" refers to a 3-12 membered saturated or partially unsaturated monocyclic or polycyclic heterocyclic ring system having 1 to 3 heteroatoms independently selected from nitrogen, oxygen, or sulfur. Fused, spiro, and bridged ring systems are also included within the scope of this definition. Examples of monocyclic heterocyclyl include, but are not limited to, oxetanyl, 1,1-dioxothienylpyrrolidinyl, tetrahydrofuranyl, tetrahydrothienyl, pyrrolyl, furanyl, thienyl, pyrazolyl, imidazolyl, triazolyl, oxazolyl, thiazolyl, piperidinyl, piperazinyl, morpholinyl, pyridinyl, pyrazinyl, pyrimidinyl, pyridazinyl, triazinyl, pyridinonyl, pyrimidinonyl, pyrazinonyl, pyrimidinonyl, pyridazinonyl, pyrrolidinyl, triazinonyl, and the like. Examples of fused heterocyclyl include, but are not limited to, phenyl- or pyridinyl-fused rings, such as quinolinyl, isoquinolinyl, quinoxalinyl, quinolizinyl, quinazolinyl, azaindolizinyl, pteridinyl, chromenyl, isochromenyl, indolyl, isoindolyl, indolizinyl, indazolyl, purinyl, benzofuranyl, isobenzofuranyl, benzimidazolyl, benzothienyl, benzothiazolyl, carbazolyl, phenazinyl, phenothiazinyl, phenanthridinyl, imidazo[1,2-*a*]pyridinyl, [1,2,4]triazolo[4,3-*a*]pyridinyl, [1,2,3]triazolo[4,3-*a*]pyridinyl, and other groups. Examples of spiroheterocyclyl include, but are not limited to, spiropyranyl, spirooxazinyl, and the like. Examples of bridged heterocyclyl include, but are not limited to, morpholinyl, hexamethylenetetramino, 3-azabicyclo[3.1.0]hexane, 8-azabicyclo[3.2.1]octane, 1-azabicyclo[2.2.2]octane, 1,4-diazabicyclo[2.2.2]octane (DABCO), and the like.

As used herein, the term "hydroxyl" refers to -OH.

As used herein, the term "nitro" refers to the -NO₂ group.

As used herein, the term "partially unsaturated" refers to a radical that includes at least one double or triple bond. The term "partially unsaturated" is intended to encompass rings having multiple sites of unsaturation, but is not intended to include aromatic (i.e., fully unsaturated) moieties.

As used herein, the term "substituted", whether preceded by the term "optionally" or not, means that one or more hydrogens of the designated moiety are replaced with a suitable substituent. It will be understood that "substitution" or "substituted with" includes the implicit proviso that such substitution is in accordance with permitted valence of the substituted atom and that the substitution results in a stable or chemically feasible compound, e.g., which does not spontaneously undergo transformation such as by rearrangement, cyclization, elimination, etc. Unless otherwise indicated, an "optionally substituted" group may have a suitable substituent at each substitutable position of the group, and when more than one position in any given structure may be substituted with more than one substituent selected from a specified group, the substituent may be either the same or different at every position. It will be understood by those skilled in the art that substituents can themselves be substituted, if appropriate. Unless specifically stated as "unsubstituted", references to chemical moieties herein are understood to include substituted variants. For example, reference to an "aryl" group or moiety implicitly includes both substituted and unsubstituted variants.

As used herein, the terms "agglomeration", "agglutination", and "aggregation" have the same meaning.

### Compounds

The present disclosure provides novel compounds of Formula (I) and pharmaceutically acceptable salts thereof, synthetic methods for making the compounds, pharmaceutical compositions containing them and various uses of the disclosed compounds.

In one aspect, the present disclosure provides a compound having Formula (I): 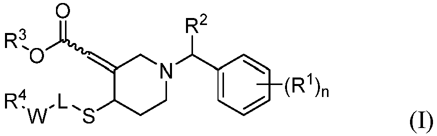 or a pharmaceutically acceptable salt thereof,
wherein 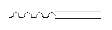
represents a double bond in Z or E configuration;
R¹ is selected from the group consisting of hydrogen, halogen, nitro, cyano, hydroxyl, amino, alkyl, alkenyl, alkynyl, heteroalkyl, heteroalkenyl, and heteroalknyl, wherein each of alkyl, alkenyl, alkynyl, heteroalkyl, heteroalkenyl, and heteroalknyl is optionally substituted with one or more R^{a};
R² is -C(O)R^{b};
R³ is selected from the group consisting of hydrogen, alkyl, alkenyl, alkynyl, heteroalkyl, heteroalkenyl, heteroalknyl, saturated or partially unsaturated cycloalkyl, saturated or partially unsaturated heterocyclyl, aryl, and heteroaryl, wherein each of alkyl, alkenyl, alkynyl, heteroalkyl, heteroalkenyl, heteroalkynyl, cycloalkyl, heterocyclyl, aryl and heteroaryl is optionally substituted with cyano, halogen, hydroxyl, amino, alkyl, alkenyl, alkynyl, heteroalkyl, heteroalkenyl, or heteroalkynyl;
L is selected from the group consisting of a direct bond, alkyl, alkenyl, alkynyl, heteroalkyl, heteroalkenyl, heteroalknyl, saturated or partially unsaturated cycloalkyl, saturated or partially unsaturated heterocyclyl, aryl, and heteraryl, wherein each of alkyl, alkenyl, alkynyl, heteroalkyl, heteroalkenyl, heteroalknyl, cycloalkyl, heterocyclyl, aryl and heteroaryl is optionally substituted with one or more R^{f};
W is selected from the group consisting of: 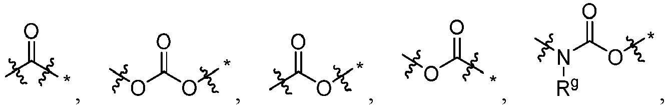 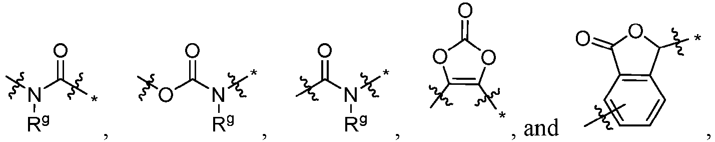
wherein the * end of W is connected to L;
R⁴ is selected from the group consisting of hydrogen, alkyl, alkenyl, alkynyl, heteroalkyl, heteroalkenyl, heteroalknyl, saturated or partially unsaturated cycloalkyl, saturated or partially unsaturated heterocyclyl, aryl, and heteraryl, wherein each of alkyl, alkenyl, alkynyl, heteroalkyl, heteroalkenyl, heteroalkynyl, cycloalkyl, heterocyclyl, aryl and heteroaryl is optionally substituted with cyano, halogen, hydroxyl, amino, alkyl, alkenyl, alkynyl, heteroalkyl, heteroalkenyl, heteroalkynyl;
each of R^{a} is independently selected from the group consisting of hydrogen, halogen, hydroxyl, amino, cyano, nitro, or -NR^{c}R^{d};
R^{b} is selected from the group consisting of hydrogen, hydroxyl, alkyl, alkenyl, alkynyl, heteroalkyl, heteroalkenyl, heteroalknyl, saturated or partially unsaturated cycloalkyl, saturated or partially unsaturated heterocyclyl, aryl, heteraryl, -NR^{c}R^{d} and -OR^{e};
each of R^{c} and R^{d} is independently selected from the group consisting of hydrogen, alkyl, alkenyl, alkynyl, heteroalkyl, heteroalkenyl, heteroalkynyl, aryl, and heteroaryl, wherein each of alkyl, alkenyl, alkynyl, heteroalkyl, heteroalkenyl, heteroalkynyl, aryl and heteroaryl is optionally substituted with cyano, halogen, hydroxyl, or amino;
R^{e} is selected from the group consisting of hydrogen, alkyl, alkenyl, alkynyl, heteroalkyl, heteroalkenyl, heteroalkynyl, saturated or partially unsaturated cycloalkyl, saturated or partially unsaturated heterocyclyl, aryl, and heteroaryl, wherein each of alkyl, alkenyl, alkynyl, heteroalkyl, heteroalkenyl, heteroalkynyl, cycloalkyl, heterocyclyl, aryl and heteroaryl is optionally substituted with cyano, halogen, hydroxyl, amino, alkyl, alkenyl, alkynyl, heteroalkyl, heteroalkenyl, or heteroalkynyl;
each of R^{f} is independently selected from the group consisting of hydrogen, cyano, halogen, hydroxyl, amino, alkyl, alkenyl, alkynyl, heteroalkyl, heteroalkenyl, heteroalkynyl, saturated or partially unsaturated cycloalkyl, saturated or partially unsaturated heterocyclyl, aryl, and heteroaryl; or
two R^{f} together with the atom to which they are both attached, form saturated or partially unsaturated cycloalkyl, or saturated or partially unsaturated heterocyclyl, wherein each of cycloalkyl and heterocyclyl is optionally substituted with cyano, halogen, hydroxyl, amino and alkyl;
R^{g} is selected from the group consisting of hydrogen, alkyl, alkenyl, alkynyl, heteroalkyl, heteroalkenyl, heteroalkynyl, saturated or partially unsaturated cycloalkyl, or saturated or partially unsaturated heterocyclyl, aryl, and heteroaryl, wherein each of hydrogen, alkyl, alkenyl, alkynyl, heteroalkyl, heteroalkenyl, heteroalkynyl, cycloalkyl, heterocyclyl, aryl and heteroaryl is optionally substituted with cyano, halogen, hydroxy, or amino;
n is 0, 1, 2, 3, 4 or 5.

In some embodiments, R¹ is selected from the group consisting of hydrogen, halogen, nitro, cyano, hydroxy, amino, alkyl, and heteroalkyl, wherein each of alkyl and heteroalkyl is optionally substituted with one or more R^{a}.

In certain embodiments, each of R^{a} is independently selected from halogen, hydroxyl, cyano or nitro.

In some embodiments, R¹ is halogen, cyano, hydroxyl, amino, or alkyl optionally substituted with one or more R^{a}.

In certain embodiments, R¹ is halogen, cyano, or alkyl optionally substituted with one or more R^{a}.

In certain embodiments, R¹ is fluoro, chloro, bromo, cyano, methyl or trifluoromethyl.

In some embodiments, n is 1, 2 or 3. In certain embodiments, n is 1 or 2. In certain embodiments, n is 1.

In some embodiments, R² is -C(O)R^{b}, wherein R^{b} is selected from the group consisting of hydrogen, hydroxyl, alkyl, alkenyl, alkynyl, heteroalkyl, heteroalkenyl, heteroalknyl, saturated or partially unsaturated cycloalkyl, saturated or partially unsaturated heterocycloalkyl, -NR^{c}R^{d} and -OR^{e}.

In certain embodiments, each of R^{c} and R^{d} is independently selected from the group consisting of hydrogen, alkyl, and alkenyl, wherein each of alkyl, and alkenyl is optionally substituted with halogen or hydroxyl.

In certain embodiments, R^{e} is selected from the group consisting of alkyl, alkenyl, heteroalkyl, heteroalkenyl, aryl, and heteroaryl, wherein each of alkyl, alkenyl, heteroalkyl, heteroalkenyl, aryl and heteroaryl is optionally substituted with cyano, halogen, hydroxy, amino, or alkyl.

In some embodiments, R² is -C(O)R^{b}, wherein R^{b} is hydrogen, hydroxyl, alkyl, saturated or partially unsaturated cycloalkyl, or -OR^{e}.

In certain embodiments, R² is -C(O)R^{b}, wherein R^{b} is saturated cycloalkyl or -OR^{e}, and R^{e} is alkyl.

In certain embodiments, R² is -C(O)R^{b}, wherein R^{b} is saturated C₃₋₆ cycloalkyl or -OR^{e}, and R^{e} is C₁₋₆ alkyl.

In certain embodiments, R² is -C(O)R^{b}, wherein R^{b} is cyclopropyl or -OR^{e}, and R^{e} is methyl, ethyl, n-propyl or isopropyl.

In some embodiments, R² is -C(O)-cyclopropyl or -C(O)OCH₃.

In some embodiments, R³ is hydrogen or alkyl optionally substituted with halogen, hydroxyl, cyano or amino.

In some embodiments, R³ is hydrogen.

In some embodiments, R³ is alkyl optionally substituted with halogen, hydroxyl, cyano or amino.

In certain embodiments, R³ is C₁₋₆ alkyl optionally substituted with halogen, hydroxyl, cyano or amino.

In certain embodiments, R³ is methyl, ethyl, n-propyl, or isopropyl.

In some embodiments, L is selected from the group consisting of a direct bond, alkyl, heteroalkyl, saturated or partially unsaturated cycloalkyl, and saturated or partially unsaturated heterocyclyl, wherein each of alkyl, heteroalkyl, cycloalkyl and heterocyclyl is optionally substituted with one or more R^{f}.

In certain embodiments, each of R^{f} is independently selected from the group consisting of hydrogen, halogen, hydroxyl, amino, alkyl, and heteroalkyl.

In certain embodiments, two R^{f} together with the atom to which they are both attached, form saturated or partially unsaturated cycloalkyl optionally substituted with cyano, halogen, hydroxyl, amino and alkyl.

In some embodiments, L is a direct bond.

In some embodiments, L is alkyl optionally substituted with one or more R^{f}.

In certain embodiments, L is C₁₋₆ alkyl optionally substituted with one or more R^{f}.

In certain embodiments, L is C₁₋₆ alkyl optionally substituted with one or more R^{f}, wherein each of R^{f} is independently selected from the group consisting of hydrogen, halogen, hydroxyl, methyl and ethyl.

In some embodiments, L is -CH₂-, -CH(CH₃)-, or -C(CH₃)₂-.

In some embodiments, W is

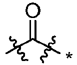

In some embodiments, W is

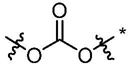

In some embodiments, W is

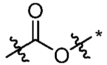

In some embodiments, W is

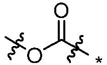

In some embodiments, W is

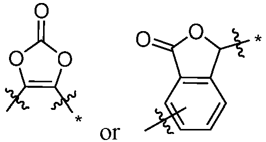

In some embodiments, W is

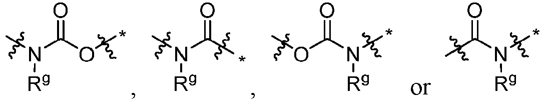

In some embodiments, R^{g} is selected from the group consisting of hydrogen, alkyl and heteroalkyl, wherein each of alkyl and heteroalkyl is optionally substituted with halogen, hydroxyl, cyano, or amino.

In certain embodiments, W is 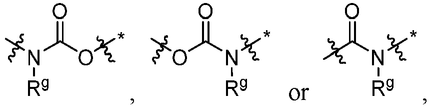 wherein R^{g} is hydrogen or C₁₋₆ alkyl. In certain embodiments, R^{g} is hydrogen, methyl or ethyl.

In some embodiments, R⁴ is hydrogen, alkyl, alkenyl, alkynyl, heteroalkyl, heteroalkenyl, heteroalkynyl or aryl, wherein each of alkyl, alkenyl, alkynyl, heteroalkyl, heteroalkenyl, heteroalkynyl and aryl is optionally substituted with cyano, halogen, hydroxyl, amino, or alkyl.

In certain embodiments, R⁴ is hydrogen, alkyl or aryl optionally substituted with halogen, hydroxyl, cyano or amino.

In certain embodiments, R⁴ is hydrogen, C₁₋₆ alkyl optionally substituted with halogen, hydroxyl, cyano or amino, C₆₋₁₂ aryl optionally substituted with halogen, hydroxyl, cyano or amino.

In certain embodiments, R⁴ is hydrogen, -CH₃, -CH₂CH₃, -CH(CH₃)₂, -C(CH₃)₃, -CH(CH₃)(NH₂), or phenyl.

In some embodiments, 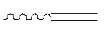 is a double bond in E configuration.

In some embodiments, 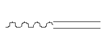 is a double bond in Z configuration.

In a further aspect, the present disclosure provides a compound having a formula selected from the group consisting of: 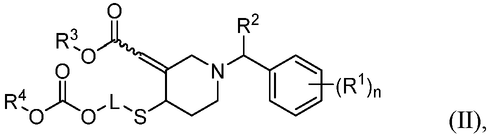 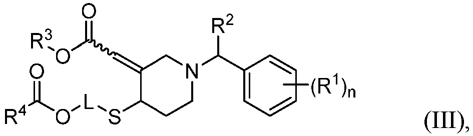 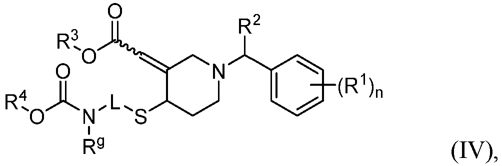 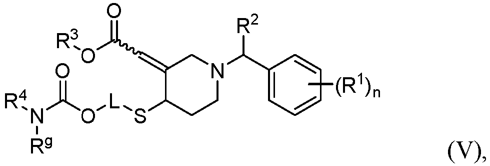 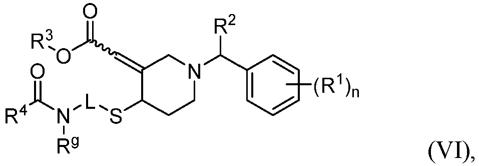 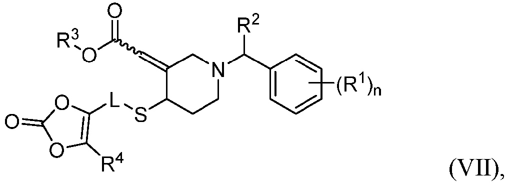 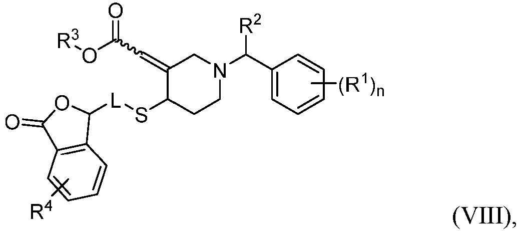 and 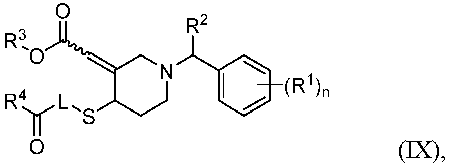 or a pharmaceutically acceptable salt thereof, wherein R¹, R², R³, R⁴, R^{g}, L and n are defined as supra.

In some embodiments, R¹ is halogen. In certain embodiments, R¹ is fluoro, chloro or bromo.

In some embodiments, n is 1, 2 or 3. In certain embodiments, n is 1 or 2. In certain embodiments, n is 1.

In some embodiments, R² is -C(O)R^{b}, wherein R¹ is hydrogen, hydroxyl, alkyl, saturated cycloalkyl or -OR^{e}. In certain embodiments, R² is -C(O)R^{b}, wherein R^{b} is saturated cycloalkyl or -OR^{e}, and R^{e} is alkyl. In certain embodiments, R² is -C(O)R^{b}, wherein R^{b} is saturated C₃₋₆ cycloalkyl or -OR^{e}, and R^{e} is C₁₋₆ alkyl. In certain embodiments, R² is -C(O)R^{b}, wherein R^{b} is cyclopropyl or -OR^{e}, and R^{e} is methyl, ethyl, n-propyl or isopropyl. In certain embodiments, R² is -C(O)-cyclopropyl or -C(O)OCH₃.

In some embodiments, R³ is hydrogen.

In some embodiments, R³ is alkyl. In certain embodiments, R³ is C₁₋₆ alkyl. In certain embodiments, R³ is methyl, ethyl, n-propyl, or isopropyl.

In some embodiments, L is a direct bond.

In some embodiments, L is alkyl optionally substituted with one or more R^{f} independently selected from hydrogen, halogen, hydroxyl, methyl and ethyl. In certain embodiments, L is C₁₋₆ alkyl optionally substituted with one or more R^{f} independently selected from hydrogen, halogen, hydroxyl, methyl and ethyl. In certain embodiments, L is -CH₂-, -CH(CH₃)-, or -C(CH₃)₂-.

In some embodiments, R⁴ is hydrogen or alkyl optionally substituted with halogen, hydroxyl, cyano or amino. In certain embodiments, R⁴ is hydrogen or C₁₋₆ alkyl optionally substituted with halogen, hydroxyl, cyano or amino. In certain embodiments, R⁴ is hydrogen, -CH₃, -CH₂CH₃, -CH(CH₃)₂, -C(CH₃)₃, or -CH(CH₃)(NH₂).

In some embodiments, R^{g} is hydrogen or alkyl. In certain embodiments, R^{g} is hydrogen or C₁₋₆ alkyl. In certain embodiments, R^{g} is hydrogen, methyl, or ethyl. In certain embodiments, R^{g} is hydrogen.

In some embodiments, 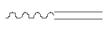 is a double bond in E configuration.

In some embodiments, 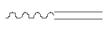 is a double bond in Z configuration.

In a further aspect, the present disclosure provides a compound having a formula selected from the group consisting of: 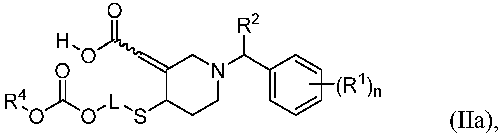 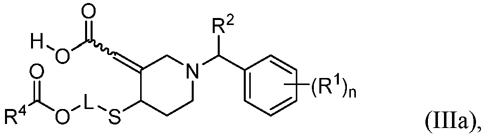 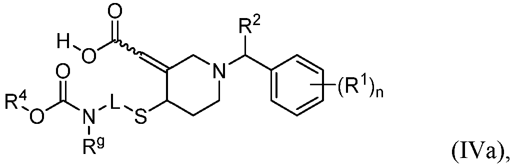 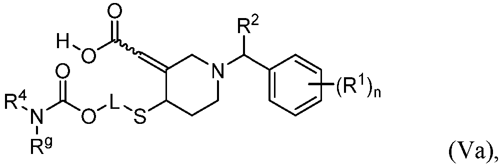 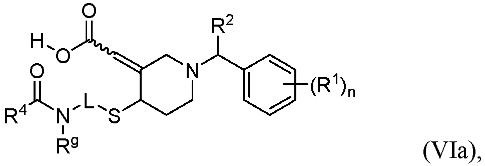 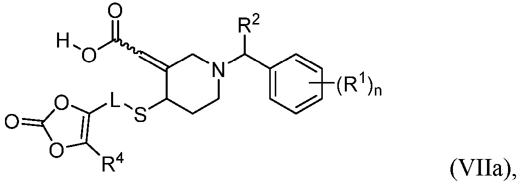 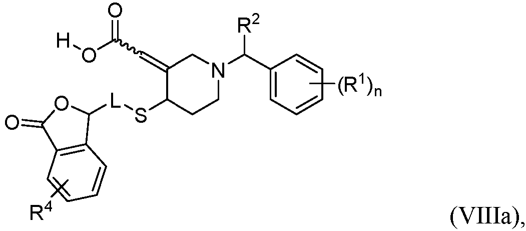 and 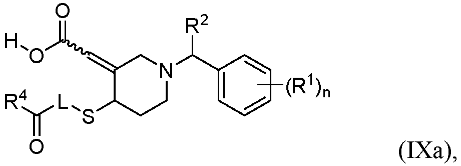 or a pharmaceutically acceptable salt thereof, wherein R¹, R², R⁴, R^{g}, L and n are defined as supra.

In some embodiments, 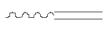 is a double bond in E configuration.

In some embodiments, 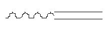 is a double bond in Z configuration.

In a further aspect, the present disclosure provides a compound having a formula selected from the group consisting of: 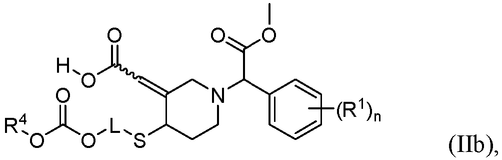 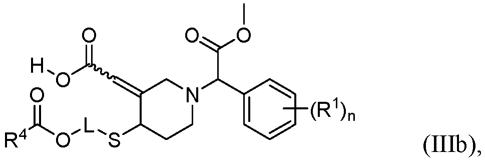 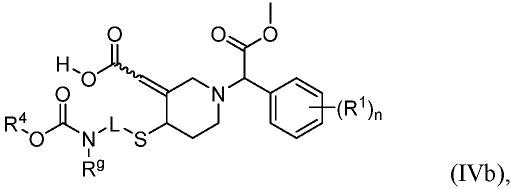 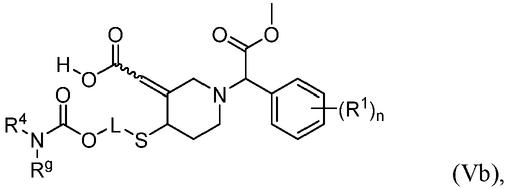 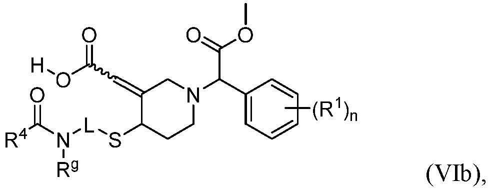 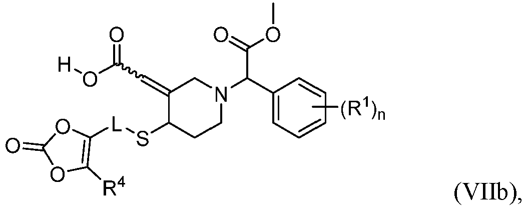 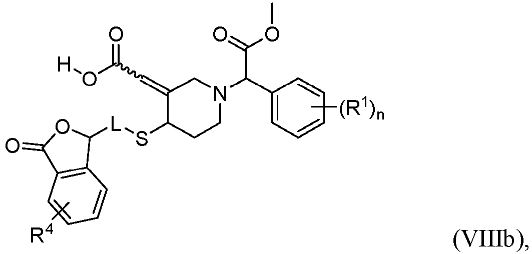 and 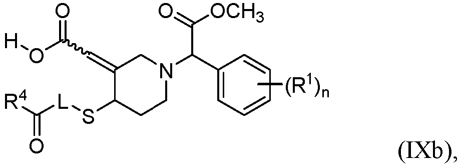 or a pharmaceutically acceptable salt thereof, wherein R¹, R⁴, R^{g}, L and n are defined as supra.

In some embodiments, 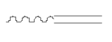 is a double bond in E configuration.

In some embodiments, 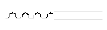 is a double bond in Z configuration.

In a further aspect, the present disclosure provides a compound having a formula selected from the group consisting of: 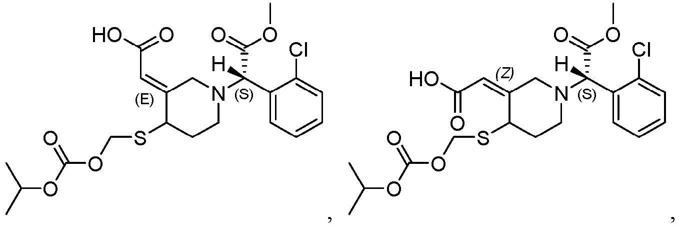 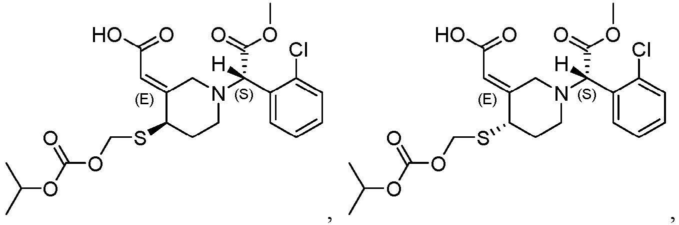 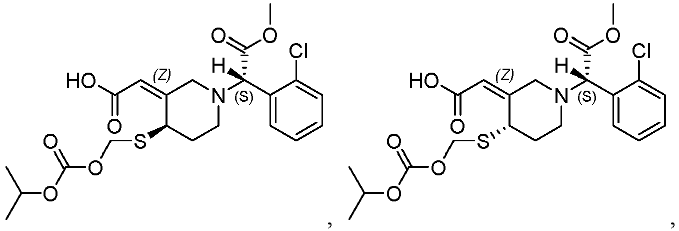 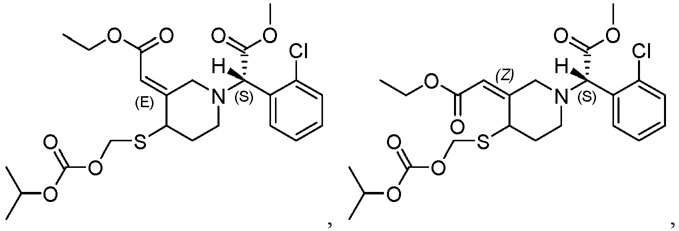 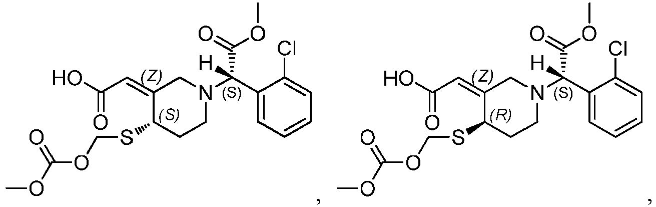 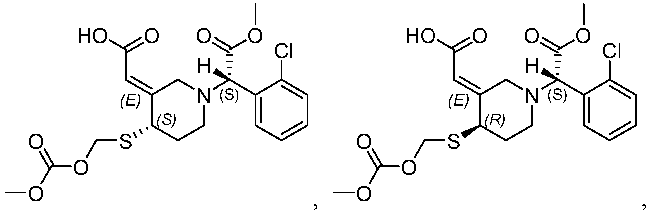 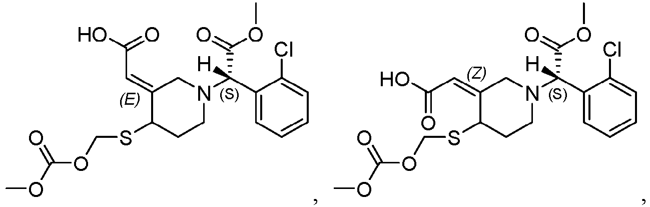 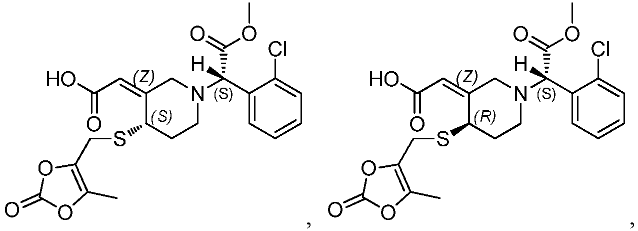 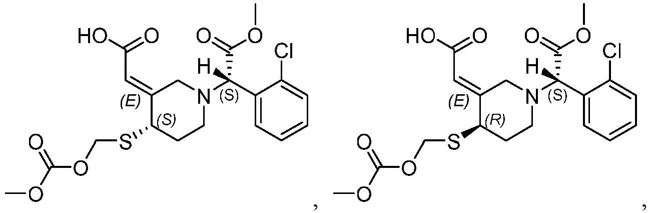 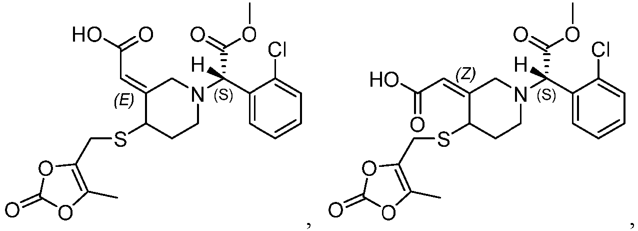 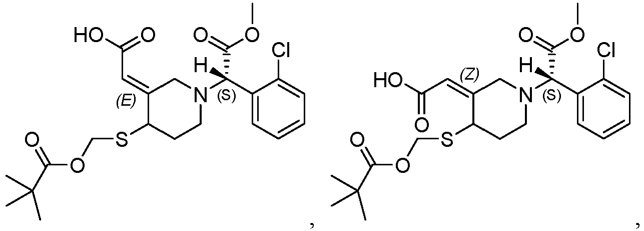 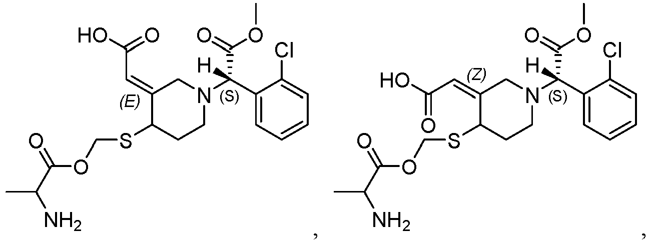 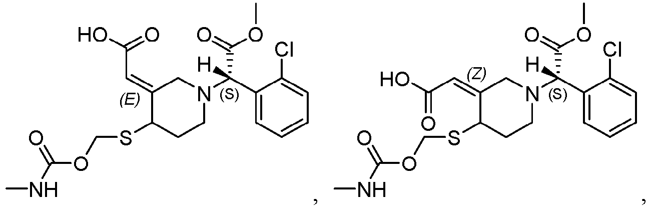 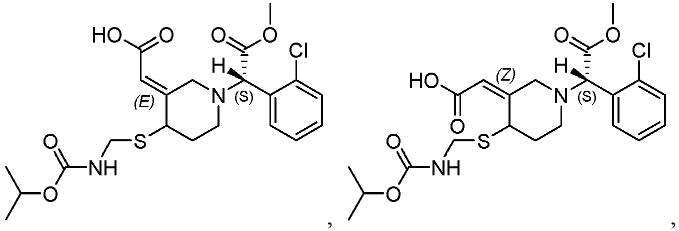 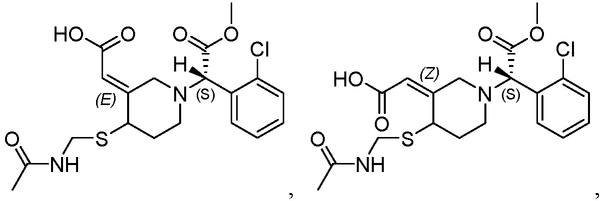 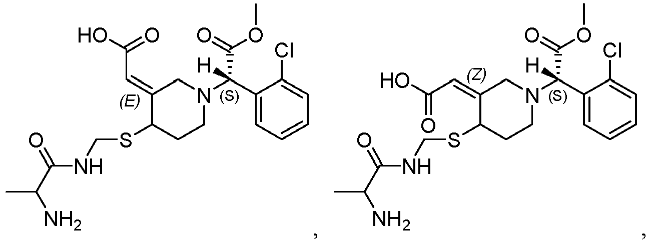 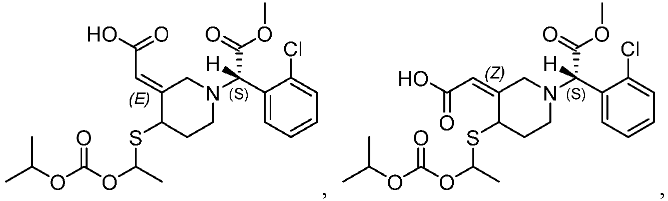 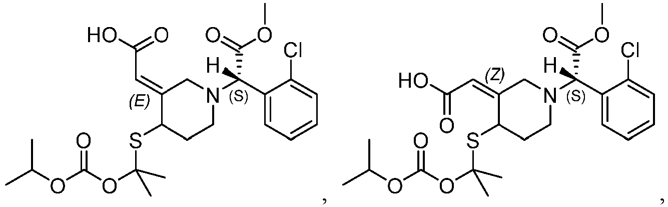 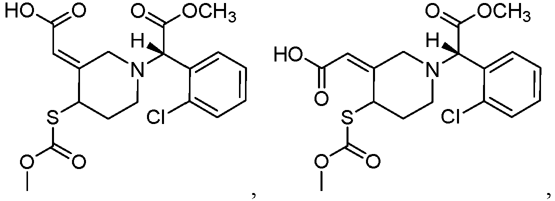 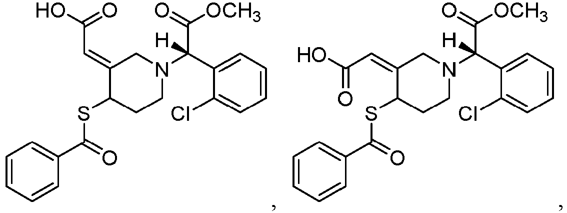 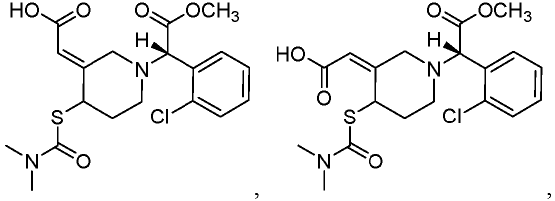 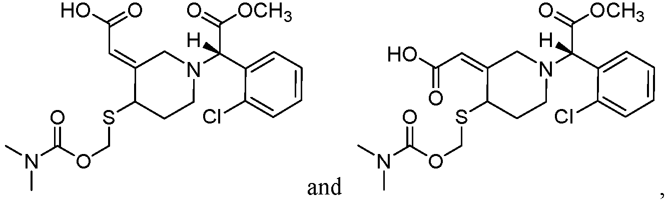 or a pharmaceutically acceptable salt thereof.

Compounds provided herein are described with reference to both generic formulae and specific compounds. In addition, compounds of the present disclosure may exist in a number of different forms or derivatives, all within the scope of the present disclosure. These include, for example, tautomers, stereoisomers, racemic mixtures, regioisomers, salts, solvated forms, amorphous forms, different crystal forms or polymorphs.

The compounds of present disclosure can comprise one or more asymmetric centers depending on substituent selection, and thus can exist in various stereoisomeric forms, e.g., enantiomers and/or diastereomers. For example, the compounds provided herein may have an asymmetric carbon center, and thus compounds provided herein may have either the (R) or (S) stereo-configuration at a carbon asymmetric center. Therefore, compounds of the present disclosure may be in the form of an individual enantiomer, diastereomer or geometric isomer, or may be in the form of a mixture of stereoisomers.

As used herein, the term "enantiomer" refers to two stereoisomers of a compound which are non-superimposable mirror images of one another. The term "diastereomer" refers to a pair of optical isomers which are not mirror images of one another. Diastereomers have different physical properties, e.g. melting points, boiling points, spectral properties, and reactivities.

Where a particular enantiomer is preferred, it may, in some embodiments be provided substantially free of the opposite enantiomer, and may also be referred to as "optically enriched". "Optically enriched", as used herein, means that the compound is made up of a significantly greater proportion of one enantiomer. In certain embodiments, the compound is made up of at least about 90% by weight of a preferred enantiomer. In other embodiments, the compound is made up of at least about 95%, 98%, or 99% by weight of a preferred enantiomer. Preferred enantiomers may be isolated from racemic mixtures by any method known to those skilled in the art, for example by chromatography or crystallization, by the use of stereochemically uniform starting materials for the synthesis or by stereoselective synthesis. Optionally a derivatization can be carried out before a separation of stereoisomers. The separation of a mixture of stereoisomers can be carried out at an intermediate step during the synthesis of a compound provided herein or it can be done on a final racemic product. Absolute stereochemistry may be determined by X-ray crystallography of crystalline products or crystalline intermediates which are derivatized, if necessary, with a reagent containing a stereogenic center of known configuration. Alternatively, absolute stereochemistry may be determined by Vibrational Circular Dichroism (VCD) spectroscopy analysis. See, for example, Jacques, et al., Enantiomers, Racemates and Resolutions (Wiley Interscience, New York, 1981); Wilen, S.H., et al., Tetrahedron 33:2725 (1977); Eliel, E.L. Stereochemistry of Carbon Compounds (McGraw-Hill, NY, 1962); Wilen, S.H. Tables of Resolving Agents and Optical Resolutions p. 268 (E.L. Eliel, Ed., Univ. of Notre Dame Press, Notre Dame, IN 1972).

In some embodiments, mixtures of diastereomers, for example mixtures of diastereomers enriched with 51% or more of one of the diastereomers, including for example 60% or more, 70% or more, 80% or more, or 90% or more of one of the diastereomers are provided.

In some embodiments, compounds provided herein may have one or more double bonds that can exist as either the Z or E isomer, unless otherwise indicated. The present disclosure additionally encompasses the compounds as individual isomers substantially free of other isomers and alternatively, as mixtures of various isomers, e.g., racemic mixtures of enantiomers.

The compounds of the present disclosure may also exist in different tautomeric forms, and all such forms are embraced within the scope of the present disclosure. The term "tautomer" or "tautomeric form" refers to structural isomers of different energies which are interconvertible via a low energy barrier. For example, proton tautomers (also known as prototropic tautomers) include interconversions via migration of a proton, such as keto-enol, amide-imidic acid, lactam-lactim, imine-enamine isomerizations and annular forms where a proton can occupy two or more positions of a heterocyclic system (for example, 1H- and 3H-imidazole, 1H-, 2H- and 4H- 1,2,4-triazole, 1H- and 2H- isoindole, and 1H- and 2H-pyrazole). Valence tautomers include interconversions by reorganization of some of the bonding electrons. Tautomers can be in equilibrium or sterically locked into one form by appropriate substitution. Compounds of the present disclosure identified by name or structure as one particular tautomeric form are intended to include other tautomeric forms unless otherwise specified.

The present disclosure is also intended to include all isotopes of atoms in the compounds. Isotopes of an atom include atoms having the same atomic number but different mass numbers. For example, unless otherwise specified, hydrogen, carbon, nitrogen, oxygen, phosphorous, sulfur, fluorine, chlorine, bromide or iodine in the compounds of present disclosure are meant to also include their isotopes, such as but not limited to ¹H, ²H, ³H, ¹¹C, ¹²C, ¹³C, ¹⁴C, ¹⁴N, ¹⁵N, ¹⁶O, ¹⁷O, ¹⁸O, ³¹P, ³²P, ³²S, ³³S, ³⁴S, ³⁶S, ¹⁷F, ¹⁸F, ¹⁹F, ³⁵Cl, ³⁷Cl, ⁷⁹Br, ⁸¹Br, ¹²⁴I, ¹²⁷I and ¹³¹I. In some embodiments, hydrogen includes protium, deuterium and tritium. In some embodiments, carbon includes ¹²C and ¹³C. Isotopically-enriched compounds of Formula (I) can be prepared without undue experimentation by conventional techniques well known to those skilled in the art or by processes analogous to those described in the Schemes and Examples herein using appropriate isotopically-enriched reagents and/or intermediates.

Compounds of the present disclosure can be formulated as or be in the form of pharmaceutically acceptable salts. Unless specified to the contrary, a compound provided herein includes pharmaceutically acceptable salts of such compound.

As used herein, the term "pharmaceutically acceptable" indicates that the substance or composition is compatible chemically and/or toxicologically, with the other ingredients comprising a formulation, and/or the subjects being treated therewith.

As used herein, the term "pharmaceutically acceptable salt", unless otherwise indicated, includes salts that retain the biological effectiveness of the free acids and bases of the specified compound and that are not biologically or otherwise undesirable. Contemplated pharmaceutically acceptable salt forms include, but are not limited to, mono, bis, tris, tetrakis, and so on. Pharmaceutically acceptable salts are non-toxic in the amounts and concentrations at which they are administered. The preparation of such salts can facilitate the pharmacological use by altering the physical characteristics of a compound without preventing it from exerting its physiological effect. Useful alterations in physical properties include lowering the melting point to facilitate transmucosal administration and increasing the solubility to facilitate administering higher concentrations of the drug.

Pharmaceutically acceptable salts include acid addition salts such as those containing sulfate, chloride, hydrochloride, fumarate, maleate, phosphate, sulfamate, acetate, citrate, lactate, tartrate, methanesulfonate, ethanesulfonate, benzenesulfonate, p-toluenesulfonate, cyclohexylsulfamate and quinate. Pharmaceutically acceptable salts can be obtained from acids such as hydrochloric acid, maleic acid, sulfuric acid, phosphoric acid, sulfamic acid, acetic acid, citric acid, lactic acid, tartaric acid, malonic acid, methanesulfonic acid, ethanesulfonic acid, benzenesulfonic acid, p-toluenesulfonic acid, cyclohexylsulfamic acid, fumaric acid, and quinic acid.

Pharmaceutically acceptable salts also include basic addition salts such as those containing benzathine, chloroprocaine, choline, diethanolamine, ethanolamine, t-butylamine, ethylenediamine, meglumine, procaine, aluminum, calcium, lithium, magnesium, potassium, sodium, ammonium, alkylamine, and zinc, when acidic functional groups, such as carboxylic acid or phenol are present. For example, see Remington's Pharmaceutical Sciences, 19thed., Mack Publishing Co., Easton, PA, Vol. 2, p. 1457, 1995; "Handbook of Pharmaceutical Salts: Properties, Selection, and Use" by Stahl and Wermuth, Wiley-VCH, Weinheim, Germany, 2002. Such salts can be prepared using the appropriate corresponding bases.

Pharmaceutically acceptable salts can be prepared by standard techniques. For example, the free-base form of a compound can be dissolved in a suitable solvent, such as an aqueous or aqueous-alcohol solution containing the appropriate acid and then isolated by evaporating the solution. Thus, if the particular compound is a base, the desired pharmaceutically acceptable salt may be prepared by any suitable method available in the art, for example, treatment of the free base with an inorganic acid, such as hydrochloric acid, hydrobromic acid, sulfuric acid, nitric acid, phosphoric acid and the like, or with an organic acid, such as acetic acid, maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an alpha-hydroxy acid, such as citric acid or tartaric acid, an amino acid, such as aspartic acid or glutamic acid, an aromatic acid, such as benzoic acid or cinnamic acid, a sulfonic acid, such as p-toluenesulfonic acid or ethanesulfonic acid, or the like.

Similarly, if the particular compound is an acid, the desired pharmaceutically acceptable salt may be prepared by any suitable method, for example, treatment of the free acid with an inorganic or organic base, such as an amine (primary, secondary or tertiary), an alkali metal hydroxide or alkaline earth metal hydroxide, or the like. Illustrative examples of suitable salts include organic salts derived from amino acids, such as L-glycine, L-lysine, and L-arginine, ammonia, primary, secondary, and tertiary amines, and cyclic amines, such as hydroxyethylpyrrolidine, piperidine, morpholine or piperazine, and inorganic salts derived from sodium, calcium, potassium, magnesium, manganese, iron, copper, zinc, aluminum and lithium.

It is also to be understood that the compounds of present disclosure can exist in unsolvated forms, solvated forms (e.g., hydrated forms), and solid forms (e.g., crystal or polymorphic forms), and the present disclosure is intended to encompass all such forms.

As used herein, the term "solvate" or "solvated form" refers to solvent addition forms that contain either stoichiometric or non-stoichiometric amounts of solvent. Some compounds have a tendency to trap a fixed molar ratio of solvent molecules in the crystalline solid state, thus forming a solvate. If the solvent is water the solvate formed is a hydrate; and if the solvent is alcohol, the solvate formed is an alcoholate. Hydrates are formed by the combination of one or more molecules of water with one molecule of the substance in which the water retains its molecular state as H₂O. Examples of solvents that form solvates include, but are not limited to, water, isopropanol, ethanol, methanol, DMSO, ethyl acetate, acetic acid, and ethanolamine.

As used herein, the terms "crystal form", "crystalline form", "polymorphic forms" and "polymorphs" can be used interchangeably, and mean crystal structures in which a compound (or a salt or solvate thereof) can crystallize in different crystal packing arrangements, all of which have the same elemental composition. Different crystal forms usually have different X-ray diffraction patterns, infrared spectral, melting points, density hardness, crystal shape, optical and electrical properties, stability and solubility. Recrystallization solvent, rate of crystallization, storage temperature, and other factors may cause one crystal form to dominate. Crystal polymorphs of the compounds can be prepared by crystallization under different conditions.

The present disclosure is also intended to include all isotopes of atoms in the compounds. Isotopes of an atom include atoms having the same atomic number but different mass numbers. For example, unless otherwise specified, hydrogen, carbon, nitrogen, oxygen, phosphorous, sulfur, fluorine, chlorine, bromide or iodine in the compounds of present disclosure are meant to also include their isotopes, such as but not limited to ¹H, ²H, ³H, ¹¹C, ¹²C, ¹³C, ¹⁴C, ¹⁴N, ¹⁵N, ¹⁶O, ¹⁷O, ¹⁸O, ³¹P, ³²P, ³²S, ³³S, ³⁴S, ³⁶S, ¹⁷F, ¹⁸F, ¹⁹F, ³⁵Cl, ³⁷Cl, ⁷⁹Br, ⁸¹Br, ¹²⁴I, ¹²⁷I and ¹³¹I. In some embodiments, hydrogen includes protium, deuterium and tritium. In some embodiments, carbon includes ¹²C and ¹³C.

### Synthesis of compounds

Synthesis of the compounds provided herein, including pharmaceutically acceptable salts thereof, are illustrated in the synthetic schemes in the examples. The compounds provided herein can be prepared using any known organic synthesis techniques and can be synthesized according to any of numerous possible synthetic routes, and thus these schemes are illustrative only and are not meant to limit other possible methods that can be used to prepare the compounds provided herein. Additionally, the steps in the Schemes are for better illustration and can be changed as appropriate. The embodiments of the compounds in examples were synthesized for the purposes of research and potentially submission to regulatory agencies.

The reactions for preparing compounds of the present disclosure can be carried out in suitable solvents, which can be readily selected by one skilled in the art of organic synthesis. Suitable solvents can be substantially non-reactive with the starting materials (reactants), the intermediates, or products at the temperatures at which the reactions are carried out, e.g. temperatures that can range from the solvent's freezing temperature to the solvent's boiling temperature. A given reaction can be carried out in one solvent or a mixture of more than one solvent. Depending on the particular reaction step, suitable solvents for a particular reaction step can be selected by one skilled in the art.

Preparation of compounds of the present disclosure can involve the protection and deprotection of various chemical groups. The need for protection and deprotection, and the selection of appropriate protecting groups, can be readily determined by one skilled in the art. The chemistry of protecting groups can be found, for example, in T. W. Greene and P. G. M. Wuts, Protective Groups in Organic Synthesis, 3rd Ed., Wiley & Sons, Inc., New York (1999), in P. Kocienski, Protecting Groups, Georg Thieme Verlag, 2003, and in Peter G.M. Wuts, Greene's Protective Groups in Organic Synthesis, 5th Edition, Wiley, 2014, all of which are incorporated herein by reference in its entirety.

Reactions can be monitored according to any suitable method known in the art. For example, product formation can be monitored by spectroscopic means, such as nuclear magnetic resonance spectroscopy (e.g.¹H or ¹³C), infrared spectroscopy, spectrophotometry (e.g. UV-visible), mass spectrometry, or by chromatographic methods such as high performance liquid chromatography (HPLC), liquid chromatography-mass spectroscopy (LCMS), or thin layer chromatography (TLC). Compounds can be purified by one skilled in the art by a variety of methods, including high performance liquid chromatography (HPLC) ("Preparative LC-MS Purification: Improved Compound Specific Method Optimization" Karl F. Blom, Brian Glass, Richard Sparks, Andrew P. Combs J. Combi. Chem. 2004, 6(6), 874-883, which is incorporated herein by reference in its entirety), and normal phase silica chromatography.

The known starting materials of the present disclosure can be synthesized by using or according to the known methods in the art, or can be purchased from commercial suppliers. Unless otherwise noted, analytical grade solvents and commercially available reagents were used without further purification.

Unless otherwise specified, the reactions of the present disclosure were all done under a positive pressure of nitrogen or argon or with a drying tube in anhydrous solvents, and the reaction flasks were typically fitted with rubber septa for the introduction of substrates and reagents via syringe. Glassware was oven dried and/or heat dried.

For illustrative purposes, the Examples section below shows synthetic route for preparing the compounds of the present disclosure as well as key intermediates. Those skilled in the art will appreciate that other synthetic routes may be used to synthesize the inventive compounds. Although specific starting materials and reagents are depicted, other starting materials and reagents can be easily substituted to provide a variety of derivatives and/or reaction conditions. In addition, many of the compounds prepared by the methods described below can be further modified in light of this disclosure using conventional chemistry well known to those skilled in the art.

### Cyclodextrin

The pharmaceutical composition of the present disclosure comprises a cyclodextrin. The term "cyclodextrin" refers to a cyclic molecule containing 6 or more α-D-glucopyranose units bonded at the 1,4 position. The cyclodextrin containing 6 sugar units is α-cyclodextrin, the cyclodextrin containing 7 sugar units is β-cyclodextrin, and the cyclodextrin containing 8 sugar units is γ-cyclodextrin. Cyclodextrin derivatives are cyclodextrins in which some -OH groups are modified to be -OR, wherein each R is independently alkyl, hydroxyalkyl, glucosyl, maltosyl, cycloalkylalkyl, or -(CH₂)₄SO₃⁻Na⁺. As used herein, the term "cyclodextrin" is intended to include cyclodextrins and derivatives thereof. Examples of alkyl derivatives of cyclodextrins include, but are not limited to, dimethyl-α-cyclodextrin, dimethyl-β-cyclodextrin, and dimethyl-γ-cyclodextrin. Examples of hydroxyalkyl derivatives of cyclodextrins include, but are not limited to, 2-hydroxypropyl-α-cyclodextrin, 2-hydroxypropyl-β-cyclodextrin, and 2-hydroxypropyl-γ-cyclodextrin. Examples of sulfoalkyl ether derivatives of cyclodextrins include, but are not limited to, sulfobutyl ether-α-cyclodextrin, sulfobutyl ether-β-cyclodextrin, and sulfobutyl ether-γ-cyclodextrin. Examples of glycosyl derivatives of cyclodextrins include, but are not limited to, glucosyl-α-cyclodextrin, glucosyl-β-cyclodextrin, glucosyl-γ-cyclodextrin, maltosyl-α-cyclodextrin, maltosyl-β-cyclodextrin, and maltosyl-γ-cyclodextrin.

Cyclodextrins that can be used in the present disclosure include α-cyclodextrin, β-cyclodextrin, and γ-cyclodextrin.

In some embodiments, the cyclodextrin is β-cyclodextrin.

In some embodiments, the cyclodextrin is β-cyclodextrin selected from sulfobutyl ether-β-cyclodextrin (SBECD), cyclobutyl alkyl ether-β-cyclodextrin, or hydroxypropyl-β-cyclodextrin (HPCD).

### Buffer

The pharmaceutical composition of the present disclosure may optionally comprise a buffer. The term "buffer" refers to an agent known to be safely used in a pharmaceutical formulation and having the ability to maintain or control the pH of the formulation within the desired range.

In some embodiments, the pharmaceutical composition of the present disclosure comprises an acidic buffer for adjusting the pH of the pharmaceutical composition to be within about 3 to about 4. Examples of suitable acidic buffers include, but are not limited to, inorganic acids (e.g., hydrochloric acid, sulfuric acid, phosphoric acid, hydrobromic acid, etc.) and organic acids (e.g., oxalic acid, maleic acid, fumaric acid, lactic acid, malic acid, tartaric acid, citric acid, benzoic acid, acetic acid, methanesulfonic acid, toluenesulfonic acid, benzenesulfonic acid, ethanesulfonic acid, etc.). Acid salts of the acids described above may also be used.

In some embodiments, the acidic buffer used in the pharmaceutical composition of the present disclosure is selected from phosphoric acid, hydrochloric acid, succinic acid, acetic acid, tartaric acid, lactic acid, citric acid, malic acid, or glycolic acid. In certain embodiments, the acidic buffer used in the pharmaceutical composition of the present disclosure is citric acid.

### pH adjuster

The pharmaceutical composition of the present disclosure may optionally comprise a pH adjuster. The term "pH adjuster" refers to an adjuster used in the art to adjust the pH. The pH adjuster includes an acidic pH adjuster and a basic pH adjuster.

As used herein, the "acidic pH adjuster" refers to a compound that is capable of providing a proton (H⁺) under the Bronsted-Lowry definition or that is an electron-pair acceptor under the Lewis definition. Examples of acids include, but are not limited to, alkanoic acid or carboxylic acid, sulfonic acid, and inorganic acids. Examples of alkanoic acid include, but are not limited to, formic acid, acetic acid, citric acid, lactic acid, oxalic acid, succinic acid, tartaric acid, malic acid, glycolic acid, and the like. Examples of inorganic acids include, but are not limited to, hydrogen halide (hydrofluoric acid, hydrochloric acid, hydrobromic acid, etc.), halogen oxyacid (hypochlorous acid, perchloric acid, etc.), sulfuric acid, nitric acid, phosphoric acid, chromic acid, boric acid, and the like. Examples of sulfonic acid include, but are not limited to, methanesulfonic acid, benzenesulfonic acid, p-toluenesulfonic acid, trifluoromethanesulfonic acid, camphorsulfonic acid, and the like. Other acids are known to those skilled in the art.

As used herein, the "basic pH adjuster" refers to a compound that is capable of accepting a proton (H⁺) under the Bronsted-Lowry definition or an electron-pair donor under the Lewis definition. Examples of Bronsted-Lowry bases include, but are not limited to, hydroxides of alkali metal or alkaline earth metal, such as lithium hydroxide, sodium hydroxide, potassium hydroxide, rubidium hydroxide, cesium hydroxide, magnesium hydroxide, strontium hydroxide, barium hydroxide, and the like. Examples of Lewis bases include, but are not limited to, amines (e.g., ammonia, trimethylamine, triethylamine, diisopropylethylamine, 1,8-diazabicycloundec-7-ene, 2,6-di-tert-butylpyridine, quinuclidine, and lithium diisopropylamide) and nucleophilic bases (e.g., butyllithium). Other bases are known to those skilled in the art.

In some embodiments, the pH adjuster is HCl. In some embodiments, the pH adjuster is NaOH. In some embodiments, the pH adjuster is NaOH and HCl.

### Pharmaceutical composition

The present disclosure provides a pharmaceutical composition comprising the compound of formula (I) or the pharmaceutically acceptable salt thereof of the present disclosure and a cyclodextrin.

In some embodiments, the pharmaceutical composition comprises the following compound:

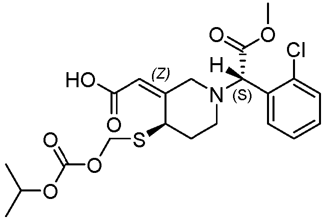

In some embodiments, the cyclodextrin is β-cyclodextrin. In certain embodiments, the β-cyclodextrin is sulfobutyl ether-β-cyclodextrin, cyclobutyl alkyl ether-β-cyclodextrin, or hydroxypropyl-β-cyclodextrin. In certain embodiments, the β-cyclodextrin is sulfobutyl ether-β-cyclodextrin or hydroxypropyl-β-cyclodextrin. In certain embodiments, the β-cyclodextrin is sulfobutyl ether-β-cyclodextrin. In certain embodiments, the β-cyclodextrin is hydroxypropyl-β-cyclodextrin.

### Preparation for injection

Prior to the present application, the difficulty in preparing an injectable formulation of the compound or the pharmaceutically acceptable salt thereof provided herein is unknown, and the type of the solubilizer used to obtain an effective injectable formulation comprising the compound or the pharmaceutically acceptable salt thereof provided herein is unknown. The present inventors have surprisingly found that the use of cyclodextrin can help to obtain an effective injectable formulation of the compound or the pharmaceutically acceptable salt thereof provided herein. By mixing the compound or the pharmaceutically acceptable salt thereof provided herein with cyclodextrin, the resulting formulation not only has good solubility and stability at pH 3-4, but also has remarkably improved drug effect and intracellular exposure.

In some embodiments, the pharmaceutical composition of the present disclosure is a solution, which may also be referred to as an injectable formulation.

In certain embodiments, the pharmaceutical composition of the present disclosure comprises water to form an injectable formulation. The water may be any suitable water, such as distilled water or safe water for injection. In certain embodiments, the water may be safe water for injection.

In some embodiments, the injectable formulation comprises 0.1-10 mg/mL of the compound of formula (I) or the pharmaceutically acceptable salt thereof. In some embodiments, the content of the compound of formula (I) or the pharmaceutically acceptable salt thereof in the injectable formulation is any value within the above range. For example, as required, the content of the compound of formula (I) or the pharmaceutically acceptable salt thereof in the injectable formulation may be at least 0.1 mg/mL, at least 0.2 mg/mL, at least 0.3 mg/mL, at least 0.4 mg/mL, at least 0.5 mg/mL, at least 0.6 mg/mL, at least 0.7 mg/mL, at least 0.8 mg/mL, at least 0.9 mg/mL, at least 1 mg/mL, at least 1.2 mg/mL, at least 1.2 mg/mL, at least 1.3 mg/mL, at least 1.4 mg/mL, at least 1.5 mg/mL, at least 1.6 mg/mL, at least 1.7 mg/mL, at least 1.8 mg/mL, at least 1.9 mg/mL, at least 2 mg/mL, at least 2 mg/mL, at least 3 mg/mL, at least 4 mg/mL, at least 5 mg/mL, at least 6 mg/mL, at least 7 mg/mL, at least 8 mg/mL, at least 9 mg/mL, or at least 10 mg/mL, and at most 10 mg/mL, at most 9 mg/mL, at most 8 mg/mL, at most 7 mg/mL, at most 6 mg/mL, at most 5 mg/mL, at most 4 mg/mL, at most 3 mg/mL, at most 2 mg/mL, at most 1 mg/mL, or at most 0.9 mg/mL.

In some embodiments, the injectable formulation comprises 0.2-10 mg/mL, 0.3-5 mg/mL, 0.4-2 mg/mL, 0.5-1 mg/mL, or 0.6-0.9 mg/mL of the compound of formula (I) or the pharmaceutically acceptable salt thereof.

In some embodiments, the injectable formulation comprises 20-400 mg/mL of cyclodextrin. In some embodiments, the content of cyclodextrin in the injectable formulation is any value within the above range. For example, as required, the content of cyclodextrin in the injectable formulation may be at least 20 mg/mL, at least 25 mg/mL, at least 30 mg/mL, at least 35 mg/mL, at least 40 mg/mL, at least 45 mg/mL, at least 50 mg/mL, at least 60 mg/mL, at least 70 mg/mL, at least 80 mg/mL, at least 90 mg/mL, at least 100 mg/mL, at least 110 mg/mL, at least 120 mg/mL, at least 130 mg/mL, at least 140 mg/mL, at least 150 mg/mL, at least 160 mg/mL, at least 170 mg/mL, at least 180 mg/mL, at least 190 mg/mL, at least 200 mg/mL, at least 210 mg/mL, at least 220 mg/mL, at least 230 mg/mL, at least 240 mg/mL, or at least 250 mg/mL, and at most 400 mg/mL, at most 390 mg/mL, at most 380 mg/mL, at most 370 mg/mL, at most 360 mg/mL, at most 350 mg/mL, at most 340 mg/mL, at most 330 mg/mL, at most 320 mg/mL, at most 310 mg/mL, at most 300 mg/mL, at most 290 mg/mL, at most 280 mg/mL, at most 270 mg/mL, at most 260 mg/mL, at most 250 mg/mL, at most 240 mg/mL, at most 230 mg/mL, at most 220 mg/mL, at most 210 mg/mL, at most 200 mg/mL, at most 190 mg/mL, at most 180 mg/mL, at most 170 mg/mL, at most 160 mg/mL, at most 150 mg/mL, at most 140 mg/mL, at most 130 mg/mL, at most 120 mg/mL, at most 110 mg/mL, or at most 100 mg/mL. In some embodiments, the injectable formulation comprises 30-300 mg/mL, 40-300 mg/mL, 50-250 mg/mL, 60-200 mg/mL, 70-150 mg/mL, or 80-100 mg/mL of cyclodextrin.

In some embodiments, the weight ratio of the cyclodextrin to the compound of formula (I) or the pharmaceutically acceptable salt thereof is 50:1 to 400:1, wherein the weight of the pharmaceutically acceptable salt of the compound of formula (I) is based on the weight of the compound of formula (I) contained therein. In some embodiments, the weight ratio of the cyclodextrin to the compound of formula (I) or the pharmaceutically acceptable salt thereof is any value within the above range. For example, as required, the weight ratio of the cyclodextrin to the compound of formula (I) or the pharmaceutically acceptable salt thereof may be at least 50:1, at least 60:1, at least 70:1, at least 80:1, at least 90:1, at least 100:1, at least 110:1, at least 120:1, at least 130:1, at least 140:1, at least 150:1, at least 160:1, at least 170:1, at least 180:1, at least 190:1, at least 200:1, at least 210:1, at least 220:1, at least 230:1, at least 240:1, or at least 250:1, and at most 400:1, at most 390:1, at most 380:1, at most 370:1, at most 360:1, at most 350:1, at most 340:1, at most 330:1, at most 320:1, at most 310:1, at most 300:1, at most 290:1, at most 280:1, at most 270:1, at most 260:1, at most 250:1, at most 240:1, at most 230:1, at most 220:1, at most 210:1, at most 200:1, at most 190:1, at most 180:1, at most 170:1, at most 160:1, at most 150:1, at most 140:1, at most 130:1, or at most 120:1, wherein the weight of the pharmaceutically acceptable salt of the compound of formula (I) is based on the weight of the compound of formula (I) contained therein.

In some embodiments, the weight ratio of the cyclodextrin to the compound of formula (I) or the pharmaceutically acceptable salt thereof may be 50:1 to 300:1, 50:1 to 250:1, 60:1 to 250:1, 70:1 to 200:1, 80:1 to 150:1, or 90:1 to 120:1, wherein the weight of the pharmaceutically acceptable salt of the compound of formula (I) is based on the weight of the compound of formula (I) contained therein.

In some embodiments, the injectable formulation further comprises a buffer. In some embodiments, the buffer is an acidic buffer. In certain embodiments, the acidic buffer may be selected from phosphoric acid, hydrochloric acid, succinic acid, acetic acid, tartaric acid, lactic acid, citric acid, malic acid, glycolic acid, or hydrates thereof. In certain embodiments, the acidic buffer is citric acid or a hydrate thereof. In certain embodiments, the acidic buffer is citric acid monohydrate. In certain embodiments, the injectable formulation comprises 1-3 mg/mL of citric acid monohydrate, e.g., 1-2.8 mg/mL, 1-2.6 mg/mL, 1-2.4 mg/mL, 1-2.2 mg/mL, 1-2 mg/mL, 1-1.9 mg/mL, 1-1.8 mg/mL, 1-1.7 mg/mL, 1-1.6 mg/mL, 1-1.5 mg/mL, 1-1.4 mg/mL, 1-1.3 mg/mL, 1-1.2 mg/mL, or 1-1.1 mg/mL of citric acid monohydrate.

In some embodiments, the injectable formulation has a pH of 3-4. The pH of the injectable formulation may be any pH within those pH ranges listed above, such as 3.1, 3.2, 3.3, 3.4, 3.5, 3.6, 3.7, 3.8, 3.9, or 4.0. In some embodiments, the injectable formulation has a pH of 3.5-4. In some embodiments, the injectable formulation has a pH of 3.5. In some embodiments, the injectable formulation has a pH of 4.

If necessary, the pH may be adjusted by adding a pH adjuster to allow the pH to be within the above pH range. For example, an acidic pH adjuster and/or a basic pH adjuster may be added to adjust the pH. In some embodiments, the pH adjuster is an inorganic acid, e.g., hydrogen halide, such as HCl. In some embodiments, the basic pH adjuster is an alkali metal hydroxide or an alkaline earth metal hydroxide, e.g., an alkali metal hydroxide, such as NaOH. In some embodiments, NaOH and HCl may be added to adjust the pH of the injectable formulation, thus allowing the pH to be within the above pH range.

In some embodiments, the injectable formulation comprises:
0.4-2 mg/mL of the compound of formula (I) or the pharmaceutically acceptable salt thereof (wherein the weight of the pharmaceutically acceptable salt of the compound of formula (I) is based on the weight of the compound of formula (I) contained therein);
40-250 mg/mL of a cyclodextrin;
optionally, a buffer;
optionally, a pH adjuster; and
water,
the injectable formulation having a pH of 3-4.

In some embodiments, the injectable formulation comprises:
0.5-1 mg/mL of the compound of formula (I) or the pharmaceutically acceptable salt thereof (wherein the weight of the pharmaceutically acceptable salt of the compound of formula (I) is based on the weight of the compound of formula (I) contained therein);
50-150 mg/mL of a cyclodextrin;
optionally, a buffer;
optionally, a pH adjuster; and
water,
the injectable formulation having a pH of 3-4.

In some embodiments, the injectable formulation comprises:
0.6-0.9 mg/mL of the compound of formula (I) or the pharmaceutically acceptable salt thereof (wherein the weight of the pharmaceutically acceptable salt of the compound of formula (I) is based on the weight of the compound of formula (I) contained therein);
60-120 mg/mL of a cyclodextrin;
optionally, a buffer;
optionally, a pH adjuster; and
water,
the injectable formulation having a pH of 3-4.

In some embodiments, the injectable formulation comprises:
0.6-0.9 mg/mL of the compound of formula (I) or the pharmaceutically acceptable salt thereof (wherein the weight of the pharmaceutically acceptable salt of the compound of formula (I) is based on the weight of the compound of formula (I) contained therein);
70-110 mg/mL of a cyclodextrin;
optionally, a buffer;
optionally, a pH adjuster; and
water,
the injectable formulation having a pH of 3-4.

In some embodiments, the injectable formulation comprises:
0.7-0.9 mg/mL of the compound of formula (I) or the pharmaceutically acceptable salt thereof (wherein the weight of the pharmaceutically acceptable salt of the compound of formula (I) is based on the weight of the compound of formula (I) contained therein);
80-100 mg/mL of a cyclodextrin;
optionally, a buffer;
optionally, a pH adjuster; and
water,
the injectable formulation having a pH of 3-4.

In some embodiments, the injectable formulation comprises a buffer. In certain embodiments, the injectable formulation comprises an acidic buffer. In certain embodiments, the injectable formulation comprises citric acid monohydrate. In certain embodiments, the injectable formulation comprises 1-2.5 mg/mL of citric acid monohydrate. In certain embodiments, the injectable formulation comprises 1-2.3 mg/mL of citric acid monohydrate. In certain embodiments, the injectable formulation comprises 1-2.1 mg/mL of citric acid monohydrate.

In some embodiments, the weight ratio of the cyclodextrin to the compound or the pharmaceutically acceptable salt thereof in the injectable formulation is 80:1 to 150:1, 90:1 to 120:1, or 100:1 to 120:1, wherein the weight of the pharmaceutically acceptable salt of the compound is based on the weight of the compound contained therein.

In some embodiments, the injectable formulation may also optionally comprise common additives used in formulations, such as other solubilizers or cosolvents, such as poloxamer, propylene glycol, PEG400, tween 80, polyoxyethylene sorbitan monolaurate, and the like; isotonic agents, such as potassium chloride, sodium chloride, glucose, glycerol, mannitol, sorbitol, and the like; stabilizers, such as sorbol, sodium edetate, and the like; and antioxidants, such as glycine, ascorbic acid, sodium citrate, and the like.

The amount of the compound of formula (I) or the pharmaceutically acceptable salt thereof in the injectable formulations of the present disclosure may be measured after storing the formulations under various conditions. In some embodiments, the amount of the compound of formula (I) or the pharmaceutically acceptable salt thereof is measured after storing the formulation at 5 °C. In some embodiments, the amount of the compound of formula (I) or the pharmaceutically acceptable salt thereof is measured after storing the injectable formulation at 15 °C. In some embodiments, the amount of the compound of formula (I) or the pharmaceutically acceptable salt thereof is measured after storing the injectable formulation at 25 °C. In some embodiments, the amount of the compound of formula (I) or the pharmaceutically acceptable salt thereof is measured after storing the injectable formulation at 40 °C. In some embodiments, the amount of the compound of formula (I) or the pharmaceutically acceptable salt thereof is measured after storing the injectable formulation for 1 h, 2 h, 3 h, or 4 h.

### Lyophilized formulation

The pharmaceutical composition of the present disclosure may also be a lyophilized composition, which may also be referred to as a lyophilized formulation.

The lyophilized formulation of the present disclosure comprises the compound of formula (I) or the pharmaceutically acceptable salt thereof and a cyclodextrin. The compound of formula (I) or the pharmaceutically acceptable salt thereof may include compounds of formula (I) or pharmaceutically acceptable salts thereof in various forms. For example, the compound of formula (I) or the pharmaceutically acceptable salt thereof may be amorphous or crystalline, or a mixture thereof. In some embodiments, the lyophilized formulation of the present disclosure comprises an amorphous compound of formula (I) or a pharmaceutically acceptable salt thereof and a cyclodextrin.

The lyophilized formulation of the present disclosure may be contained in any suitable container, such as a sealed vial. In some embodiments, the present disclosure provides a sealed vial containing the lyophilized formulation.

The lyophilized formulation of the present disclosure may be obtained by lyophilizing the injectable formulation of the present disclosure.

### Preparation method for pharmaceutical composition

When the pharmaceutical composition of the present disclosure is formulated, the compound of formula (I) or the pharmaceutically acceptable salt thereof is added to an acidic cyclodextrin solution, the pH is increased, and then a certain amount of water for injection is added to bring the total volume to a desired volume, so that the time for formulating the liquid can be reduced, and the solubility and the stability of the resulting pharmaceutical composition can be improved.

In some embodiments, the present disclosure provides a method for preparing the pharmaceutical composition of the present disclosure, which comprises:
mixing the cyclodextrin with water to form a first mixture;
optionally, adding a buffer to the first mixture to form a second mixture;
adding an acidic pH adjuster to the second mixture to adjust the pH to 1-1.5, thus forming a third mixture;
adding the compound of formula (I) or the pharmaceutically acceptable salt thereof to the third mixture to form a fourth mixture;
adding a basic pH adjuster to the fourth mixture to adjust the pH to 3-4, thus forming a fifth mixture; and
optionally, adding water for injection to the fifth mixture to form the pharmaceutical composition.

In some embodiments, the method for preparing the pharmaceutical composition of the present disclosure comprises the step of adding a buffer to the first mixture to form a second mixture. In certain embodiments, the buffer is citric acid, such as citric acid monohydrate.

In some embodiments, the acidic pH adjuster is HCl. In some embodiments, the basic pH adjuster is NaOH.

In some embodiments, the pharmaceutical composition may be sterilely filtered through a filter membrane (e.g., a 0.22 µm filter membrane) and filled into vials. The vials are sealed and finally sterilized.

In some embodiments, those skilled in the art may formulate the pharmaceutical composition of the present disclosure as a solution used directly or as a pre-concentrate that is diluted before use.

In some embodiments, the pharmaceutical composition may be further lyophilized, thereby forming a lyophilized composition.

### Use of pharmaceutical composition

The compound of formula (I) or the pharmaceutically acceptable salt thereof provided herein is capable of inhibiting platelet agglomeration and is therefore suitable for being used as therapeutic agents or prophylactic agents for various thrombotic diseases.

As used herein, the term "therapy" is intended to have its normal meaning of dealing with a disease in order to entirely or partially relieve one, some or all of its symptoms, or to correct or compensate for the underlying pathology, thereby achieving beneficial or desired clinical results. For purposes of this disclosure, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Therapy" can also mean prolonging survival as compared to expected survival if not receiving it. Those in need of therapy include those already with the condition or disorder as well as those prone to have the condition or disorder or those in which the condition or disorder is to be prevented. The term "therapy" also encompasses prophylaxis unless there are specific indications to the contrary. The terms "therapeutic" and "therapeutically" should be interpreted in a corresponding manner.

The term "treatment" is used synonymously with "therapy". Similarly the term "treat" can be regarded as "applying therapy" where "therapy" is as defined herein.

As used herein, the term "prophylaxis" is intended to have its normal meaning and includes primary prophylaxis to prevent the development of the disease and secondary prophylaxis whereby the disease has already developed and the patient is temporarily or permanently protected against exacerbation or worsening of the disease or the development of new symptoms associated with the disease.

In some embodiments, the compounds of the present disclosure can convert to active thiol-metabolite after administration. For a prodrug of the active thiol-metabolite, it can be desirable that the prodrug remains stable (resistance to environment), while converts to the active thiol-metabolite in the target tissue with high conversion rate. Further, it can be desirable that the prodrug has a fast onset of action and thus a low loading dose and low side effect, a high solubility in aqueous solution which allows for formulating into injection for first aids and surgeries.

In some embodiments, the compounds provided herein can undergo a hydrolysis process via hydrolase to form the active thiol-metabolite. Due to the high in vivo activity and the wide spread of hydrolase in intestine, liver and plasma, the compounds provided herein can convert to the active thiol-metabolite in vivo at a higher conversion rate and less interpatient variability, thereby providing a fast onset of antiplatelet action without the need to use a high loading dose. In addition, since the metabolism of the compound provided herein is mediated by hydrolase rather than CYP enzymes, use of these compounds is not limited by potential interactions with other CYP-targeted drugs.

As used herein, the terms "thiol metabolite", "thiol active metabolite", and "active thiol metabolite" are used interchangeably and refer to compound H4 described above.

In some embodiments, the compounds provided herein show a faster onset of antiplatelet action than clopidogrel at the same dose. In some embodiments, the compounds provided herein show an onset of antiplatelet action less than that of clopidogrel at a dose lower than that of clopidogrel. In some embodiments, at a dose 2-fold lower than that of clopidogrel, the compounds provided herein show an onset of antiplatelet action less than that of clopidogrel. In some embodiments, at a dose 3-fold lower than that of clopidogrel, the compounds provided herein show an onset of antiplatelet action less than that of clopidogrel. In some embodiments, at a dose 4-fold lower than that of clopidogrel, the compounds provided herein shows an onset of antiplatelet action less than that of clopidogrel. In some embodiments, at a dose 5-fold lower than that of clopidogrel, the compounds provided herein shows an onset of antiplatelet action less than that of clopidogrel.

In some embodiments, at a dose 5-fold lower than that of clopidogrel, the compounds provided herein show an onset of antiplatelet action of less than 120 minutes, less than 110 minutes, less than 100 minutes, less than 90 minutes, less than 80 minutes, less than 70 minutes, less than 60 minutes, less than 50 minutes, less than 40 minutes, or even less than 30 minutes.

The improved solubility of the compound or the pharmaceutically acceptable salt thereof provided herein provides an opportunity to expand the use of the compound in inhibiting platelet agglomeration. In some embodiments, the compound or the pharmaceutically acceptable salt thereof provided herein can be formulated into injection administration for first aid and surgery.

Accordingly, the pharmaceutical composition comprising the compound of formula (I) or the pharmaceutically acceptable salt thereof provided herein is used for injection administration for first aid and surgery.

The mode of administration of the pharmaceutical composition of the present disclosure is not particularly limited, and the pharmaceutical composition may be administered to a subject by a suitable mode depending on the age, sex, disease degree, etc., of the subject. For example, the pharmaceutical composition of the present disclosure may be administered intravenously, intra-arterially, intramuscularly, intradermally, subcutaneously, intraspinally, or intraperitoneally alone. In some embodiments, the pharmaceutical composition of the present disclosure is injected intramuscularly. In some embodiments, the pharmaceutical composition of the present disclosure is injected intravenously. In some embodiments, the pharmaceutical composition of the present disclosure is injected subcutaneously. In addition, the pharmaceutical composition of the present disclosure may be mixed with other injections and administered in the form of a mixture of the pharmaceutical composition of the present disclosure and other injections. There are no particular limitations on other injections that can be used, and commercially available injections can be used, such as glucose injection, xylitol injection, D-mannitol injection, fructose injection, normal saline, dextran 40 injection, dextran 70 injection, amino acid injection, Ringer's solution, lactic acid-Ringer's solution, and the like.

The composition of the present disclosure may be directly used via injection in the form of an injectable formulation or diluted before use and then used via injection, or may be in the form of a lyophilized formulation and used after reconstitution.

As used herein, the "reconstitution" comprises dissolving the lyophilized composition using water for injection and then mixing the resulting solution with other injections, or mixing the lyophilized composition directly with injections.

In some embodiments, the present disclosure provides a method for treating a vascular disease in a subject in need thereof, which comprises administering to the subject a therapeutically effective amount of the pharmaceutical composition of the present disclosure.

In certain embodiments, the vascular disease is selected from atherothrombosis, ischemia, stroke, cerebral thrombosis, artery thrombosis, thrombotic cerebrovascular diseases, cardiovascular diseases, and blood clot.

In other embodiments, the present disclosure provides a method for inhibiting platelet aggregation in a subject in need thereof, which comprises administering to the subject a therapeutically effective amount of the pharmaceutical composition of the present disclosure.

### Examples

### I. Compound

For the purpose of illustration, the following examples are included. However, it is to be understood that these examples do not limit the present disclosure and are only meant to suggest a method of practicing the present disclosure. Persons skilled in the art will recognize that the chemical reactions described may be readily adapted to prepare a number of other compounds of the present disclosure, and alternative methods for preparing the compounds of the present disclosure are deemed to be within the scope of the present disclosure. For example, the synthesis of non-exemplified compounds according to the present disclosure may be successfully performed by modifications apparent to those skilled in the art, e.g., by appropriately protecting interfering groups, by utilizing other suitable reagents and building blocks known in the art other than those described, and/or by making routine modifications of reaction conditions. Alternatively, other reactions disclosed herein or known in the art will be recognized as having applicability for preparing other compounds of the present disclosure.

### Synthetic example 1

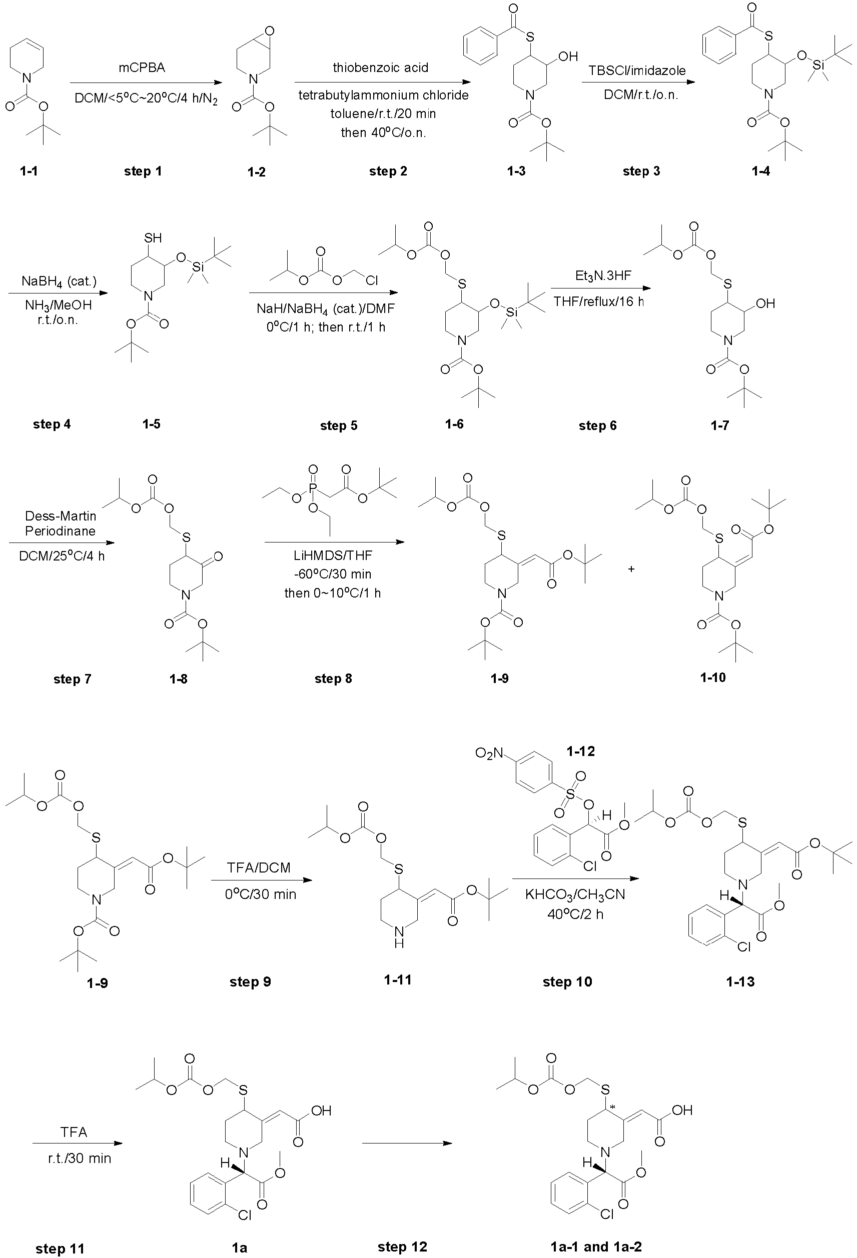

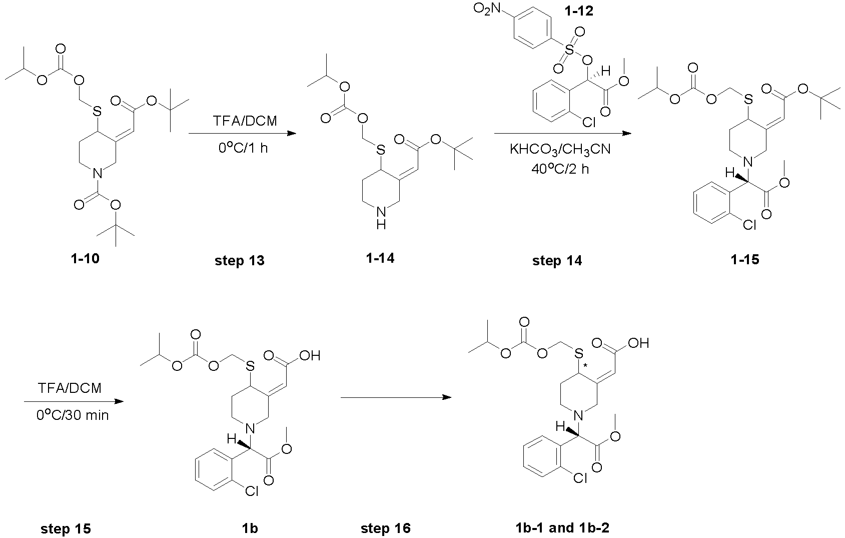

### Step 1. Synthesis of 1-2

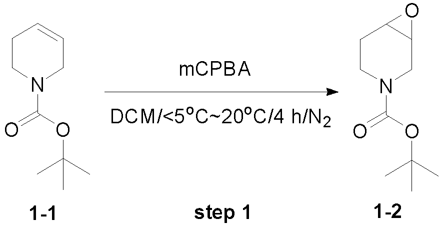

A solution of 1-1 (56.7 g, 310 mmol) in DCM (500 mL) was stirred in an ice-bath (T< 5°C) under N₂ protection. *m*CPBA (107.0 g, 620 mmol) was added in portions to the above solution. After addition, the resulting mixture was stirred at 20°C for 4 h. The mixture was poured into a solution of Na₂S₂O₃ (90.0 g) and NaHCO₃ (45.0 g) in water (300 mL) with stirring. The resulting mixture was extracted with DCM (300 mL*2). The combined organic layer was dried over Na₂SO₄, and filtered. The filtrate was concentrated and the residue was purified by silica gel chromatography (Petroleum Ether/EtOAc = 100/1 to 5/1) to give 1-2 (67.0 g, 98% yield) as yellow oil.

### Step 2. Synthesis of 1-3

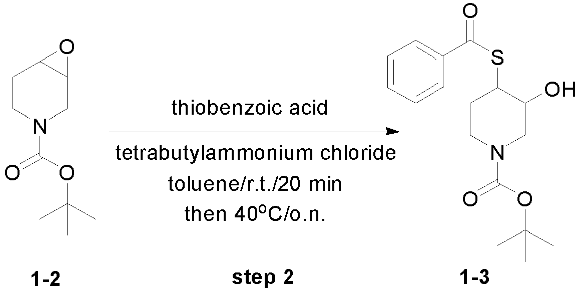

A mixture of **1-2** (67.0 g, 337 mmol), thiobenzoic acid (56.7 g, 370 mmol) and tetrabutylammonium chloride (4.67 g, 17 mmol) in toluene (300 mL) was stirred at r.t. for 20 min and then at 40°C overnight. Then the reaction mixture was concentrated under vacuum. To the residue was added sat. Na₂CO₃ (400 mL) with stirring, which was then extracted with EtOAc (400 mL*2). The organic layer was dried over Na₂SO₄, and filtered. The filtrate was concentrated and the residue was purified by silica gel chromatography (Petroleum Ether/EtOAc = 10/1 to 5/1) to give **1-3** (87.5 g, 77% yield) as a white solid.

### LC-MS [M+1-100]⁺ = 238.1

¹H NMR (400 MHz, Chloroform-*d*) δ 7.97 (d, *J=* 7.2 Hz, 2H), 7.59 (s, 1H), 7.46 (t, *J* = 7.7 Hz, 2H), 4.24 (d, *J* = 16.3 Hz, 1H), 4.17-3.81 (m, 1H), 3.73 (s, 1H), 3.60 (s, 1H), 2.92 (t, *J* = 24.3 Hz, 2H), 2.72 (s, 1H), 2.12 (d, *J* = 16.8 Hz, 1H), 1.71 (d, *J =* 11.6 Hz, 1H), 1.46 (s, 9H).

### Step 3. Synthesis of 1-4

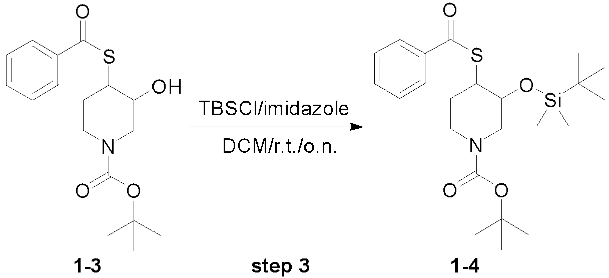

To a solution of **1-3** (157.0 g, 467.3 mmol) in DCM (1.5 L) were added TBSCl (141.2 g, 935 mmol), and imidazole (159.0 g, 2.34 mol). The resulting mixture was stirred at r.t. for overnight and then concentrated under reduce pressure. The residue was purified by silica gel chromatography (Petroleum ether/EtOAc = 10/1) to give **1-4** (273.0 g, 94% yield) as a white solid. LC-MS [M+1-100]⁺ = 352.1.

### Step 4. Synthesis of 1-5

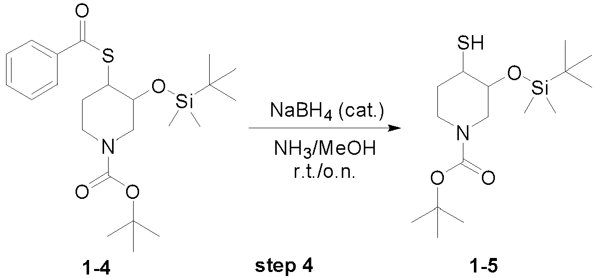

To a solution of 1-4 (263.0 g, 583.1 mmol) in NH₃/MeOH (7M , 2.0 L) was added NaBH₄ (222 mg, 5.8 mmol). The resulting mixture was stirred at r.t. for overnight and then concentrated under reduce pressure. The residue was purified by silica gel chromatography (Petroleum ether/EtOAc = 20/1) to give 1-5 (210.0 g, 100% yield) as light yellow oil.

### Step 5. Synthesis of 1-6

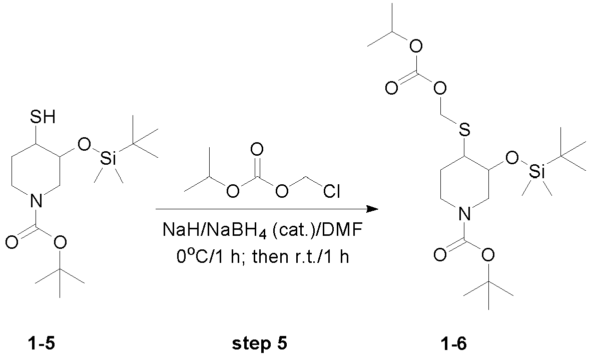

To a solution of NaBH₄ (1.1 g, 28.8 mmol) in DMF (1.0 L) was added NaH (20.7 g, 864.6 mmol) at 0°C under N₂ with stirring. 1-5 (200.0 g, 576.4 mmol) was added dropwise at 0°C, which was then stirred at 0°C for 1 h. Then, chloromethyl isopropyl carbonate (100.7 g, 662.8 mmol) was added at 0°C and the resulting mixture was stirred at r.t. for 1 h. H₂O (1.0 L) was added to the mixture, which was then extracted with EtOAc (1.0 L*3). The organic layer was washed with brine, dried over Na₂SO₄, and filtered. The filtrate was concentrated under vacuum and the residue was purified by silica gel chromatography (Petroleum/EtOAc = 20/1) to give **1-6** (135.0 g, 50% yield) as colorless oil.

¹H NMR (400 MHz, Chloroform-*d*) δ 5.25 (q, *J=* 12.0 Hz, 2H), 4.92-4.79 (m, 1H), 3.85 (d, *J* = 58.8 Hz, 2H), 3.45 (s, 1 H), 2.97-2.74 (m, 3H), 2.13-2.02 (m, 1H), 1.59-1.47 (m, 1H), 1.41 (s, 9H), 1.25 (dd, *J=* 15.5, 4.6 Hz, 6H), 0.89 (s, 9H), 0.19-0.01 (m, 6H).

### Step 6. Synthesis of 1-7

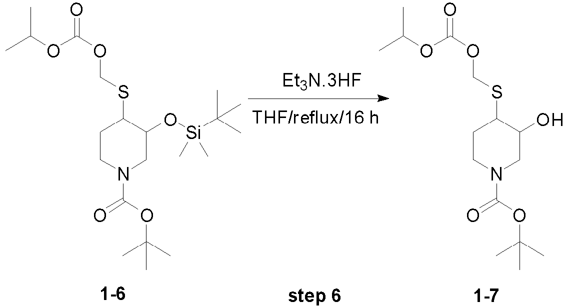

To a solution of 1-6 (129.0 g, 278.6 mmol) in THF (1.1 L) was added Et₃N.3HF (135.0 g, 835.9 mmol), which was stirred under reflux for 16 h. After completion, the reaction mixture was concentrated under reduce pressure, and the residue was purified by silica gel chromatography (Petroleum ether/EtOAc = 3/1 ) to give 1-7 (80.0 g, 82% yield) as light yellow oil.

### Step 7. Synthesis of 1-8

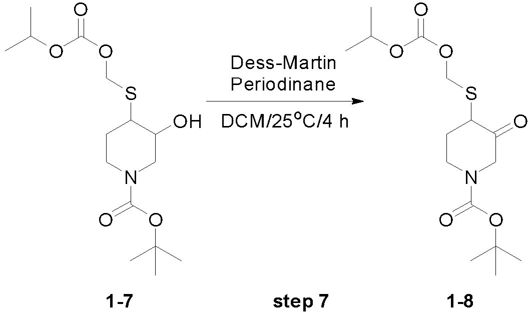

To a solution of **1-7** (30.0 g, 86.9 mmol) in DCM (300 mL) was added Dess-Martin Periodinane (72.9 g, 171.9 mmol), and the resulting mixture was stirred at 25°C for 4 h. After completion, the above reaction mixture was added to a mixed solution of sat. Na₂S₂O₃/sat. NaHCO₃ (600 mL/600 mL), which was then extracted with EtOAc (400 mL*2). The combined organic layers was washed with sat. NaHCO₃, dried over Na₂SO₄, and filtered. The filtrate was concentrated under reduce pressure and the residue was purified by silica gel chromatography (Petroleum ether/EtOAc = 20/1 to 8/1) to give **1-8** (22.0 g, 73% yield) as colorless oil.

### Step 8. Synthesis of 1-9 and 1-10

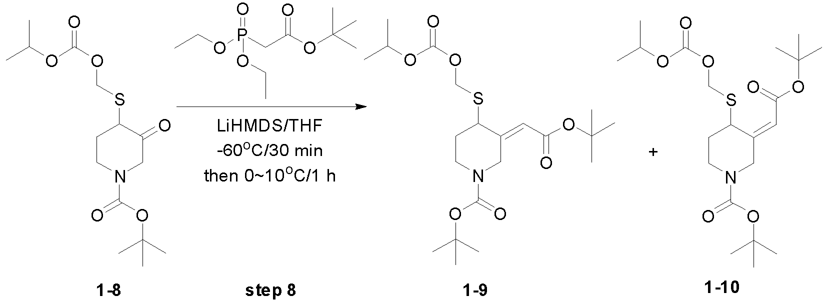

To a solution of *tert*-butyl 2-(diethoxyphosphoryl)acetate (11.4 g, 43.2 mmol) in THF (100 mL) was added LiHMDS (37.4 mL, 37.4 mmol) at -60°C under N₂, which was stirred at -60°C for 30 min. **1-8** (10.0 g, 28.8 mmol) was then added dropwise at -60°C, and the resulting mixture was stirred at 0~10°C for 1 h. Then the reaction mixture was added to sat. NH₄Cl (300 mL), which was then extracted with EtOAc (150 mL*2). The combined organic layer was washed with brine, dried over Na₂SO₄ and filtered. The filtrate was concentrated under vacuum and the residue was purified silica gel chromatography (Petroleum ether/EtOAc = 60/1) to give **1-9** (2.5 g, 19% yield) as light yellow oil and **1-10** (1.3 g, 10% yield) as light yellow oil.

**1-9:** ¹H NMR (400 MHz, chloroform-*d*) δ 5.68 (s, 1H), 5.47 (d, *J* = 15.2 Hz, 1H), 5.24 (s, 1H), 4.89-4.94 (m, 2H), 3.95 (s, 1H), 3.87-3.92 (m, 1H), 3.79 (s, 1H), 3.17-3.19 (m, 1H), 2.15-2.16 (m, 1H), 1.87-1.90 (m, 1H), 1.48 (s, 9H), 1.43 (s, 9H), 1.29 (d, *J=* 4 Hz, 6H).

**1-10:** ¹HNMR (400 MHz, chloroform-*d*) δ 5.74 (s, 1H), 5.48 (s, 1H), 5.27 (d, *J=* 12 Hz, 1H), 5.14 (d, *J* = 12 Hz, 1H), 4.85-4.89 (m, 1H), 4.25-4.26 (m, 1H), 3.93-3.94 (m, 2H), 3.15 (s, 1H), 2.01-2.04 (m, 1H), 1.85-1.88 (m, 1H), 1.46 (s, 9H), 1.44 (s, 9H), 1.28 (d, *J=* 4 Hz, 6H).

### Step 9. Synthesis of 1-11

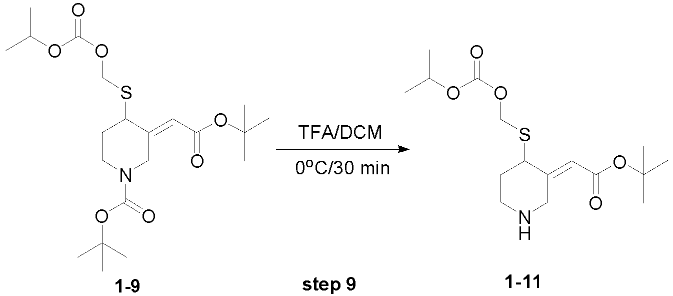

To a solution of **1-9** (3.0 g, 6.7 mmol) in DCM (20 mL) was added TFA (10 mL) at 0°C, then the reaction was stirred at 0°C for 30 min. After completion, the reaction mixture was added to a solution of sat. NaHCO₃ (100 mL), which was then extracted with DCM (100 mL). The organic layer was dried over Na₂SO₄, and filtered. The filtrate was concentrated by reduce pressure to give crude **1-11** (3.0 g, >100 % yield) as yellow oil, which was used in the in the next step without further purification. LC-MS [M+1]⁺ = 346.1

### Step 10. Synthesis of 1-13

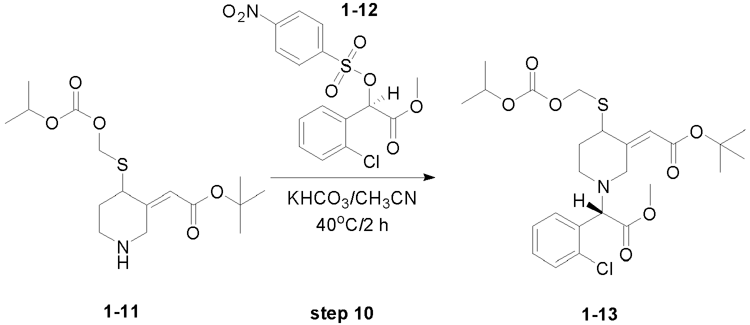

To a solution of **1-11** (3.0 g, crude) in CH₃CN (15 mL) were added **1-12** (2.6 g, 6.7 mmol), and KHCO₃ (1.35 g, 13.5 mmol). The resulting mixture was stirred at 40°C for 2 h. After completion, the reaction mixture was concentrated under reduce pressure and the residue was purified by reversed-phase column chromatography (C18, CH₃CN/H₂O = 80/20) to give **1-13** (1.8 g, 51% yield) as a white solid. LC-MS [M+1]⁺ = 528.2.

### Steps 11 and 12. Synthesis of 1a-1 and 1a-2

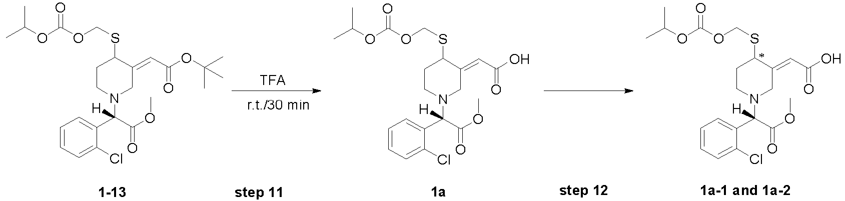

A solution of **1-13** (1.8 g, 3.4 mmol) in TFA (10 mL) was stirred at r.t. for 30 min. After completion, the reaction mixture was added to a solution of sat. NaHCO₃ (100 mL), which was then extracted with EtOAc (100 mL*3). The combined organic layer was washed with sat. NaHCO₃, dried over Na₂SO₄ and filtered. The filtrate was concentrated under reduce pressure and the residue was purified by reversed-phase column chromatography (C18, CH₃CN/H₂O = 80/20) to give **1a** (550 mg, 34% yield). **1a** was purified by chiral column chromatography to give **1a-1** and **1a-2.**

### 1a:

### LC-MS [M+1]⁺ = 472.1

¹H NMR (400 MHz, Chloroform-*d*) δ 7.59 (s, 1H), 7.38 (d, *J =* 4 Hz, 1H), 7.32-7.26 (m, 2H), 5.86 (s, 1H), 5.22 (dd, *J =* 12.2, 2.6 Hz, 1H), 5.00-4.83 (m, 3H), 4.50 (dd, *J =* 66.2, 11.9 Hz, 1H), 3.82 (s, 1H), 3.70 (d, *J =* 4.9 Hz, 3H), 3.52 (dd, *J =* 37.9, 12.9 Hz, 1H), 2.92-2.64 (m, 2H), 2.45-2.30 (m, 1 H), 1.95-1.84 (m, 1H), 1.30 (d, *J =* 6.2 Hz, 6H).

### 1a-1:

¹H NMR (400 MHz, CDCl₃) δ 7.65 (s, 1H), 7.46 - 7.43 (m, 1H), 7.33 (dd, *J =* 6.3, 2.7 Hz, 2H), 5.91 (s, 1H), 5.27 (d, *J =* 12.3 Hz, 1H), 5.04 - 4.87 (m, 3H), 4.49 (d, *J =* 13.7 Hz, 1H), 3.88 (s, 1H), 3.75 (s, 3H), 3.58 (d, *J =* 14.0 Hz, 1H), 2.87 (s, 2H), 2.44 (s, 1H), 1.95 (dd, *J* = 14.2, 3.3 Hz, 1H), 1.35 (d, *J =* 6.2 Hz, 6H).

### 1a-2:

¹H NMR (400 MHz, CDCl₃) δ 7.63 (s, 1H), 7.44 (dt, *J =* 8.2, 3.1 Hz, 1H), 7.35 - 7.31 (m, 2H), 5.92 (s, 1H), 5.25 (d, *J =* 12.3 Hz, 1H), 5.07 (s, 1H), 4.94 (td, *J =* 12.5, 6.5 Hz, 2H), 4.68 (d, *J =* 13.4 Hz, 1H), 3.87 (s, 1H), 3.76 (s, 3H), 3.50 (d, *J =* 13.4 Hz, 1H), 2.90 (s, 1H), 2.75 (d, *J =* 12.3 Hz, 1H), 2.44 (s, 1H), 1.96 (d, *J =* 13.2 Hz, 1H), 1.34 (d, *J =* 6.3 Hz, 6H).

### Step 13. Synthesis of 1-14

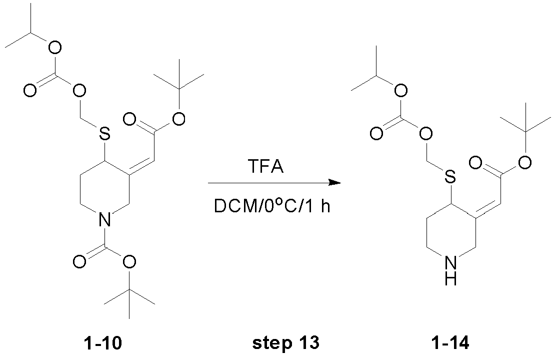

To a solution of **1-10** (1.8 g, 4.0 mmol) in DCM (10 mL) was added TFA (5 mL) at 0°C, which was stirred at 0°C for 1 h. After completion, the reaction mixture was added to a solution of sat. NaHCO₃ (100 mL), which was then extracted with DCM (100 mL*3). The combined organic layer was dried over Na₂SO₄, and filtered. The filtrate was concentrated under reduce pressure to give crude **1-14** (2.0 g, >100% yield) as yellow oil, which was used in the next step without further purification. LC-MS [M+1]⁺ = 346.1

### Step 14. Synthesis of 1-15

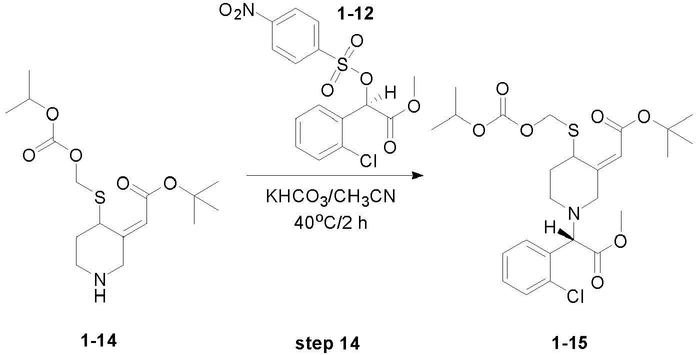

To a solution of **1-14** (2.0 g, crude) in CH₃CN (20 mL) were added **1-12** (1.5 g, 4.0 mmol), and KHCO₃ (800 mg, 8.0 mmol). The resulting mixture was stirred at 40°C for 2 h and then concentrated under reduce pressure. The residue was purified by reversed-phase column chromatography (C18, CH₃CN/H₂O = 80/20) to give **1-15** (500 mg, 24% yield) as a white solid.

### LC-MS [M+1]⁺ = 528.2

### Steps 15 and 16. Synthesis of 1b-1 and 1b-2

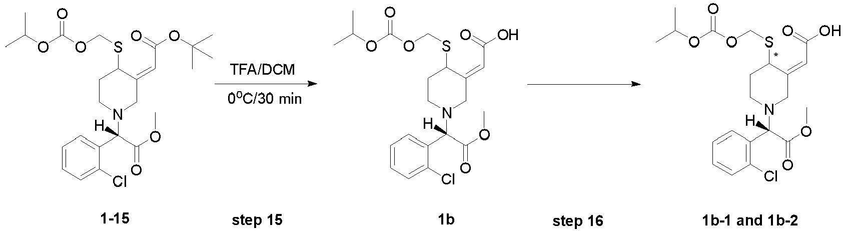

To a solution of **1-15** (500 mg, 0.95 mmol) in DCM (2 mL) at 0°C was added TFA (3 mL), which was stirred at 0°C for 30 min. After completion, the reaction was added to a solution of sat. NaHCO₃ (30 mL) which was then extracted with EtOAc (30 mL*3). The combined organic layer was washed with sat. NaHCO₃, dried over Na₂SO₄, and filtered. The filtrate was concentrated under reduce pressure and the residue was purified by reversed-phase column chromatography (C18, CH₃CN/H₂O = 80/20), followed by prep-HPLC (Mobile Phase: A (H₂O) / B (MeCN); Range of ratio: A/B (80%/20%) to A/B (55%/45%) 10 min and to A/B (20%/80%) 35 min; Rt of Peak: (67% of B); V = 80 mL/min, wavelength 214 nm), and prep-TLC (DCM/MeOH = 10/1) to give **1b** (50 mg, 11% yield). **1b** was purified by chiral column chromatography to give **1b-1** and **1b-2.**

### 1b:

### LC-MS [M+1]⁺ = 472.1

¹H NMR (400 MHz, CDCl₃) δ 7.58 (d, *J =* 5.6 Hz, 1H), 7.44-7.36 (m, 1H), 7.27 (s, 2H), 5.77-5.65 (m, 1H), 5.41 (s, 1H), 5.25 (dd, *J =* 12.0, 6.3 Hz, 1H), 5.19-5.11 (m, 1H), 4.92-4.83 (m, 1H), 4.80 (s, 1H), 3.70 (d, *J =* 4.6 Hz, 3H), 3.52 (dd, *J =* 34.3, 12.2 Hz, 1H), 3.19 (d, *J =* 12.9 Hz, 0.5H), 2.98 (d, *J =* 12.5 Hz, 0.5H), 2.90-2.84 (m, 0.5 H), 2.78-2.61 (m, 1.5 H), 2.31-2.16 (m, 1 H), 1.97-1.82 (m, 1H), 1.28 (d, *J =* 5.4 Hz, 6H).

### 1b-1:

¹H NMR (400 MHz, CDCl₃) δ 7.60-7.57 (m, 1H), 7.41-7.39 (m, 1H), 7.33 - 7.26 (m, 2H), 5.64 (s, 1H), 5.41 (s, 1H), 5.25 (d, *J =* 12.1 Hz, 1H), 5.14 (d, *J =* 12.0 Hz, 1H), 4.91 - 4.81 (m, 1H), 4.79 (s, 1H), 3.69 (s, 3H), 3.47 (d, *J =* 12.4 Hz, 1H), 2.97 (d, *J =* 12.5 Hz, 1H), 2.86 (d, *J =* 10.6 Hz, 1H), 2.72 (dd, *J =* 22.5, 10.6 Hz, 1H), 2.29-2.20 (m, 1H), 1.92 (d, *J =* 14.3 Hz, 1H), 1.27 (d, *J* = 6.2 Hz, 6H).

### 1b-2:

¹H NMR (400 MHz, CDCl₃) δ 7.62 - 7.56 (m, 1H), 7.41-7.38 (m, 1H), 7.30 - 7.26 (m, 2H), 5.77 (s, 1H), 5.43 (s, 1H), δ 5.27 (d, *J =* 12.1 Hz, 1H), 5.17 (d, *J =* 12.0 Hz, 1H), 4.91-4.86 (m, 1H), 4.80 (s, 1H), 3.71 (s, 3H), 3.56 (d, *J =* 12.4 Hz, 1H), 3.17 (d, *J =* 12.4 Hz, 1H), 2.66 (d, *J =* 8.0 Hz, 2H), 2.20-2.17 (m, 1H), 1.87 (d, *J* = 14.4 Hz, 1H), 1.29 (dd, *J =* 6.2, 2.5 Hz, 6H).

### Synthetic example 2

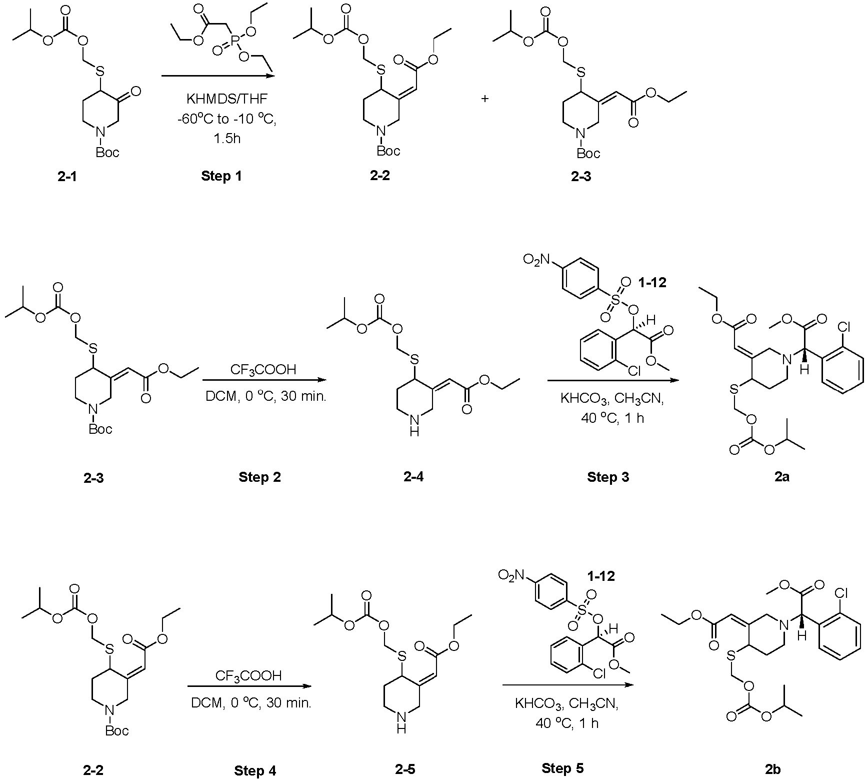

### Step 1. Synthesis of 2-2 and 2-3

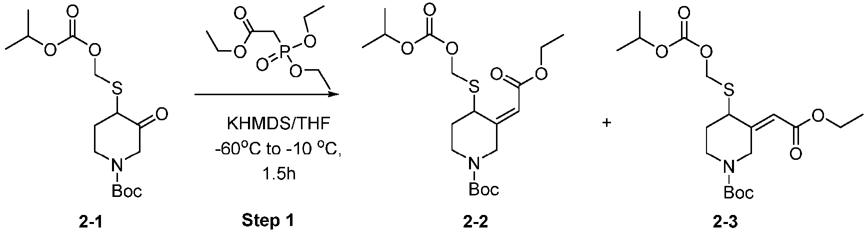

To a solution of ethyl 2-(diethoxyphosphoryl) acetate (20.6 g, 86.5 mmol) in THF (300 mL) was added KHMDS (75 mL, 74.9 mmol) at -60 °C under N₂, which was stirred at -60 °C for 1 h. **2-1** (20.0 g, 57.6 mmol) was then added dropwise at -60 °C, and the resulting mixture was stirred at -10 °C for 0.5 h. Then the reaction mixture was added to *sat.*NH₄Cl (1000 mL). The resulting mixture was extracted with EtOAc (500 mL*2). The combined organic layers were washed with brine, dried over Na₂SO₄ and filtered. The filtrate was concentrated under vacuum and the residue was purified by silica gel chromatography (Petroleum ether/EtOAc=60/1) to give **2-3** (7.5 g, 30% yield) as light yellow oil and **2-2** (4.5 g, 18% yield) as light yellow oil.

**2-2:** ¹H NMR (400 MHz, CDCl₃) δ 5.76 (s, 1H), 5.50 (d, *J =* 15.8 Hz, 1H), 5.23 (d, *J* = 12.1 Hz, 1H), 4.97-4.83 (m, 2H), 4.23-4.06 (m, 3H), 4.01-3.91 (m, 1H), 3.91-3.77 (m, 2H), 3.28-3.12 (m, H,), 2.23- 2.10 (m, 1H), 1.88 (dd, *J =* 23.1, 11.5 Hz, 1H), 1.42 (s, 9H), 1.31-1.24 (m, 9 H).

**2-3:** ¹H NMR (400 MHz, CDCl₃) δ 5.83 (s, 1H), 5.48 (s, 1H), 5.28 (d, *J=* 12.1 Hz, 1H), 5.16 (d, *J =* 12.1 Hz, 1H), 4.94-4.82 (m, 1H), 4.32-4.09 (m, 3H), 4.03-3.85 (m, 2H), 3.23-3.05 (m, 1H), 2.08-1.98 (m, 1H), 1.89 (dd, *J =* 14.2, 1.9 Hz, 1H), 1.44 (s, 10H), 1.31-1.23 (m, 10H).

### Step 2. Synthesis of 2-4

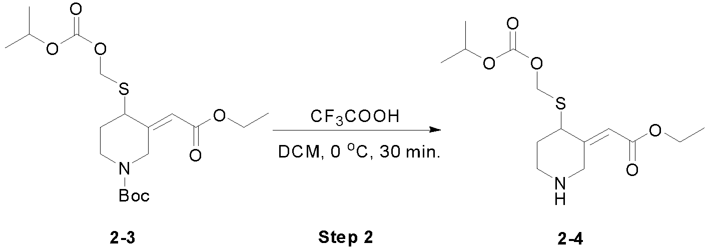

To a solution of **2-3** (3.0 g, 7.2 mmol) in DCM (20 mL) was added TFA (9 mL) at 0 °C, the reaction mixture was stirred at 0 °C for 30 min. After completion, the resulting mixture was added to a solution of *sat.* NaHCO₃ (200 mL). The resulting mixture was then extracted with DCM (200 mL). The organic layer was dried over Na₂SO₄ and filtered. The filtrate was concentrated under reduce pressure to give crude **2-4** (3.0 g, >100 % yield) as yellow oil, which was used in next step without further purification.

### LC-MS [M+1-100]⁺ = 318.1

### Step 3. Synthesis of 2a

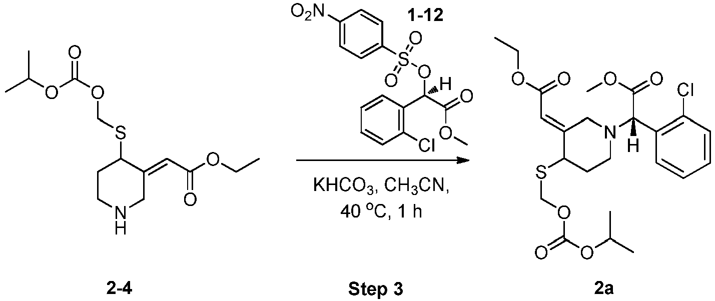

To a solution of **2-4** (3.0 g, crude) in CH₃CN (15 mL) were added **1-12** (2.8 g, 7.2 mmol) and KHCO₃ (1.4 g, 14.4 mmol). The resulting mixture was stirred at 40 °C for 1 h. After completion, the reaction mixture was concentrated under reduce pressure and the residue was purified by reversed-phase column chromatography (C18, CH₃CN/H₂O=80/20) to give **2a** (1.7 g, 47% yield).

### LC-MS [M+1]⁺ = 500.1

¹H NMR (400 MHz, CDCl₃) δ 7.66-7.52 (m, 1H), 7.43-7.32 (m, 1H), 7.25 (s, 2H), 5.80 (s, 1H), 5.22 (d, *J* = 12.2 Hz, 1H), 4.97-4.79 (m, 3H), 4.49 (dd, *J* = 63.7, 12.8 Hz, 1H), 4.18-3.99 (m, 2H), 3.78 (s, 1H), 3.71 (d, *J =* 7.6 Hz, 3H), 3.47 (dd, *J =* 38.1, 13.2 Hz, 1H), 2.72 (dd, *J =* 45.3, 17.4 Hz, 2H), 2.32 (s, 1H), 1.87 (d, *J =* 13.7 Hz, 1H), 1.30 (d, *J =* 6.1 Hz, 6H), 1.24 (t, *J =* 7.2 Hz, 3H).

### Step 4. Synthesis of 2-5

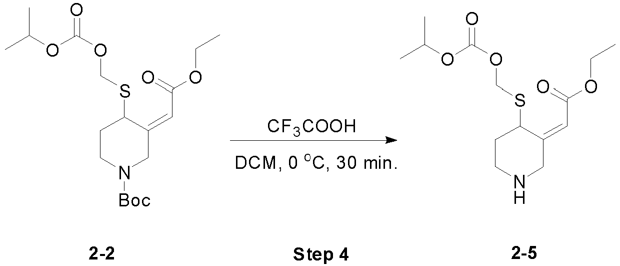

To a solution of **2-2** (100 mg, 0.26 mmol) in DCM (3 mL) was added TFA (0.6 mL) at 0 °C, the reaction mixture was stirred at 0 °C for 30 min. After completion, the reaction mixture was added to a solution of *sat.* NaHCO₃ (20 mL). The resulting mixture was then extracted with DCM (20 mL). The organic layer was dried over Na₂SO₄, and filtered. The filtrate was concentrated under reduce pressure to give crude **2-5** (120.0 mg, >100 % yield) as yellow oil, which was used in the in the next step without further purification.

### LC-MS [M+1-100]⁺ = 318.1

### Step 5. Synthesis of 2b

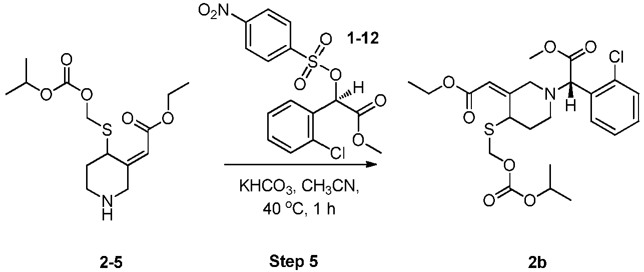

To a solution of **2-5** (120.0 mg, crude) in CH₃CN (3 mL) were added **1-12** (88 mg, 0.23 mmol), and KHCO₃ (92 mg, 0.92 mmol). The resulting mixture was stirred at 40 °C for 1 h. After completion, the reaction mixture was concentrated under reduce pressure and the residue was purified by reversed-phase column chromatography (C18, CH₃CN/H₂O=80/20) to give **2b** (23 mg, 20% yield).

### LC-MS [M+1]⁺ = 500.1.

¹H NMR (400 MHz, CDCl₃) δ 7.59 (d, *J =* 5.1 Hz, 1H), 7.38 (d, *J* = 6.4 Hz, 1H), 7.32-7.25 (m, 2H), 5.74 (s, 0.5H), 5.61 (s, 0.5H), 5.43 (s, 1H), 5.25 (dd, *J =* 11.9, 5.4 Hz, 1H), 5.15 (dd, *J =* 11.9, 4.6 Hz, 1H), 4.93-4.81 (m, 1H), 4.77 (s, 1H), 4.23-4.06 (m, 2H), 3.69 (d, *J =* 3.9 Hz, 3H), 3.51 (d, *J =* 11.9 Hz, 0.5H), 3.42 (d, *J* = 12.1 Hz, 0.5H), 3.14 (d, *J* = 12.3 Hz, 0.5H), 2.97-2.80 (m, 1H), 2.77-2.67 (m, 0.5H), 2.63 (d, *J =* 7.5 Hz, 1H), 2.31-2.10 (m, 1H), 1.88 (dd, *J* = 21.5, 15.1 Hz, 1H), 1.26 (s, 9H).

### Synthetic example 3

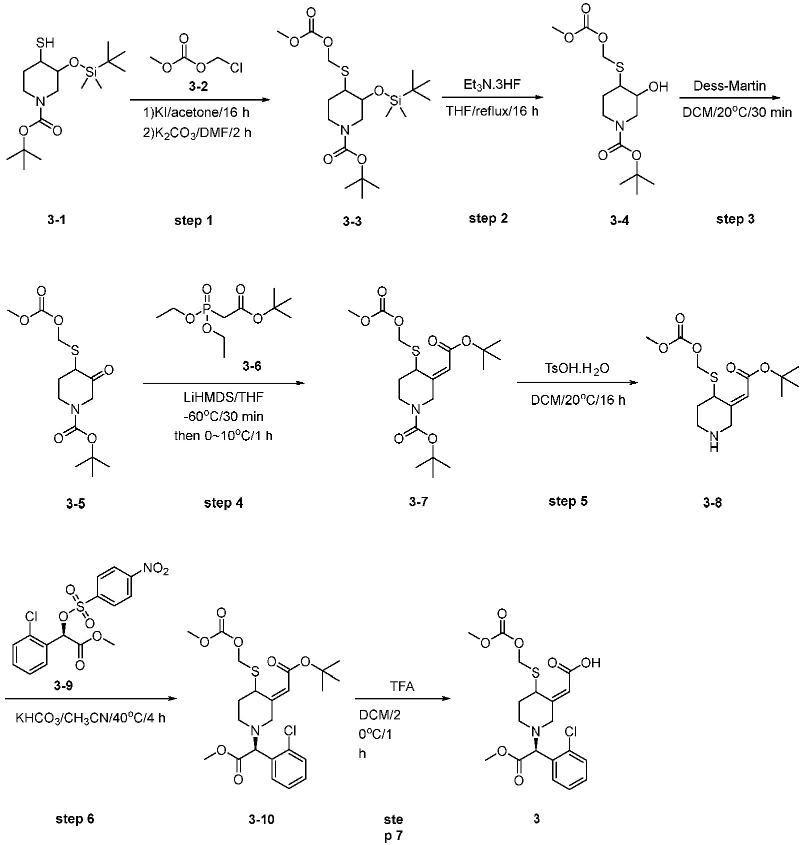

### Step 1. Synthesis of 3-3

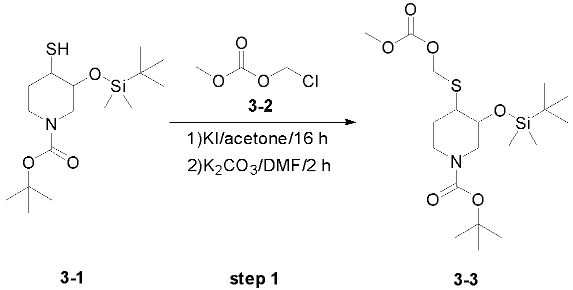

A solution of **3-2** (79.98 g, 0.64 mol) and KI (142.76 g, 0.86 mol) in acetone (1.5 L) was stirred at 16 °C for 16 h. After that, the reaction mixture was concentrated under reduced pressure. The residue was dissolved in DMF (1.5 L) and to the solution above was added **3-1** (150.0 g, 0.43 mol) and K₂CO₃ (88.32 g, 0.64 mol). After addition, the mixture was stirred at 16 °C for 2 h. The reaction was diluted with water (3 L) and extracted by EtOAc (1 L*3). The combined organic layers were dried over anhydrous sodium sulfate and concentrated under reduced pressure. The residue was purified by silica gel chromatography (Petroleum Ether/EtOAc = 20/1) to give **3-3** (79.0 g, 28% yield) as colorless oil.

¹H NMR (400 MHz, Chloroform-*d*) δ 5.36-5.25 (m, 2H), 4.02-3.91 (m, 1H), 3.88-3.83 (m, 1H), 3.80 (s, 3H), 3.52-3.41 (m, 1H), 2.98-2.84 (m, 2H), 2.84-2.74 (m, 1H), 2.14-2.06 (m, 1H), 1.44 (s, 9H), 0.89 (s, 9H), 0.12 (s, 6H).

### Step 2. Synthesis of 3-4

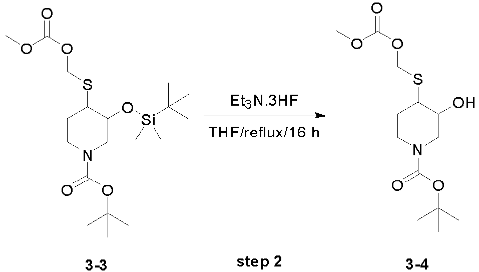

A solution of **3-3** (79.0 g, 0.18 mol) and Et₃N.3HF (90.4 g, 0.54 mol) in THF (800 mL) was refluxed for 16 h under stirred. After cooled down to room temperature, the reaction mixture was concentrated under reduced pressure. The residue was purified by silica gel chromatography (Petroleum Ether/EtOAc = 3/1) to give **3-4** (41.0 g, 68% yield) as colorless oil.

### Step 3. Synthesis of 3-5

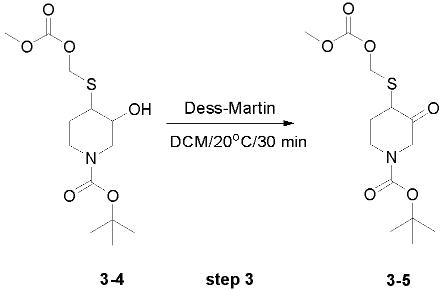

To a solution of **3-4** (41.0 g, 0.12 mol) in DCM (500 mL) was added Dess-Martin (64.9 g, 0.15 mol) at 20 °C. After addition, the mixture was stirred at 20 °C for 30 min. The resulting mixture was washed with saturated Na₃SO₃ aqueous solution (500 mL), saturated NaHCO₃ aqueous solution (500 mL*2) and brine. The organic layer was separated, dried over Na₂SO₄, and filtered. The filtrate was concentrated under vacuum and the residue was purified by silica gel chromatography (Petroleum/EtOAc = 3/1) to give **3-5** (37.0 g, 92% yield) as yellow oil.

### Step 4. Synthesis of 3-7-Z and 3-7-E

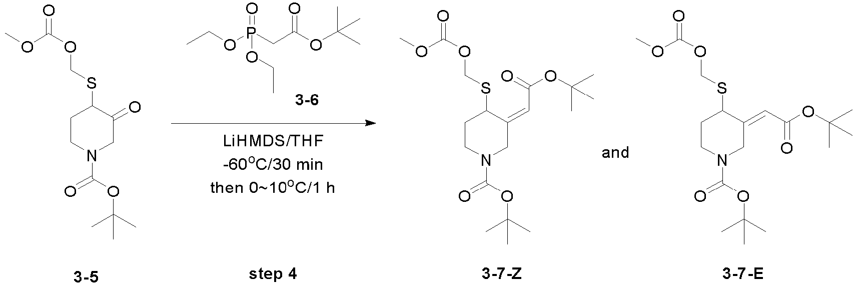

To a solution of **3-6** (37.9 g, 0.15 mol) in dry THF (500 mL) was added LiHMDS (151 mL, 0.15 mol) at -60 °C under N₂. The resulting mixture was stirred at -60 °C for 30 min before **3-5** (37.0 g, 0.11 mol) was added dropwise at -60 °C. The reaction mixture was warmed up to 0 °C and stirred at 0∼10°C for 1 h. Then the resulting mixture was added to *sat.* NH₄Cl (100 mL, *aq.*) and the resulting mixture was extracted with EtOAc (500 mL*2). The combined organic layers were washed with brine, dried over Na₂SO₄ and filtered. The filtrate was concentrated under vacuum and the residue was purified silica gel chromatography (Petroleum ether/EtOAc = 50/1) to give **3-7-Z** (7.0 g, 14% yield) and **3-7-E** (14 g, 28% yield).

### 3-7-Z

¹H NMR (400 MHz, Chloroform-*d*) δ 5.76 (s, 1H), 5.50 (s, 1H), 5.32 (d, *J =* 12 Hz, 1H), 5.17 (d, *J =* 12 Hz, 1H), 4.32-3.87 (m, 3H), 3.79 (s, 3H), 3.24-3.03 (m, 1H), 2.13-2.00 (m, 1H), 1.93-1.85 (m, 1H), 1.47 (s, 9H), 1.45 (s, 9H).

### 3-7-E

¹H NMR (400 MHz, Chloroform-*d*) δ 5.68 (s, 1H), 5.52-5.43 (s, 1H), 5.26 (d, *J =* 12.4 Hz, 1H), 4.96 (d, *J =* 12.4 Hz, 1H), 3.97-3.85 (m, 2H), 3.80 (s, 3H), 3.79-3.75 (m, 1H), 3.27-3.13 (m, 1H), 2.21-2.09 (m, 1H), 1.93-1.83 (m, 1H), 1.48 (s, 9H), 1.43 (s, 9H).

### Step 5. Synthesis of 3-8

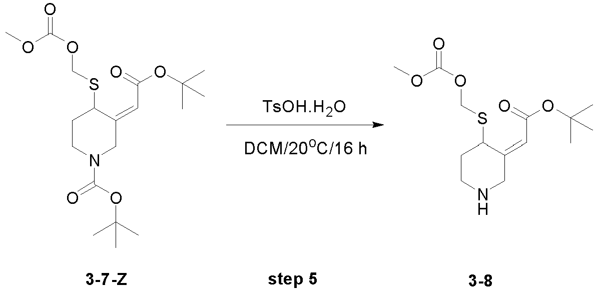

A solution of **3-7-Z** (5.5 g, 13.2 mmol) and TsOH.H₂O (5.0 g, 26.4 mmol) in DCM (60 mL) was stirred at 20 °C for 16 h. After that, the reaction mixture was diluted with saturated NaHCO₃ aqueous solution (100 mL) and extracted by DCM (50 mL*2). The combined organic layers were washed with brine, dried over Na₂SO₄ and filtered. The filtrate was concentrated under vacuum to give **3-8** (3 g, 71% yield) as yellow oil, which was used to next step without further purification. LC-MS [M+1]⁺ = 318.1

### Step 6: Synthesis of 3-10

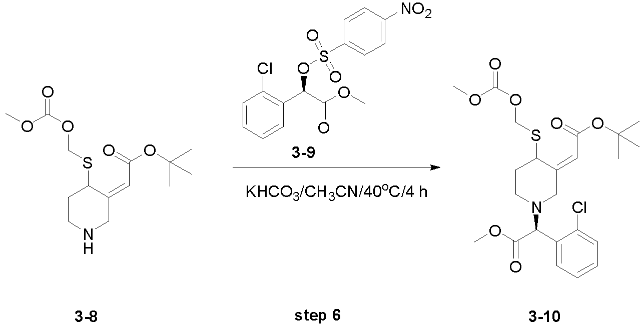

To a solution of **3-8** (3.0 g, 9.4 mmol) in CH₃CN (10 mL) were added **3-9** (3.6 g, 9.4 mmol) and KHCO₃ (2.8 g, 28.2 mmol). The resulting mixture was stirred at 40 °C for 4 h. After completion, the reaction mixture was concentrated under reduce pressure and the residue was purified by reversed-phase column chromatography (C18, CH₃CN/H₂O = 90/10) to give **3-10** (2.5 g, 53% yield) as yellow oil. LC-MS [M+1]⁺ = 500.2

### Steps 7 Synthesis of 3

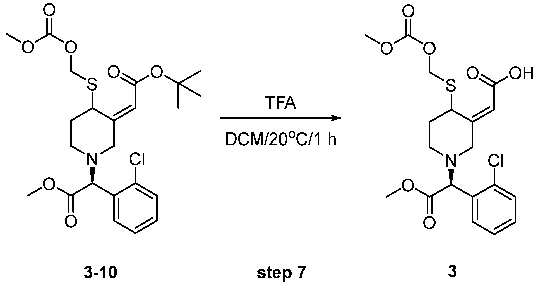

To a solution **of 3-10** (2.5 g, 5.0 mmol) in DCM (20 mL) was added TFA (5 mL), which was stirred at 20 °C for 1 h. After completion, the reaction was added to a solution of saturated aqueous NaHCO₃ solution (50 mL) and extracted with DCM (50 mL*3). The combined organic layers were washed with brine, dried over Na₂SO₄ and filtered. The filtrate was concentrated under reduce pressure and the residue was purified by Prep-TLC (DCM/MeOH = 10/1) to give **3** (800 mg yield 36%).

### 3:

### LC-MS [M+1]⁺ = 444.1.

¹H NMR (400 MHz, Chloroform-*d*) δ 7.60-7.54 (m, 1H), 7.41-7.36 (m, 1H), 7.31-7.22 (m, 2H), 5.77 (s, 0.5H), 5.65 (s, 0.5H), 5.43-5.37 (m, 1H), 5.30-5.23 (m, 1H), 5.19-5.12 (m, 1H), 4.81-4.77 (m, 1H), 3.76 (s, 3H), 3.69 (d, *J =* 4.4 Hz, 3H), 3.54 (d, *J =* 12.4 Hz, 0.5H), 3.45 (d, *J* = 12.4 Hz, 0.5H), 3.17 (d, *J =* 12.4 Hz, 0.5H), 2.96 (d, *J =* 12.4 Hz, 0.5H), 2.85 (d, *J =* 12.0 Hz, 0.5H), 2.73 (d, *J =* 12.0 Hz, 0.5H), 2.70-2.62 (m, 1H), 2.30-2.13 (m, 1H), 2.02-1.82 (m, 1H).

### Synthetic example 4

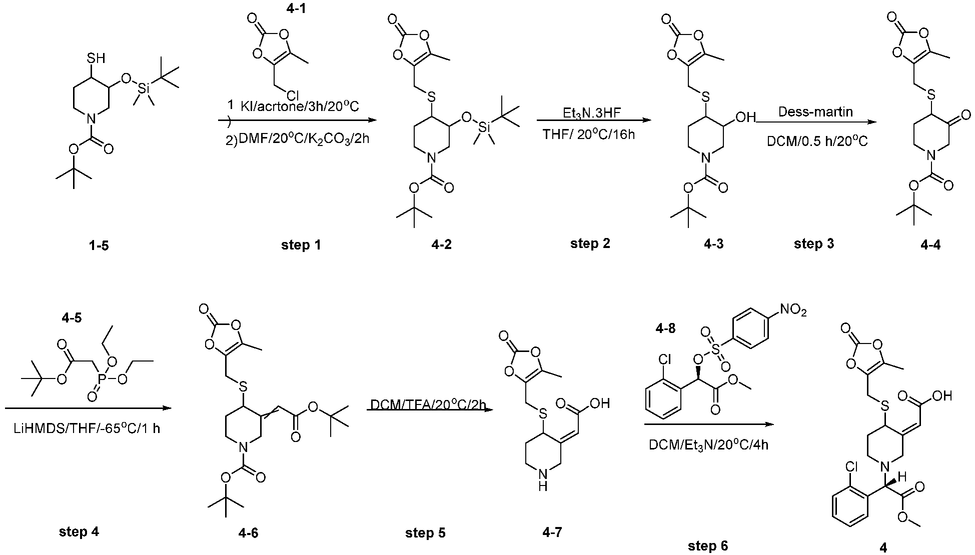

### Step 1. Synthesis of 4-2

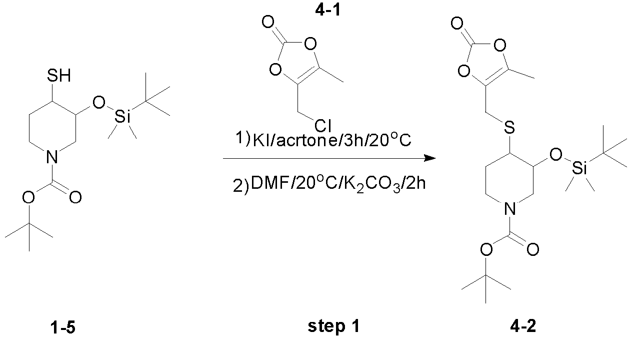

A solution of **4-1** (64 g, 0.43 mol) and KI (96.3 g, 0.86 mol) in acetone (0.8 L) was stirred at 20 °C for 3 h. The reaction mixture was concentrated under reduced pressure. The residue was dissolved in DMF (1 L). To the solution above was added **1-5** (150.0 g, 0.43mol) and K₂CO₃ (120 g, 0.864 mol). After addition, the resulting mixture was stirred at 20 °C for 2 h. The reaction was diluted with water (2 L) and extracted by EtOAc (600 L*2). The combined organic layers were dried over anhydrous sodium sulfate and concentrated under reduced pressure to give the **4-2** (250 g, 100% yield) as dark oil, which was used to next step without further purification.

### Step 2. Synthesis of 4-3

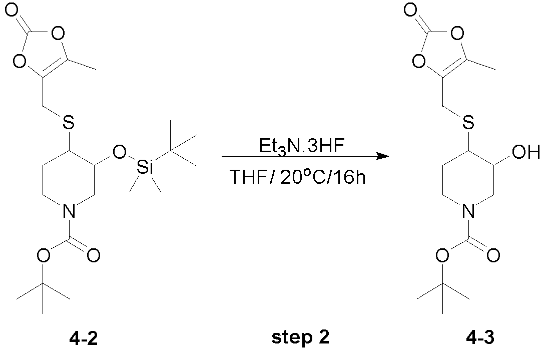

A solution of **4-2** (250 g, 0.43 mol) and Et₃N.3HF (210 g, 1.296 mol) in THF (1 L) was stirred at 40 °C for 16 h. The resulting mixture was concentrated under reduced pressure and the residue was diluted with EA (1.5L). The solution formed was washed with brine (500ml*2) and the organic layer was separated, dried over Na₂SO₄ and concentrated under reduced pressure. The residue was purified by silica gel chromatography (Petroleum Ether/EtOAc = 3/1) to give **4-3** (108 g, 73% yield) as white solid. LC-MS [M+1]⁺ -100= 246.2.

¹H NMR (400 MHz, CDCl3) δ 4.19 (d, *J =* 12.1 Hz, 1H), 3.98 (s, 1H), 3.72 (s, 1H), 3.52 (d, *J =* 15.2 Hz, 2H), 2.90 - 2.78 (m, 1H), 2.77 - 2.67 (m, 1H), 2.66 - 2.56 (m, 1H), 2.11 (s, 3H), 2.02 (d, *J =* 12.0 Hz, 1H), 1.63 - 1.49 (m, 1H), 1.45 (s, 9H).

### Step 3. Synthesis of 4-4

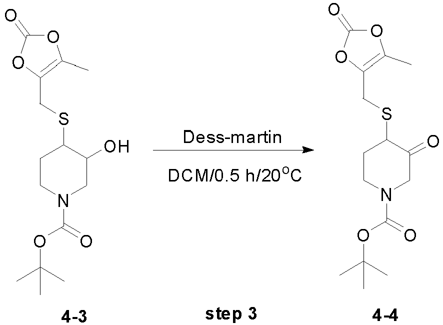

To a solution of **4-3** (108 g, 0.313 mol) in DCM (1 L) was added Dess-Martin (159 g, 0.375 mol) at 20 °C. After addition, the resulting mixture was stirred at 20 °C for 30 min. The reaction mixture was washed with saturated Na₂S₂O₃ solution (1 L), saturated NaHCO₃ solution (1L*2), brine, dried over Na₂SO₄ and concentrated under vacuum. The residue was purified by silica gel chromatography (Petroleum/EtOAc = 3/1) to give **4-4** (80.0 g, 74.5% yield) as an orange oil. LC-MS [M+1]⁺ +Na=366.1.

¹H NMR (400 MHz, CDCl₃) δ 4.29 (d, *J =* 17.9 Hz, 1H), 4.16 (d, *J* = 18.4 Hz, 1H), 3.82 (s, 1H), 3.48 (d, *J =* 15.6 Hz, 1H), 3.44 - 3.32 (m, 2H), 3.27 (s, 1H), 2.43 - 2.29 (m, 1H), 2.16 (s, 3H), 2.09 - 2.04 (m, 1H), 1.46 (s, 9H).

### Step 4. Synthesis of 4-6-Z and 4-6-E

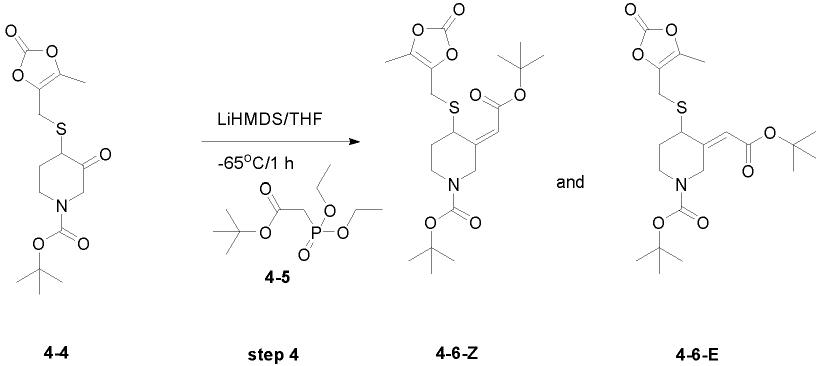

To a solution of **4-5** (77 g, 0.302 mol) in dry THF (800 mL) was added LiHMDS (303 mL, 0.303 mol) at -60°C under N₂. The reaction mixture was stirred at -60°C for 30 min before **4-4** (80 g, 0.233 mol) was added dropwise at -60°C. The resulting mixture was stirred at 0~10°C for 1 h. Then the reaction mixture was added to saturated NH₄Cl solution (800 mL) and extracted with EtOAc (700 mL*2). The combined organic layers were washed with brine, dried over Na₂SO₄ and filtered. The filtrate was concentrated under vacuum and the residue was purified by silica gel chromatography (Petroleum ether/EtOAc = 20/1) to give **4-6-Z** (12 g, 9% yield) as a light orange oil and **4-6-E** (15 g, 11% yield) as an off-white solid. LC-MS [M+1]⁺ +23= 464.2.

### 4-6-Z

¹H NMR (400 MHz, CDCl₃) δ 5.74 (s, 1H), 4.27 (s, 1H), 4.07 - 3.83 (s, 2H), 3.68 (d, *J* = 15.1 Hz, 1H), 3.44 (d, *J =* 15.2 Hz, 1H), 3.25 - 3.10 (m,1H), 2.09 (s, 3H), 2.02 (d, *J =* 14.6 Hz, 1H), 1.85 (d, *J =* 13.7 Hz, 1H), 1.54 - 1.32 (m, 18H).

### 4-6-E

¹H NMR (400 MHz, CDCl3) 6 5.59 (s, 1H), 5.49 (d, *J* = 15.6 Hz, 1H), 4.00 (d, *J* = 15.7 Hz, 1H), 3.95- 3.80 (m, 1H), 3.61 (s, 1H), 3.40 - 3.14 (m, 3H), 2.21- 2.11 (m, 1H), 2.08 (s, 3H), 1.89 (d, *J* = 11.4 Hz, 1H), 1.52- 1.42 (m,18H).

### Step 5: Synthesis of 4-7

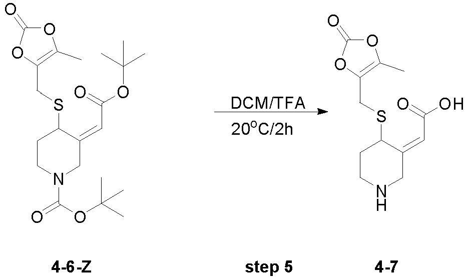

A solution of **4-6-Z** (10 g, 0.023 mol) and TFA (20 ml) in DCM (80 mL) was stirred at 20 °C for 2 h. The resulting mixture was concentrated under vacuum to give **4-7** (15 g, 100% yield) as dark oil, which was used to next step without further purification. LC-MS [M+1]⁺ = 286.2.

### Steps 6 and 7. Synthesis of 4

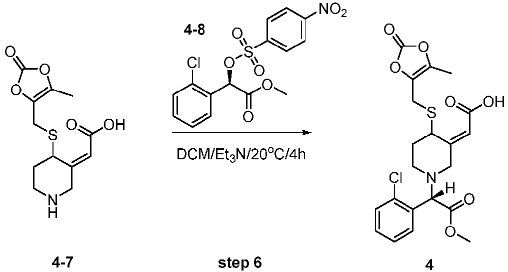

A solution of **4-7** (15 g, crude, 0.023mol) and **4-8** (5 mL) in DCM was added Et₃N dropwise at 20 °C. After addition, the mixture was stirred at 20 °C for 4h. The resulting mixture was concentrated under reduce pressure. The residue was diluted with EA (200mL) and water (300ml). The pH value was adjusted to 3 with HCl (1M, aq.). The organic layer was separated, washed with brine, dried over Na₂SO₄ and concentrated under reduced pressure. The residue was purified by reverse phase column (C18, ACN/H₂O = 70/30) to give **4** (1.8 g, 16.7% yield).

### 4:

### LC-MS [M+1]+ = 468.1.

¹H NMR (400 MHz, CDCl₃) δ 7.57 (d, *J* = 6.7 Hz, 1H), 7.40 (d, *J* = 6.2 Hz, 1H), 7.32-7.23 (m, 2H), 5.79 (s, 0.5H), 5.67 (s, 0.5H), 5.23 (s, 1H), 4.77 - 4.67 (m, 1H), 3.71 (d, *J =* 4.5 Hz, 3H), 3.60- 3.52 (m, *1.5*H), 3.50 - 3.36 (m, 1.5H), 3.17 (d, *J =* 12.3 Hz, 0.5H), 2.97 (d, *J* = 12.4 Hz, 0.5H), 2.90- 2.82 (m, 0.5H), 2.80- 2.70 (m, 0.5H), 2.66 (d, *J =* 8.6 Hz, 1H), 2.30-2.13 (m, 1H), 2.06 (s, 3H), 1.93- 1.81 (m, 1H).

### II. Pharmaceutical Compositions

### Example 1

The pharmaceutical composition was prepared according to the composition shown in Table 1 below. SBECD was added to 36 mL of water for injection, and the mixture was stirred until the SBECD was completely dissolved. Citric acid monohydrate was then added, and the resulting mixture was stirred until the citric acid monohydrate was completely dissolved. Hydrochloric acid was then added to adjust the pH to 1.2. Compound 1b-2 was added to the solution, and the mixture was stirred until the compound was completely dissolved. Sodium hydroxide was added to the resulting solution to adjust the pH to 4, and water for injection was then added to bring the volume to 60 mL.

**Table 1**

| **Component** | **Effect** | **Content** |
|---|---|---|
| Compound 1b-2 | Active ingredient | 0.048 g |
| SBECD | Solubilizer | 2.88 g |
| Citric acid monohydrate | Buffer | 0.126 g |
| Hydrochloric acid (1 N) | pH adjuster | - |
| Sodium hydroxide | pH adjuster | - |
| Water for injection | Solvent | Adding to bring the volume to 60 mL |

### Example 2

The pharmaceutical composition was prepared according to the composition shown in Table 2 below. SBECD was added to 36 mL of water for injection, and the mixture was stirred until the SBECD was completely dissolved. Citric acid monohydrate was then added, and the resulting mixture was stirred until the citric acid monohydrate was completely dissolved. Hydrochloric acid was then added to adjust the pH to 1.2. Compound 1b-2 was added to the solution, and the mixture was stirred until the compound was completely dissolved. Sodium hydroxide was added to the resulting solution to adjust the pH to 4, and water for injection was then added to bring the volume to 60 mL.

**Table 2**

| **Component** | **Effect** | **Content** |
|---|---|---|
| Compound 1b-2 | Active ingredient | 0.048 g |
| SBECD | Solubilizer | 4.8 g |
| Citric acid monohydrate | Buffer | 0.126 g |
| Hydrochloric acid (1 N) | pH adjuster | - |
| Sodium hydroxide | pH adjuster | - |
| Water for injection | Solvent | Adding to bring the volume to 60 mL |

### Example 3

The pharmaceutical composition was prepared according to the composition shown in Table 3 below. SBECD was added to 36 mL of water for injection, and the mixture was stirred until the SBECD was completely dissolved. Citric acid monohydrate was then added, and the resulting mixture was stirred until the citric acid monohydrate was completely dissolved. Hydrochloric acid was then added to adjust the pH to 1.2. Compound 1b-2 was added to the solution, and the mixture was stirred until the compound was completely dissolved. Sodium hydroxide was added to the resulting solution to adjust the pH to 4, and water for injection was then added to bring the volume to 60 mL.

**Table 3**

| **Component** | **Effect** | **Content** |
|---|---|---|
| Compound 1b-2 | Active ingredient | 0.048 g |
| SBECD | Solubilizer | 9.6 g |
| Citric acid monohydrate | Buffer | 0.126 g |
| Hydrochloric acid (1 N) | pH adjuster | - |
| Sodium hydroxide | pH adjuster | - |
| Water for injection | Solvent | Adding to bring the volume to 60 mL |

### Example 4

The pharmaceutical composition was prepared according to the composition shown in Table 4 below. HPCD was added to 30 mL of water for injection, and the mixture was stirred until the HPCD was completely dissolved. Hydrochloric acid was then added to adjust the pH to 1.2. Compound 1b-2 was added to the solution, and the mixture was stirred until the compound was completely dissolved. Sodium hydroxide was added to the resulting solution to adjust the pH to 4, and water for injection was then added to bring the volume to 50 mL.

**Table 4**

| **Component** | **Effect** | **Content** |
|---|---|---|
| Compound 1b-2 | Active ingredient | 0.05 g |
| HPCD | Solubilizer | 10.6 g |
| Hydrochloric acid (1 N) | pH adjuster | - |
| Sodium hydroxide | pH adjuster | - |
| Water for injection | Solvent | Adding to bring the volume to 50 mL |

### Example 5

The pharmaceutical composition was prepared according to the composition shown in Table 5 below. SBECD was added to 36 mL of water for injection, and the mixture was stirred until the SBECD was completely dissolved. Citric acid monohydrate was then added, and the resulting mixture was stirred until the citric acid monohydrate was completely dissolved. Hydrochloric acid was then added to adjust the pH to 1.2. Compound 1b-2 was added to the solution, and the mixture was stirred until the compound was completely dissolved. Sodium hydroxide was added to the resulting solution to adjust the pH to 3.5, and water for injection was then added to bring the volume to 60 mL.

**Table 5**

| **Component** | **Effect** | **Content** |
|---|---|---|
| Compound 1b-2 | Active ingredient | 0.048 g |
| SBECD | Solubilizer | 4.8 g |
| Citric acid monohydrate | Buffer | 0.126 g |
| Hydrochloric acid (1 N) | pH adjuster | - |
| Sodium hydroxide | pH adjuster | - |
| Water for injection | Solvent | Adding to bring the volume to 60 mL |

### Example 6

The pharmaceutical composition was prepared according to the composition shown in Table 6 below. SBECD was added to 20 mL of water for injection, and the mixture was stirred until the SBECD was completely dissolved. Hydrochloric acid was then added to adjust the pH to 1.2. Compound 1b-2 was added to the solution, and the mixture was stirred until the compound was completely dissolved. Sodium hydroxide was added to the resulting solution to adjust the pH to 4, and water for injection was then added to bring the volume to 28 mL.

**Table 6**

| **Component** | **Effect** | **Content** |
|---|---|---|
| Compound 1b-2 | Active ingredient | 0.048 g |
| SBECD | Solubilizer | 5.6 g |
| Hydrochloric acid (1 N) | pH adjuster | - |
| Sodium hydroxide | pH adjuster | - |
| Water for injection | Solvent | Adding to bring the volume to 28 mL |

### Example 7

The pharmaceutical composition was prepared according to the composition shown in Table 7 below. SBECD was added to 36 mL of water for injection, and the mixture was stirred until the SBECD was completely dissolved. Citric acid monohydrate was then added, and the resulting mixture was stirred until the citric acid monohydrate was completely dissolved. Hydrochloric acid was then added to adjust the pH to 1.2. Compound 1b-2 was added to the solution, and the mixture was stirred until the compound was completely dissolved. Sodium hydroxide was added to the resulting solution to adjust the pH to 4, and water for injection was then added to bring the volume to 56 mL.

**Table 7**

| **Component** | **Effect** | **Content** |
|---|---|---|
| Compound 1b-2 | Active ingredient | 0.048 g |
| SBECD | Solubilizer | 5.6 g |
| Citric acid monohydrate | Buffer | 0.058 g |
| Hydrochloric acid (1 N) | pH adjuster | - |
| Sodium hydroxide | pH adjuster | - |
| Water for injection | Solvent | Adding to bring the volume to 56 mL |

### Example 8

The pharmaceutical composition was prepared according to the composition shown in Table 8 below. SBECD was added to 36 mL of water for injection, and the mixture was stirred until the SBECD was completely dissolved. Citric acid monohydrate was then added, and the resulting mixture was stirred until the citric acid monohydrate was completely dissolved. Hydrochloric acid was then added to adjust the pH to 1.2. Compound 1b-2 was added to the solution, and the mixture was stirred until the compound was completely dissolved. Sodium hydroxide was added to the resulting solution to adjust the pH to 4, and water for injection was then added to bring the volume to 112 mL.

**Table 8**

| **Component** | **Effect** | **Content** |
|---|---|---|
| Compound 1b-2 | Active ingredient | 0.048 g |
| SBECD | Solubilizer | 5.6 g |
| Citric acid monohydrate | Buffer | 0.176 g |
| Hydrochloric acid (1 N) | pH adjuster | - |
| Sodium hydroxide | pH adjuster | - |
| Water for injection | Solvent | Adding to bring the volume to 112 mL |

### Examples 9-11

Pharmaceutical compositions were prepared according to the compositions shown in Table 9 below in the same manner as in Example 1.

**Table 9**

| **Component** | **Effect** | **Content** | | |
|---|---|---|---|---|
| | | **Example 9** | **Example 10** | **Example 11** |
| Compound 1b-2 | Active ingredient | 0.048 g | 0.048 g | 0.048 g |
| SBECD | Solubilizer | 2.88 g | 5.76 g | 9.6 g |
| Citric acid monohydrate | Buffer | 0.0756 g | 0.0756 g | 0.0756 g |
| Hydrochloric acid (1 N) | pH adjuster | - | - | - |
| Sodium hydroxide | pH adjuster | - | - | - |
| Water for injection | Solvent | Adding to bring the volume to 60 mL | Adding to bring the volume to 60 mL | Adding to bring the volume to 60 mL |

### III. Biochemical Analysis

### Biochemical Assays

### Assay 1: Pharmacokinetic in Rats

### Male Sprague-Dawley rats were used to conduct pharmacokinetic experiment.

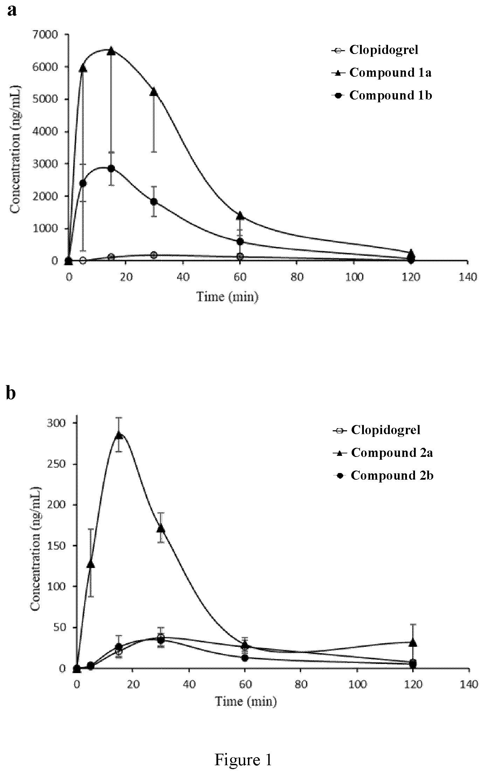

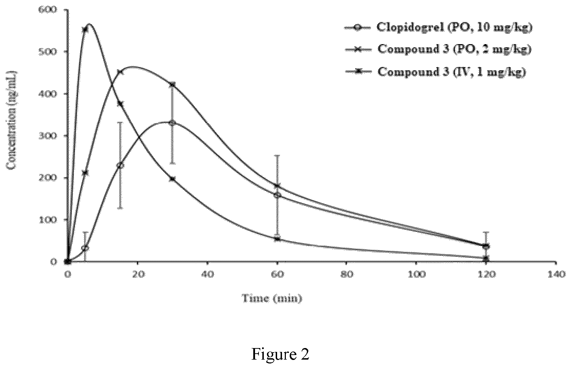

Test compounds (clopidogrel and exemplary compounds provided herein) was administrated orally or intravenously to rat under fast condition. Blood samples were collected via jugular vein at 5 min, 15 min, 30 min, 60 min and 120 min time points with EDTA-K₂ (anticoagulant), 3'-methoxyphenacyl bromide (MPBr, derivatization reagent) and phenylmethylsulfonyl fluoride (PMSF, stabilizer). Plasma samples were then harvested by centrifuging at 1500 g for 10 min under 2~8°C and stored at -80°C after separation. Plasma samples were loaded to a LC-MS/MS instrument after extraction to determine the concentration of the thiol active metabolite. The concentration results in rat plasma were demonstrated in Figures 1 and 2.

As shown in Figure 1, at a dose level of 10 mg/kg, compounds 1a, 1b and 2a provided herein reached peak concentration of the thiol active metabolite in less than 20 minutes after administration, compared to clopidogrel that reached peak concentration at about 30 minutes after administration. Furthermore, the peak concentrations of the thiol active metabolite for compounds 1a, 1b and 2a are significantly higher than that for clopidogrel. These results indicate that compounds 1a, 1b and 2a provide faster and more efficient release of the active metabolite than clopidogrel.

As shown in Figure 2, when administered orally, compound 3 provided herein at a dose level of 2 mg/kg reached peak concentration of the thiol active metabolite at about 20 minutes after administration, compared to clopidogrel at a much higher dose level of 10 mg/kg that reached peak concentration at about 30 minutes after administration. When administered intravenously, compound 3 provided herein at a dose level of only 1 mg/kg reached peak concentration of the thiol active metabolite at about 6 minutes after administration. These results indicate that compound 3 provides faster and more efficient release of the active metabolite than clopidogrel.

### Assay 2: Antiaggregatory Action in Rats

Male Sprague-Dawley rats were used for ex vivo platelet aggregation experiments. After oral administrated with (clopidogrel, exemplary compounds provided herein and vehicle (control)) to rats, blood was collected via jugular vein at 0.5 hour, 1 hour and 2 hour time points, using 3.8% (w/v) sodium citrate solution as an anticoagulant (1/9 volume of whole blood). Blood samples with citrate were centrifuged at a low speed of 1000 rmp for 5 min to obtain platelet-rich plasma (PRP). After separation of PRP, the remained blood was further centrifuged at a high speed of 3000 rmp for 10min to obtain platelet-poor plasma (PPP). The number of platelets in PRP was measured by hematology analyzer (Siemens, ADVIA2120), and adjusted to 4×10⁸/mL with PPP.

Platelet aggregation was determined using turbidimetric aggregometry method by an automatic platelet aggregometer (PRECIL LBY-NJ4). The aggregometer was primarily warmed up to 37°C, and PRP (290 µL) sample was added to the cuvette and set in the automatic platelet aggregometer. After a 5min pre-incubation, calibrated the aggregometer using PPP to representing 100% aggregation and PRP to representing 0% aggregation. Finally, a volume of 10 µL ADP solution (final concentration 10 µM) was added to PRP sample to initial platelet aggregation. Platelet aggregation was monitored for 5 min and maximum platelet aggregation (%) was reported within the duration. Antiaggregatory action of test compounds was expressed as inhibition (%) which determined by the relation: Inhibition (%) = (maximum platelet aggregation (%) of control - maximum platelet aggregation (%) of test compound)/(maximum platelet aggregation (%) of control)* 100

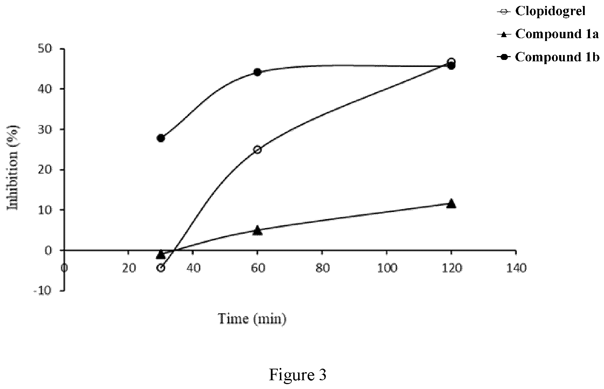

The inhibition (%) results for the test compounds were demonstrated in Figure 3. The dose levels were 10 mg/kg, 0.5 mg/kg and 2 mg/kg for clopidogrel, 1a and 1b respectively. As can be seen from Figure 2, clopidogrel reaches maximum inhibition of platelet aggregation at about 45% in about 120 minutes after administration, whereas compound 1b shows the maximum inhibition at about 45% in 60 minutes after administration at a much lower dose level than clopidogrel, indicating a much faster onset of action and much higher potency than clopidogrel.

### IV. Formulation Stability

The formulation samples obtained in the examples were each treated as follows: the sample was filtered through a 0.22 µm filter membrane and placed in a clear glass vial. The water bath was adjusted to 25 °C, and the sample vials containing the formulations were placed in the water bath. The stability of the formulation samples was measured at 0 h, 1 h, 2 h, and 4 h.

The sample stability was determined by the following method: the sample content was analyzed using ultra-high performance liquid chromatography/ultraviolet detector. The chromatographic conditions were as follows:
Chromatographic column: ACQUITY UPLC BEH C18 1.7 µm 2.1 × 100 mm;
Mobile phase A: 0.1% aqueous formic acid;
Mobile phase B: 0.1% formic acid in acetonitrile.

The relevant results are shown in the table below.

**Table 10. Content of compound 1b-2 in samples at different time points**

| Example | Content of compound 1b-2 (%) | | | |
|---|---|---|---|---|
| | 0 h | 1 h | 2 h | 4 h |
| 6 | 100 | 99.5 | 99.1 | 97.9 |
| 7 | 100 | 99.5 | 99.6 | 99.0 |
| 8 | 100 | 99.8 | 98.2 | 99.5 |
| 9 | 100 | 101.2 | 101.2 | 101.1 |
| 10 | 100 | 100.3 | 100.4 | 100.1 |
| 11 | 100 | 100.1 | 99.1 | 99.6 |

As can be seen from Table 10, the content of compound 1b-2 in the samples of different examples was no less than 97% in 4 hours, indicating that the formulations of the present disclosure have good stability in the case of different ingredient ratios and different formulation concentrations.

### V. Drug Effect Experiment

The formulations of Examples 1-3 above were each diluted to 0.12 mg/mL (based on compound 1b-2) using 0.9% sodium chloride injection to obtain formulations 1-3. In addition, citric acid monohydrate was dissolved in water for injection, sodium hydroxide was added to adjust the pH to 4, and then water for injection was added to bring the total volume to a desired volume, thus obtaining a first solution in which the content of citric acid was 2 mg/mL; the first solution was mixed with 0.9% sodium chloride injection according to a ratio of 3:17 to obtain a second solution; citric acid monohydrate was dissolved in water for injection, hydrochloric acid was added to adjust the pH to 1, and then water for injection was added to bring the total volume to a desired volume, thus obtaining a third solution in which the content of citric acid was 2 mg/mL; compound 1b was added to an appropriate amount of the third solution to be completely dissolved, then an appropriate amount of the second solution was added, a sodium hydroxide solution was added to adjust the pH to 4.0±0.04, and then the second solution was added until the content of the compound 1b-2 was 0.12 mg/mL (based on the compound 1b-2), thus obtaining a formulation 4.

The test animals, male beagle dogs, were divided into 4 groups, the above formulations 1-4 were administered to the groups by intravenous infusion at a dose of 5 mL/Kg/h for 0.5 h, respectively, and the following experiments were performed.

PK experiment: each group of animals were subjected to collection of 0.5 mL of whole blood via the neck vein before administration and 0.17 h, 0.33 h, 0.5 h, 1 h, 2 h, 4 h, 8 h, and 24 h after administration. The whole blood collected was immediately transferred to blood collection tubes to which 3'-methoxyphenacyl bromide (MPBr, derivatization reagent), phenylmethylsulfonyl fluoride solution (PMSF, stabilizer), and EDTA-K2 had been added. The mixtures were each mixed uniformly and centrifuged, and plasma was collected and stored at no more than -70 °C. Plasma samples were treated and then assayed using an LC-MS/MS instrument (Shimadzu 20A/API4000 Qtrap LC-MS instrument) to determine the peak concentration of the thiol active metabolite (Cₘₐₓ) and the area under the curve (AUC₀₋ₜ). The results are shown in Table 11 below.

**Table 11. PK experimental results for different formulations**

| Formulation | Cₘₐₓ (ng/mL) | AUC₀₋ₜ (ng/mL*h) |
|---|---|---|
| Formulation 1 | 536.33 | 328.42 |
| Formulation 2 | 514.67 | 297.48 |
| Formulation 3 | 512.67 | 304.90 |
| Formulation 4 | 390.67 | 247.25 |

As can be seen from Table 11, compared with formulation 4 without cyclodextrin, the exposure of the formulations corresponding to the formulations of the present disclosure in the experimental animals was significantly improved.

PD experiment: each group of animals were subjected to collection of 1.8 mL of whole blood via the neck vein before administration and 0.17 h, 0.5 h, 2 h, 4 h, 8 h, 24 h, 48 h, 72 h, and 96 h after administration, and the whole blood was mixed with 3.2% sodium citrate, the anticoagulant, according to the volume ratio of 1:9 for anticoagulation, thus obtaining sodium citrate anticoagulation blood samples.

A part of each of the above sodium citrate anticoagulation blood samples was taken for platelet counting by using a Mindray fully automatic hematology analyzer; the remaining sodium citrate anticoagulation blood samples were each centrifuged at RT and 100 g for 10 min (brake switched off) to prepare PRP, and the PLT concentration was determined. PPP was then isolated by centrifugation at RT and 2000 g for 10 min (brake switched on), and the PLT concentration was determined. At the same time, an aggregation inducer (ADP, 150 µM) was prepared.

The reaction system for determining the platelet aggregation rate was 145 µL of PRP + 5 µL of inducer. During determining, firstly, the instrument was calibrated with PPP plasma at 100% and PRP at 0%; when PRP was determined, 145 µL of PRP was added to the bottom of a reaction cup and mixed uniformly, the cup was preheated at 37 °C for 1 min, and then 5 µL of an aggregation inducer was added, and the maximum aggregation rate under ADP stimulation for 10 min was determined and used for evaluating the anti-platelet aggregation capability.

Meanwhile, the inhibition rate was calculated, and the calculation formula is as follows: Inhibition rate = (maximum aggregation rate at time point of administration - maximum aggregation rate before administration)/maximum aggregation rate before administration × 100%.

The results are shown in Table 12 below.

**Table 12. Inhibition rates at different time points of administration for each formulation**

| Formulation | Administration time | | |
|---|---|---|---|
| | 0 min | 10 min | 30 min |
| Formulation 1 | 0.00% | 43.48% | 69.85% |
| Formulation 3 | 0.00% | 61.27% | 78.47% |
| Formulation 4 | 0.00% | 19.72% | 74.59% |

As can be seen from Table 12, compared with formulation 4 without cyclodextrin, the formulations corresponding to the formulations of the present disclosure exhibited significantly increased inhibition rate in experimental animals at 10 min and faster speed of onset of therapeutic effect and better drug effect.

The foregoing description is considered as illustrative only of the principles of the present disclosure. Further, since numerous modifications and changes will be readily apparent to those skilled in the art, it is not desired to limit the invention to the exact construction and process shown as described above. Accordingly, all suitable modifications and equivalents may be considered to fall within the scope of the invention as defined by the claims that follow.

## Claims

1. A pharmaceutical composition, comprising:
a compound of formula (I): 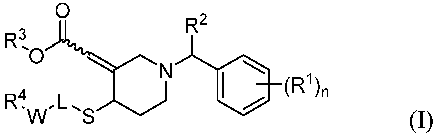
or a pharmaceutically acceptable salt thereof, and
a cyclodextrin,
wherein
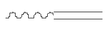 represents a double bond in Z or E configuration;
R¹ is selected from the group consisting of hydrogen, halogen, nitro, cyano, hydroxyl, amino, alkyl, alkenyl, alkynyl, heteroalkyl, heteroalkenyl, and heteroalknyl, wherein each of alkyl, alkenyl, alkynyl, heteroalkyl, heteroalkenyl, and heteroalknyl is optionally substituted with one or more R^{a};
R² is -C(O)R^{b};
R³ is selected from the group consisting of hydrogen, alkyl, alkenyl, alkynyl, heteroalkyl, heteroalkenyl, heteroalknyl, saturated or partially unsaturated cycloalkyl, saturated or partially unsaturated heterocyclyl, aryl, and heteroaryl, wherein each of alkyl, alkenyl, alkynyl, heteroalkyl, heteroalkenyl, heteroalkynyl, cycloalkyl, heterocyclyl, aryl and heteroaryl is optionally substituted with cyano, halogen, hydroxyl, amino, alkyl, alkenyl, alkynyl, heteroalkyl, heteroalkenyl, or heteroalkynyl;
L is selected from the group consisting of a direct bond, alkyl, alkenyl, alkynyl, heteroalkyl, heteroalkenyl, heteroalknyl, saturated or partially unsaturated cycloalkyl, saturated or partially unsaturated heterocyclyl, aryl, and heteraryl, wherein each of alkyl, alkenyl, alkynyl, heteroalkyl, heteroalkenyl, heteroalknyl, cycloalkyl, heterocyclyl, aryl and heteroaryl is optionally substituted with one or more R^{f};
W is selected from the group consisting of: 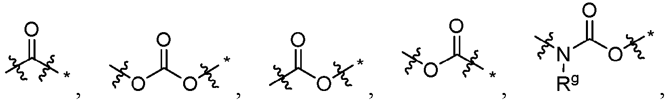 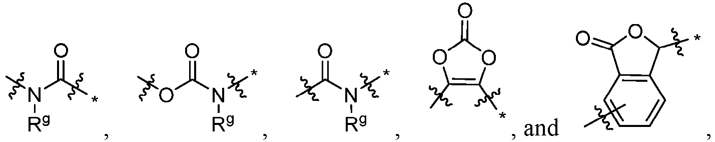
wherein the * end of W is connected to L;
R⁴ is selected from the group consisting of hydrogen, alkyl, alkenyl, alkynyl, heteroalkyl, heteroalkenyl, heteroalknyl, saturated or partially unsaturated cycloalkyl, saturated or partially unsaturated heterocyclyl, aryl, and heteraryl, wherein each of alkyl, alkenyl, alkynyl, heteroalkyl, heteroalkenyl, heteroalkynyl, cycloalkyl, heterocyclyl, aryl and heteroaryl is optionally substituted with cyano, halogen, hydroxyl, amino, alkyl, alkenyl, alkynyl, heteroalkyl, heteroalkenyl, heteroalkynyl;
each of R^{a} is independently selected from the group consisting of hydrogen, halogen, hydroxyl, amino, cyano, nitro, or -NR^{c}R^{d};
R^{b} is selected from the group consisting of hydrogen, hydroxyl, alkyl, alkenyl, alkynyl, heteroalkyl, heteroalkenyl, heteroalknyl, saturated or partially unsaturated cycloalkyl, saturated or partially unsaturated heterocyclyl, aryl, heteraryl, -NR^{c}R^{d} and -OR^{e};
each of R^{c} and R^{d} is independently selected from the group consisting of hydrogen, alkyl, alkenyl, alkynyl, heteroalkyl, heteroalkenyl, heteroalkynyl, aryl, and heteroaryl, wherein each of alkyl, alkenyl, alkynyl, heteroalkyl, heteroalkenyl, heteroalkynyl, aryl and heteroaryl is optionally substituted with cyano, halogen, hydroxyl, or amino;
R^{e} is selected from the group consisting of hydrogen, alkyl, alkenyl, alkynyl, heteroalkyl, heteroalkenyl, heteroalkynyl, saturated or partially unsaturated cycloalkyl, saturated or partially unsaturated heterocyclyl, aryl, and heteroaryl, wherein each of alkyl, alkenyl, alkynyl, heteroalkyl, heteroalkenyl, heteroalkynyl, cycloalkyl, heterocyclyl, aryl and heteroaryl is optionally substituted with cyano, halogen, hydroxyl, amino, alkyl, alkenyl, alkynyl, heteroalkyl, heteroalkenyl, or heteroalkynyl;
each of R^{f} is independently selected from the group consisting of hydrogen, cyano, halogen, hydroxyl, amino, alkyl, alkenyl, alkynyl, heteroalkyl, heteroalkenyl, heteroalkynyl, saturated or partially unsaturated cycloalkyl, saturated or partially unsaturated heterocyclyl, aryl, and heteroaryl; or
two R^{f} together with the atom to which they are both attached, form saturated or partially unsaturated cycloalkyl, or saturated or partially unsaturated heterocyclyl, wherein each of cycloalkyl and heterocyclyl is optionally substituted with cyano, halogen, hydroxyl, amino and alkyl;
R^{g} is selected from the group consisting of hydrogen, alkyl, alkenyl, alkynyl, heteroalkyl, heteroalkenyl, heteroalkynyl, saturated or partially unsaturated cycloalkyl, saturated or partially unsaturated heterocyclyl, aryl, and heteroaryl, wherein each of hydrogen, alkyl, alkenyl, alkynyl, heteroalkyl, heteroalkenyl, heteroalkynyl, cycloalkyl, heterocyclyl, aryl and heteroaryl is optionally substituted with cyano, halogen, hydroxyl, or amino;
n is 0, 1, 2, 3, 4 or 5.

2. The pharmaceutical composition according to claim 1, wherein the cyclodextrin is β-cyclodextrin.

3. The pharmaceutical composition according to claim 2, wherein the β-cyclodextrin is sulfobutyl ether-β-cyclodextrin, cyclobutyl alkyl ether-β-cyclodextrin, or hydroxypropyl-β-cyclodextrin.

4. The pharmaceutical composition according to any one of claims 1-3, wherein the pharmaceutical composition is a solution.

5. The pharmaceutical composition according to claim 4, wherein the solution comprises 0.1-10 mg/mL, 0.2-10 mg/mL, 0.3-5 mg/mL, 0.4-2 mg/mL, 0.5-1 mg/mL, or 0.6-0.9 mg/mL of the compound or the pharmaceutically acceptable salt thereof.

6. The pharmaceutical composition according to claim 4 or 5, wherein the solution comprises 20-400 mg/mL, 30-300 mg/mL, 40-300 mg/mL, 50-250 mg/mL, 60-200 mg/mL, 70-150 mg/mL, or 80-100 mg/mL of the cyclodextrin.

7. The pharmaceutical composition according to any one of claims 1-6, wherein the weight ratio of the cyclodextrin to the compound or the pharmaceutically acceptable salt thereof is 50:1 to 400:1, 50:1 to 300:1, 50:1 to 250:1, 60:1 to 250:1, 70:1 to 200:1, 80:1 to 150:1, or 90:1 to 120:1, wherein the weight of the pharmaceutically acceptable salt of the compound is based on the weight of the compound contained therein.

8. The pharmaceutical composition according to any one of claims 1-7, further comprising a buffer.

9. The pharmaceutical composition according to claim 8, wherein the buffer is an acidic buffer.

10. The pharmaceutical composition according to claim 9, wherein the acidic buffer is selected from phosphoric acid, hydrochloric acid, succinic acid, acetic acid, tartaric acid, lactic acid, citric acid, malic acid, glycolic acid, or hydrates thereof.

11. The pharmaceutical composition according to claim 10, wherein the acidic buffer is citric acid monohydrate.

12. The pharmaceutical composition according to claim 11, wherein the solution comprises 1-3 mg/mL, 1-2.5 mg/mL, or 1-2 mg/mL of citric acid monohydrate.

13. The pharmaceutical composition according to any one of claims 4-11, wherein the pharmaceutical composition has a pH of 3-4.

14. The pharmaceutical composition according to any one of claims 4-11, wherein the pharmaceutical composition is a lyophilized composition formed by lyophilization of the solution.

15. The pharmaceutical composition according to any one of claims 1-14, wherein R¹ is selected from the group consisting of hydrogen, halogen, nitro, cyano, hydroxyl, amino, alkyl, and heteroalkyl, wherein each of alkyl and heteroalkyl is optionally substituted with one or more R^{a}.

16. The pharmaceutical composition according to claim 15, wherein each of R^{a} is independently selected from halogen, hydroxyl, amino, cyano or nitro.

17. The pharmaceutical composition according to claim 15, wherein R¹ is halogen, cyano, methyl or trifluoromethyl.

18. The pharmaceutical composition according to claim 17, wherein R¹ is fluoro or chloro.

19. The pharmaceutical composition according to any one of claims 1-14, wherein R² is -C(O)R^{b}, and R^{b} is selected from the group consisting of hydrogen, hydroxyl, alkyl, alkenyl, alkynyl, heteroalkyl, heteroalkenyl, heteroalknyl, saturated or partially unsaturated cycloalkyl, -NR^{c}R^{d} and -OR^{e}.

20. The pharmaceutical composition according to claim 19, wherein each of R^{c} and R^{d} is independently selected from the group consisting of hydrogen, alkyl, and alkenyl, wherein each of alkyl and alkenyl is optionally substituted with halogen or hydroxyl.

21. The pharmaceutical composition according to claim 19, wherein R^{e} is selected from the group consisting of alkyl, alkenyl, heteroalkyl, heteroalkenyl, aryl, and heteroaryl, wherein each of alkyl, alkenyl, heteroalkyl, heteroalkenyl, aryl and heteroaryl is optionally substituted with cyano, halogen, hydroxyl, amino, or alkyl.

22. The pharmaceutical composition according to claim 19, wherein R² is -C(O)R^{b}, where R^{b} is hydrogen, hydroxyl, alkyl, saturated cycloalkyl or -OR^{e}.

23. The pharmaceutical composition according to claim 22, wherein R^{e} is alkyl, for example, methyl, ethyl, n-propyl or isopropyl.

24. The pharmaceutical composition according to claim 19, wherein R² is -C(O)-cyclopropyl or -C(O)OCH₃.

25. The pharmaceutical composition according to any one of claims 1-14, wherein R³ is selected from hydrogen, alkyl, alkenyl, heteroalkyl, or heteroalkenyl, wherein each of alkyl, alkenyl, heteroalkyl, and heteroalkenyl is optionally substituted with cyano, halogen, hydroxyl, amino, or alkyl.

26. The pharmaceutical composition according to claim 25, wherein R³ is hydrogen or alkyl optionally substituted with halogen, hydroxyl, cyano or amino.

27. The pharmaceutical composition according to claim 25, wherein R³ is hydrogen, methyl, ethyl, or propyl.

28. The pharmaceutical composition according to any one of claims 1-14, wherein L is selected from the group consisting of a direct bond, alkyl, heteroalkyl, saturated or partially unsaturated cycloalkyl, and saturated or partially unsaturated heterocyclyl, wherein each of alkyl, heteroalkyl, cycloalkyl and heterocyclyl is optionally substituted with one or more R^{f}.

29. The pharmaceutical composition according to claim 28, wherein each of R^{f} is independently selected from the group consisting of hydrogen, halogen, hydroxyl, amino, alkyl, and heteroalkyl.

30. The pharmaceutical composition according to claim 28, wherein two R^{f} together with the atom to which they are both attached, form saturated or partially unsaturated cycloalkyl optionally substituted with cyano, halogen, hydroxyl, amino and alkyl.

31. The pharmaceutical composition according to claim 28, wherein L is alkyl optionally substituted with one or more R^{f}.

32. The pharmaceutical composition according to claim 31, wherein each of R^{f} is independently selected from the group consisting of hydrogen, halogen, hydroxyl, methyl and ethyl.

33. The pharmaceutical composition according to claim 28, wherein L is a direct bond, -CH₂-, -CH(CH₃)-, or -C(CH₃)₂-.

34. The pharmaceutical composition according to any one of claims 1-14, wherein W is

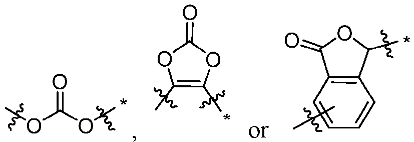

35. The pharmaceutical composition according to any one of claims 1-14, wherein W is

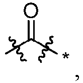

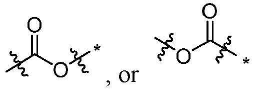

36. The pharmaceutical composition according to any one of claims 1-14, wherein W is

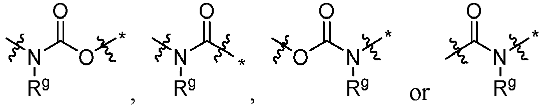

37. The pharmaceutical composition according to claim 36, wherein R^{g} is selected from the group consisting of hydrogen, alkyl and heteroalkyl, wherein each of alkyl and heteroalkyl is optionally substituted with halogen, hydroxyl, cyano, or amino.

38. The pharmaceutical composition according to claim 37, wherein R^{g} is hydrogen, methyl, or ethyl.

39. The pharmaceutical composition according to any one of claims 1-14, wherein R⁴ is hydrogen, alkyl, alkenyl, heteroalkyl, heteroalkenyl or aryl, wherein each of alkyl, alkenyl, heteroalkyl, heteroalkenyl and aryl is optionally substituted with cyano, halogen, hydroxyl, amino, or alkyl.

40. The pharmaceutical composition according to claim 39, wherein R⁴ is hydrogen, alkyl or aryl optionally substituted with halogen, hydroxyl, cyano or amino.

41. The pharmaceutical composition according to claim 40, wherein R⁴ is hydrogen, methyl, ethyl, n-propyl, isopropyl, n-butyl, i-butyl, s-butyl or t-butyl, each of which is optionally substituted with halogen, hydroxyl, cyano or amino.

42. The pharmaceutical composition according to claim 40, wherein R⁴ is hydrogen, -CH₃, -CH₂CH₃, -CH(CH₃)₂, -C(CH₃)₃, -CH(CH₃)(NH₂) or phenyl.

43. The pharmaceutical composition according to any one of claims 1-14, wherein 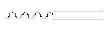 is a double bond in Z configuration.

44. The pharmaceutical composition according to any one of claims 1-14, wherein 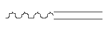 is a double bond in E configuration.

45. The pharmaceutical composition according to any one of claims 1-14, wherein n is 1.

46. The pharmaceutical composition according to any one of claims 1-14, having a formula selected from the group consisting of: 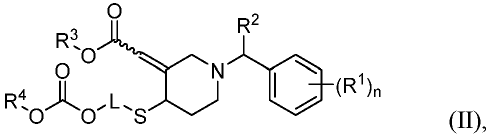 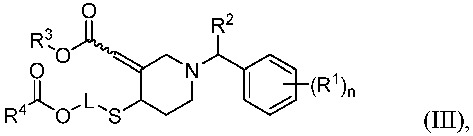 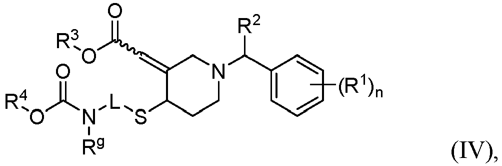 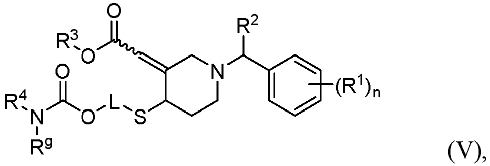 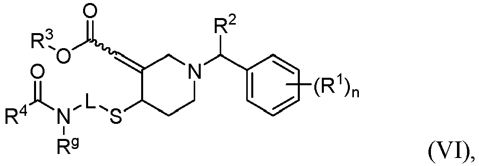 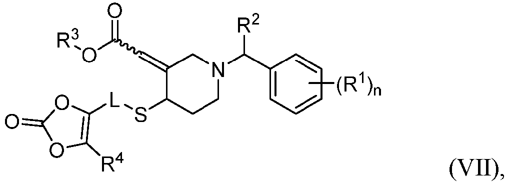 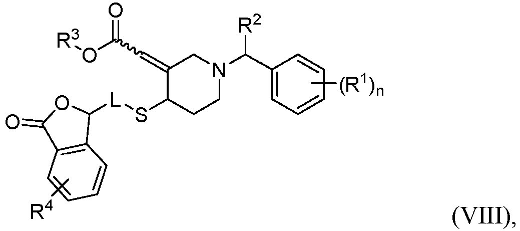 and

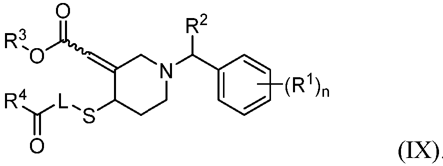

47. The pharmaceutical composition according to any one of claims 1-14, having a formula selected from the group consisting of: 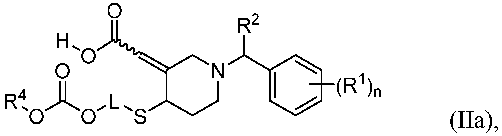 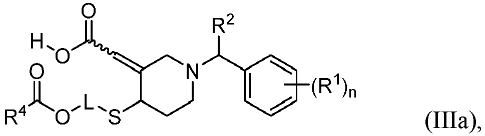 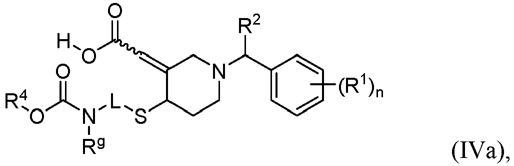 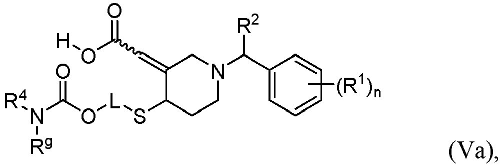 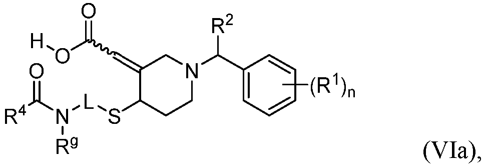 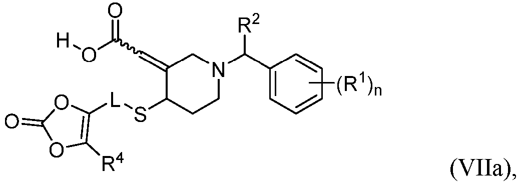 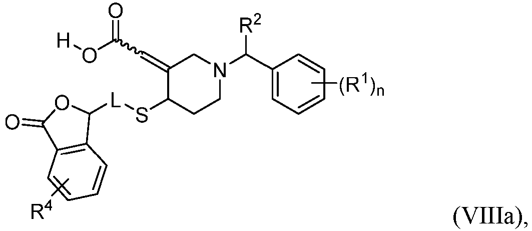 and

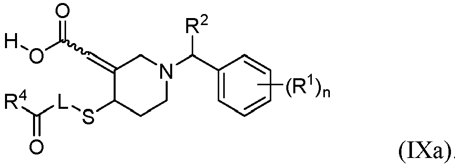

48. The pharmaceutical composition according to any one of claims 1-14, having a formula selected from the group consisting of: 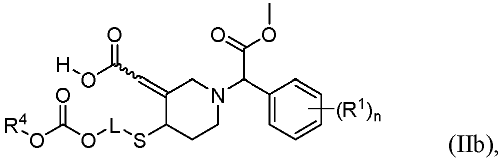 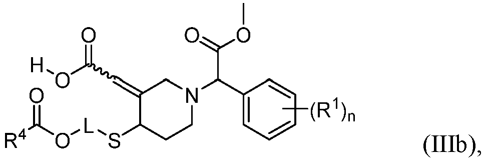 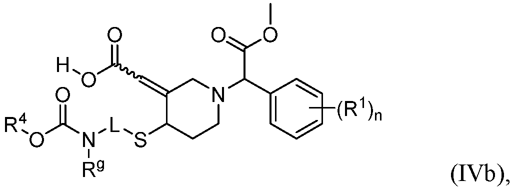 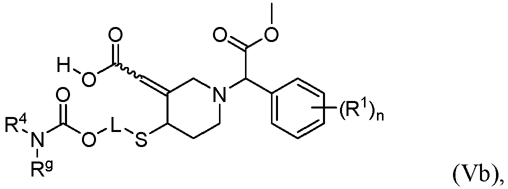 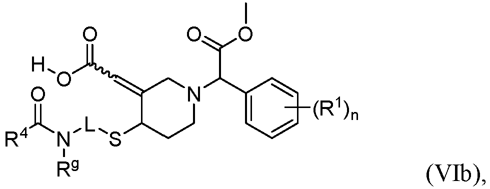 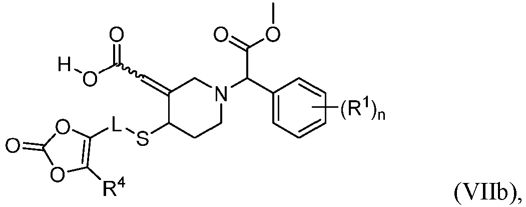 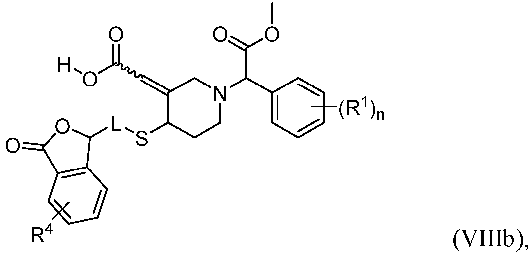 and

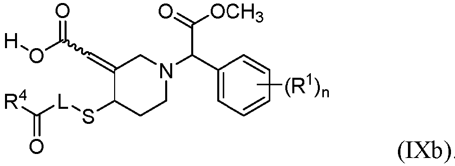

49. The pharmaceutical composition according to any one of claims 46-48, wherein 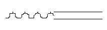 is a double bond in Z configuration.

50. The pharmaceutical composition according to any one of claims 46-48, wherein 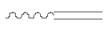 is a double bond in E configuration.

51. The pharmaceutical composition according to any one of claims 46-48, wherein R¹ is halogen and n is 1.

52. The pharmaceutical composition according to any one of claims 1-51, wherein the compound has a formula selected from the group consisting of:

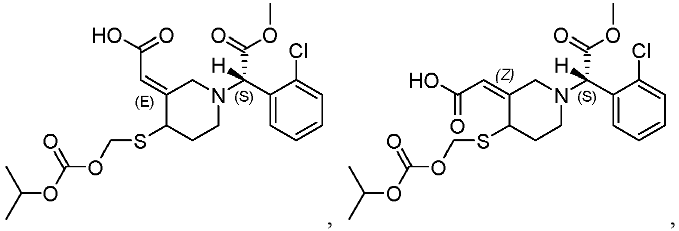

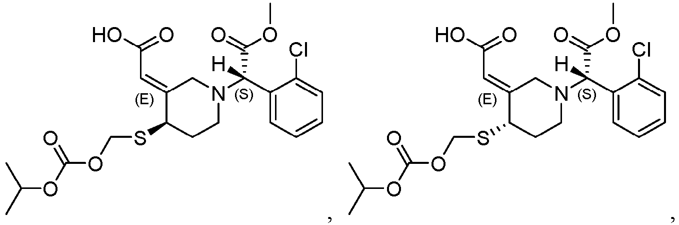

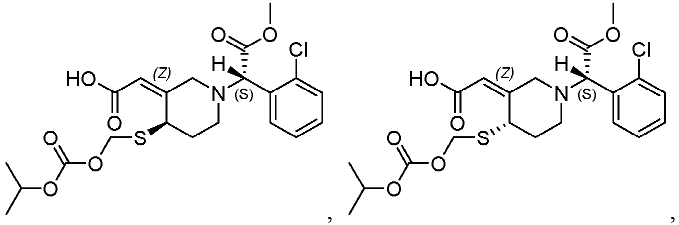

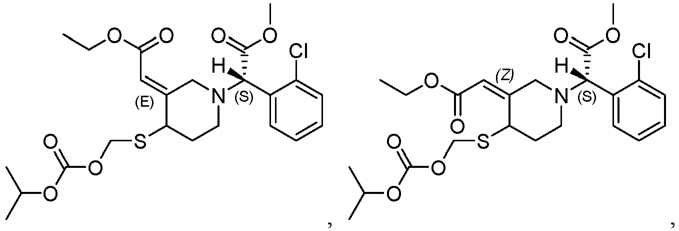

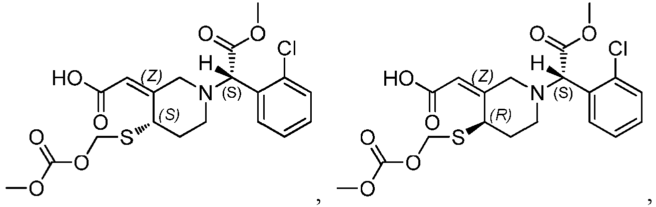

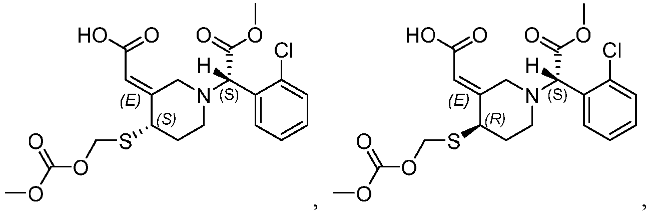

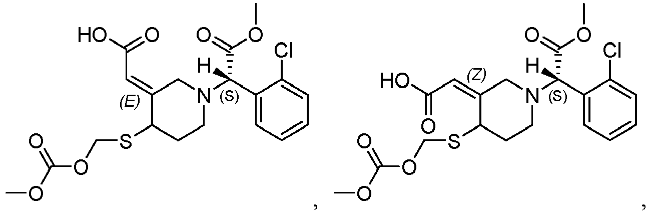

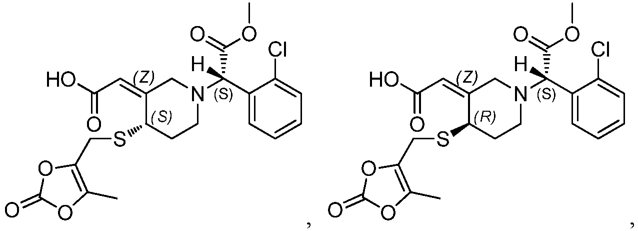

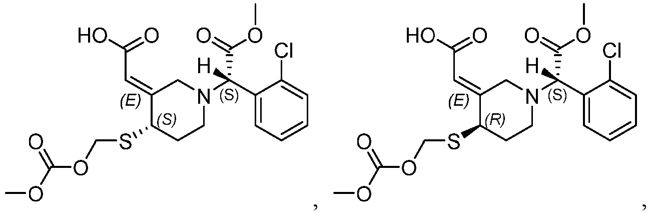

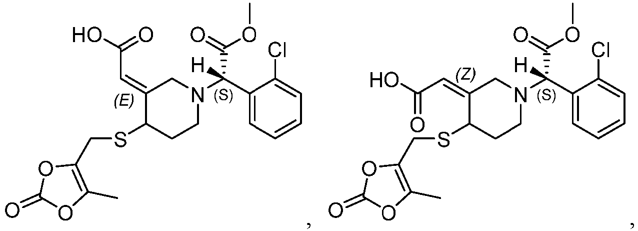

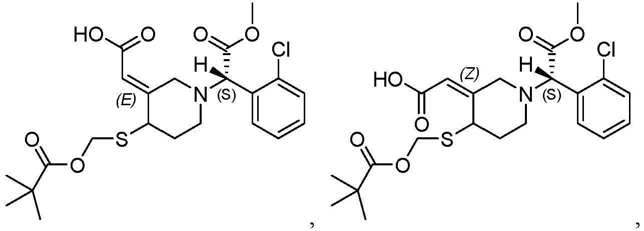

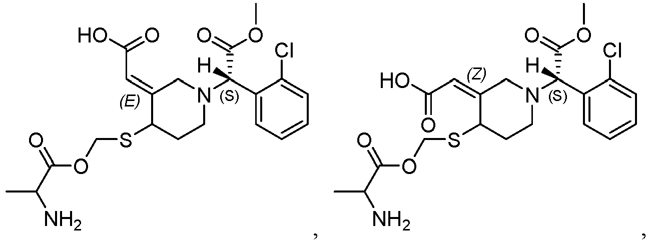

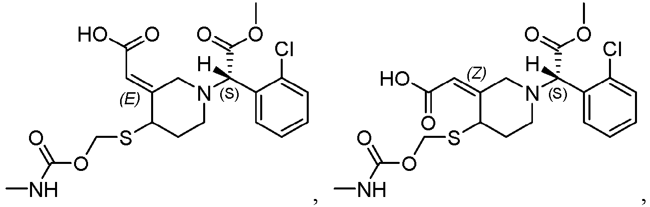

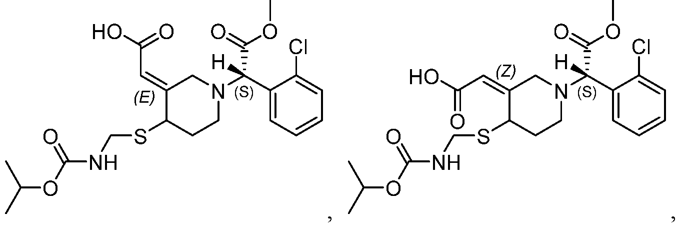

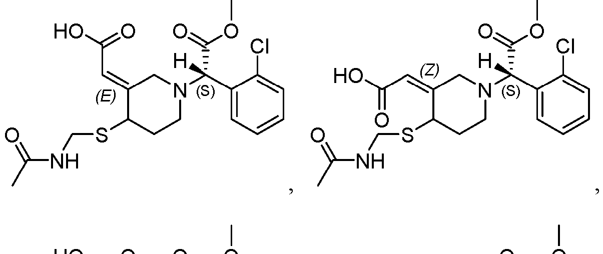

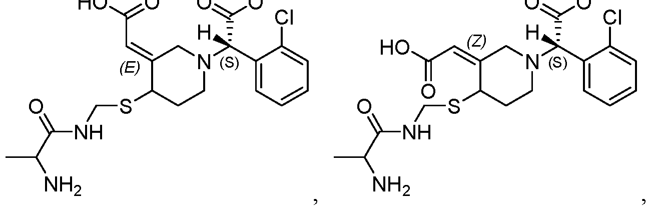

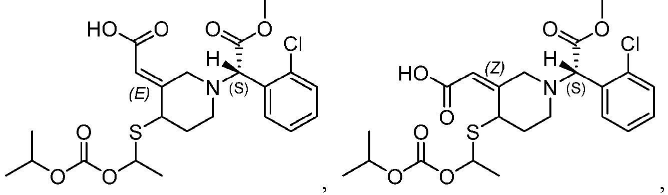

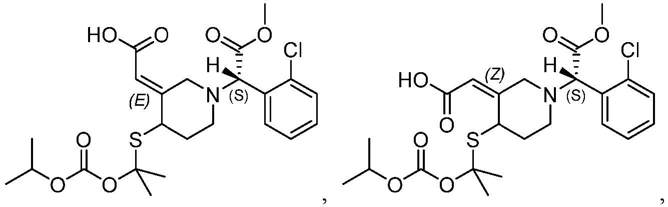

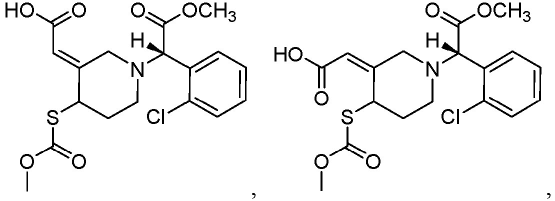

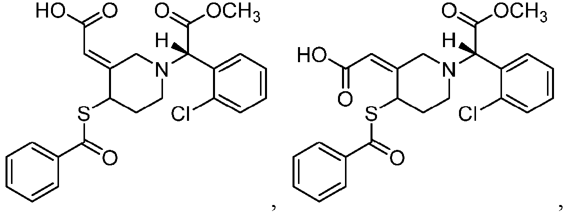

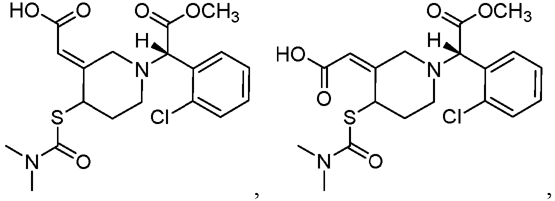

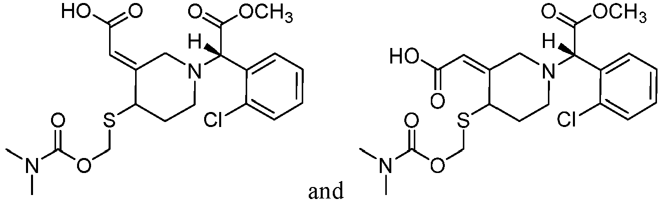

53. The pharmaceutical composition according to any one of claims 1-52, wherein the compound is:

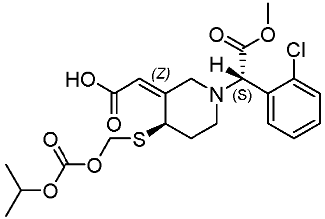

54. A method for preparing the pharmaceutical composition according to any one of claims 1-53, comprising:
mixing the cyclodextrin with water to form a first mixture;
optionally, adding a buffer to the first mixture to form a second mixture;
adding an acidic pH adjuster to the second mixture to adjust the pH to 1-1.5, thereby forming a third mixture;
adding the compound or the pharmaceutically acceptable salt thereof to the third mixture to form a fourth mixture;
adding a basic pH adjuster to the fourth mixture to adjust the pH to 3-4, thereby forming a fifth mixture; and
optionally, adding water for injection to the fifth mixture to form the pharmaceutical composition.

55. The method according to claim 54, further comprising lyophilizing the pharmaceutical composition to form a lyophilized composition.

56. A method for treating a vascular disease in a subject in need thereof, comprising administering to the subject a therapeutically effective amount of the pharmaceutical composition according to any one of claims 1-53.

57. The method according to claim 56, wherein the vascular disease is selected from atherothrombosis, ischemia, stroke, cerebral thrombosis, arterial thrombosis, thrombotic cerebrovascular diseases, cardiovascular diseases, and blood clot.

58. A method for inhibiting platelet aggregation in a subject in need thereof, comprising administering to the subject a therapeutically effective amount of the pharmaceutical composition according to any one of claims 1-53.
